# EUROPEAN PATENT SPECIFICATION

(11) **EP 3 035 058 B1**
(45) Date of publication and mention of the grant of the patent: **04.09.2019**
(21) Application number: 14836455.7
(22) Date of filing: 25.07.2014
(51) Int. Cl.: G01N 33/574, G01N 33/68, G01N 33/48

(54) **CANCER MARKER SCREENING METHOD THROUGH DETECTION OF DEGLYCOSYLATION OF GLYCOPROTEIN AND HEPATOCELLULAR CANCER MARKER**
KREBSMARKER-SCREENING-VERFAHREN DURCH ERKENNUNG VON GLYCOPROTEIN-DEGLYCOSYLIERUNGEN UND HEPATOZELLULÄREN KREBSMARKERN
PROCÉDÉ DE CRIBLAGE DE MARQUEUR DE CANCER PAR L'INTERMÉDIAIRE D'UNE DÉTECTION DE DÉGLYCOSYLATION DE GLYCOPROTÉINE ET DE MARQUEUR DE CANCER HÉPATOCELLULAIRE

(30) Priority: 13.08.2013 KR 20130095978
(43) Date of publication of application: 22.06.2016
(73) Proprietor: Seoul National University R&DB Foundation, Gwanak-gu Seoul 08826 (KR)
(72) Inventor: KIM, Youngsoo, Seoul 135-969 (KR); YOON, Junghwan, Seoul 110-744 (KR); KIM, Hyunsoo, Bucheon-si Gyeonggi-do 420-804 (KR); KIM, Kyunggon, Goyang-si Gyeonggi-do 411-781 (KR); JIN, Jonghwa, Seoul 136-725 (KR)
(74) Representative: Ter Meer Steinmeister & Partner
(86) International application number: PCT/KR2014/006795
(87) International publication number: WO 2015/023068

(56) References cited:
- WO-A2-2013/025322
- KR-A- 20080 011 287
- KR-A- 20100 028 088
- KR-A- 20100 120 788
- KR-A- 20120 125 157
- US-A1- 2007 037 221
- ZE LIU ET AL: "Tandem 18O Stable Isotope Labeling for Quantification of N-Glycoproteome", JOURNAL OF PROTEOME RESEARCH., vol. 9, no. 1, 4 January 2010 (2010-01-04) , pages 227-236, XP055378635, US ISSN: 1535-3893, DOI: 10.1021/pr900528j
- SHU ZHANG ET AL: "iTRAQ plusO: A new technique for target glycoprotein analysis", TALANTA, ELSEVIER, AMSTERDAM, NL, vol. 91, 16 January 2012 (2012-01-16), pages 122-127, XP028461786, ISSN: 0039-9140, DOI: 10.1016/J.TALANTA.2012.01.033 [retrieved on 2012-01-21]

## Description

### BACKGROUND OF THE INVENTION

### Field of the Invention

The present disclosure generally relates to methods for screening cancer biomarkers and biomarker specific for cancer diagnosis.

### Description of the Related Art

Biomarkers are widely used to diagnosis various diseases including cancer in which the early detection or diagnosis is crucial for the successful treatment or the accurate diagnosis is difficult with conventional methods. Nucleic acid molecules or proteins are two commonly used types of biomarkers with which the expression levels or any changes in the amount are used as parameters for diagnosis. Recently post-translational modifications of proteins have been developed as biomarkers and one of them is to detect the glycosylation of proteins.

Thus methods have been developed to detect or analyze the changes or differences in the glycosylation levels of proteins. For example, glycoproteins are hydrolyzed to release glycans, which are then collected to profile the glycosylation status (Cooke C.L. et al., Anal. Chem., 2007,79:8090-8097). Although such methods can be used to differentiate a healthy person from a patient, they have limitations in that various information such as specific information on the glycosylated proteins, positons of the glycosylation and isoforms are required for an accurate diagnosis.

ZE LIU ET AL: "Tandem 180 Stable Isotope Labeling for Quantification of N-Glycoproteome", JOURNAL OF PROTEOME RESEARCH., vol. 9, no. 1, 4 January 2010 discloses a new strategy of labelling the N-glycoproteome.

SHU ZHANG ET AL: "iTRAQ plus 18O: A new technique for target glycoprotein analysis", TALANTA, ELSEVIER, AMSTERDAM, NL, vol. 91, 16 January 2012 (2012-01-16), pages 122-127 disclose a new strategy to identify N-glycosylation sites.

Korean Patent Publication No. 2012-0125157 relates to biomarkers and methods to diagnosis cancer using the information on the aberrant glycosylation and discloses steps of isolating proteins abnormally glycosylated during the development or progression of cancers using lectins, and selecting and quantifying marker peptides generated from the hydrolysis of the isolated glycosylated proteins.

Korean Patent Publication No. 2010-0120788 relates to methods to diagnose a cancer using the glycosylation of proteins and discloses the use of specific changes in the hydrolysis pattern of particular peptides for the diagnosis of cancer.

However the glycosylation of proteins in patients with cancer or cured of cancer may occur at various amino acids residues such as aspargine, threonine, or serine and the like as in healthy patients. Thus, the specific glycosylation patterns or structure associated with a particular cancer may occur at one of the residues as above and coexist with the glycosylation found in normal cases leading to a microheterogeneity. Therefore the specific glycosylation associated with a particular cancer is present in a minute amount relative to a total amount of proteins, existing as a part of many glycan-isoforms found in any one of the residues. This requires a development of a more sensitive and specific methods for a reliable measurement of the glycosylation changes associated with a particular cancer.

### SUMMARY OF THE INVENTION

The present disclosure is to provide a method of screening caner specific biomarker, which is based on the detection of the glycosylation ratio of particular proteins, and biomarkers screened using the methods and uses thereof as defined in claim 1. Preferred embodiments are defined in claims 2 and 3.

In one aspect, there is provided a method of screening a biomarker for diagnosing or detecting cancer comprising steps of: de-glycosylating proteins having a N-linked glycosylation motif; fragmenting the de-glycosylated proteins; determining in the fragmented proteins the amount of the de-glycosylated peptide at the N-linked motif and the amount of the non-glycosylated peptide at the non-glycosylated motif which does not contain the N-linked motif and the ratio therebetween; and selecting the proteins as a biomarker if the ratio is changed compared to that of a control.

In other aspect, there is provided a biomarker or its use for diagnosis or prognosis of hepatocellular carcinoma, which is selected from a group consisting of Alpha-2-antiplasmin (SERPINF2), Alpha-2-macroglobulin (A2M), Apolipoprotein B-100 (APOB), Beta-galactosidase (GLB1), Bone morphogenetic protein 1 (BMP1), Corticosteroid-binding globulin (SERPINA6), Complement factor H (CFH), Cholinesterase (BCHE), Clusterin (CLU), Collagen alpha-1(XII) chain (COL12A1), Carboxypeptidase N subunit 2 (CPN2), Versican core protein (VCAN), Receptor tyrosine-protein kinase erbB-3 (ERBB3), Coagulation factor V (F5), Coagulation factor XI (F11), Follistatin-related protein 1 (FSTL1), N-acetylglucosamine-6-sulfatase (GNS), G-protein coupled receptor 126 (GPR126), Heparin cofactor 2 (SERPIND1), Hypoxia up-regulated protein 1 (HYOU1), Integrin alpha-2 (ITGA2), Integrin alpha-3 (ITGA3), Integrin alpha-6 (ITGA6), Integrin alpha-M (ITGAM), Integrin beta-2 (ITGB2), Plasma kallikrein (KLKB1), Kinectin (KTN1), Lysosome-associated membrane glycoprotein 2 (LAMP2), Galectin-3-binding protein (LGALS3BP), Plexin-Al (PLXNA1), Periostin (POSTN), Inactive tyrosine-protein kinase 7 (PTK7), Roundabout homolog 4 (ROBO4), Tenascin (TNC) and Vitronectin (VTN).

In other aspect, there is provided a method of detecting HCC in vitro, or diagnose or prognosis of HCC in a subject or a sample in need thereof comprising steps of: measuring, at a protein or a nucleic acid level, the concentration and/or the presence of, or the glycosylation level of at least one biomarkers as described above; and comparing the result to that of a corresponding marker from a control sample wherein the change in the concentration and/or presence of the biomarker at a nucleic acid and/or protein level or in the glycosylation level in the subject or the sample compared to the control indicates the presence of HCC in the subject or the sample.

In the present methods, the detection of each biomarker is performed by determining the amount of the de-glycosylated peptides and/or the non-glycosylated peptides of at least one of the proteins as disclosed in Tables 17-1 to 17-3 or the ratio therebetween.

The foregoing summary is illustrative only and is not intended to be in any way limiting. Additional aspects and/or advantages of the invention will be set forth in part in the description which follows and, in part, will be obvious from the description, or may be learned by practice of the invention.

### ADVANTAGEOUS EFFECTS

The present methods can be advantageously used for screening biomarkers for diagnosis or prognosis or monitoring cancers with a high specificity and sensitivity by measuring the glycosylation ratio of the conventional markers. Also the biomarkers screened by the present methods are having a higher specificity or sensitivity than the conventional markers, which can be used advantageously to diagnose, monitor the cancer or determine the stages of the cancer. Also the biomarkers and the methods of the present disclosure employed in the glycosylation analysis provides a simple and non-invasive way of diagnose or monitoring cancer using blood as a sample.

### BRIEF DESCRIPTION OF THE DRAWINGS

These and/or other aspects and advantages of the invention will become apparent and more readily appreciated from the following description of the embodiments, taken in conjunction with the accompanying drawings of which:
FIG. 1 is a schematic representation of the analysis principal of the glycosylated fragments using LC-Mass in one embodiment of the present disclosure, in which glycosylated and non-glycosylated peptides are indicated as green and glycosylated amino acids are indicated as red.
FIG.2 is an amino acid sequence (SEQ ID NO: 1) of Invertase-1 used as a standard glycosylated protein in one embodiment of the present disclosure.
FIGs.3a to 3c are graphs showing the results of MRM analysis of the glycosylated peptide 1 (NPVLAANSTQFR) of the glycosylated standard protein, in which the peak area according to the concentrations are represented.
FIGs.4a to 4c are graphs showing the results of MRM analysis of the glycosylated peptide 2 (FATNTTLTK) of the glycosylated standard protein, in which the peak area according to the concentrations are represented.
FIGs.5a to 5c are graphs showing the results of MRM analysis of the non-glycosylated peptide 1 (IEIYSSDDLK) of the glycosylated standard protein, in which the peak area according to the concentrations are represented.
FIGs.6a to 6c are graphs showing the results of MRM analysis of the non-glycosylated peptide 2 (VVDFGK) of the glycosylated standard protein, in which the peak area according to the concentrations are represented.
FIG.7 is a graph showing the peak area obtained from MRM analysis of the endogenous peptides corresponding to each target peptide as in FIGs 3 to 6.
FIG.8 is an amino acid sequence of AFP (Alpha fetoprotein) (SEQ ID NO: 2), in which glycosylated peptide and non-glycosylated peptide are indicated as red and green, respectively.
FIGs.9a and 9b are results of MRM analysis of glycosylated peptide (VNFTEIQ) and de-glycosylated peptide (VDFTEIQ) (SEQ ID NO: 9) of AFP, respectively using the pooled normal control sample.
FIGs.9c and 9d are results of MRM analysis of glycosylated peptide (VNFTEIQ) and de-glycosylated peptide (VDFTEIQ) of AFP, respectively using the pooled liver cancer patient sample.
FIGs.10a and 10b are results of MRM analysis of non-glycosylated peptide (GYQELLEK) and de-glycosylated peptide (GYQELLEK) (SEQ ID NO: 10) of AFP, respectively using the pooled normal control sample.
FIGs.10c and 10d are results of MRM analysis of non-glycosylated peptide (GYQELLEK) and de-glycosylated peptide (GYQELLEK) of AFP, respectively using the pooled liver cancer patient sample.
FIG. 11 is a graph showing the difference in the peak area between the normal sample and liver cancer sample obtained from MRM analysis for AFP target peptides.
FIG.12 is a CE optimization result for AFP target peptide using MRM analysis.
FIG.13a is a result of MRM analysis of the endogenous AFP de-glycosylated target peptide (VDFTEIQK).
FIG.13b is a result of MRM analysis of the endogenous AFP non-glycosylated target peptide (GYQELLEK)
FIG.14 is a result of MRM analysis showing the linearity which was performed to confirm the quantifiable property of the heavy labelled synthetic peptides to AFP target peptide.
FIG.15 is a result of MRM analysis using clinical samples in which de-glycosylation of the glycosylated peptide and non-glycosylated peptide of AFP were analyzed.

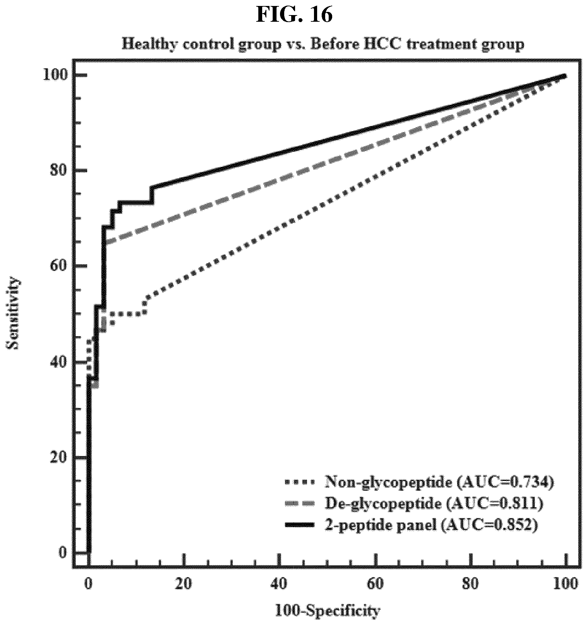

FIG.16 is a result comparing AUC values of AFP target peptide and 2-peptides (de-glycosylated and non-glycosylated peptides) panel.
FIGs.17a to 17z are results each showing AUC values of standardized de-glycosylated peptide and standardized non-glycosylated peptide and the ratio thereof of a biomarker protein screened by the present methods.
FIGs.18a to 18i are results each showing AUC values of standardized de-glycosylated peptide and standardized non-glycosylated peptide and the ratio thereof of a biomarker protein screened by the present methods.
FIGs.19a to 19z are results each showing an interactive plot in which the amount of the standardized de-glycosylated peptide and non-glycosylated peptide and the ratio thereof of a biomarker protein screened by the present methods are compared between a normal subject and a liver cancer subject samples.
FIGs.20a to 20i are results each showing an interactive plot in which the amount of the standardized de-glycosylated peptide and non-glycosylated peptide and the ratio thereof of a biomarker protein screened by the present methods are compared between a normal subject and a liver cancer subject samples.

### DETAILED DESCRIPTION OF THE EMBODIMENTS

The present disclosure is based on the findings that the level of glycosylation of particular proteins in comparison to its non-glycosylated forms occurring during the post translational modification can be used effectively not only to diagnose cancers and also for screening biomarkers to detect cancers.

In one aspect, the present disclosure provides a method of screening a biomarker for diagnosing or detecting cancer comprising steps of: de-glycosylating proteins having a N-linked glycosylation motif; fragmenting the de-glycosylated proteins; determining in the fragmented proteins the amount of the de-glycosylated peptide at the N-linked motif and the amount of the non-glycosylated peptide at the non-glycosylated motif which does not contain the N-linked motif and the ratio therebetween; and selecting the proteins as a biomarker if the ratio is changed compared to that of a control as defined in claim 1.

The terms, marker in relation to cancer, biomarker, cancer marker, cancer or biomarker as used herein refers to an agent that may discriminate a cancer tissues or cells from normal cells or tissues, or a treated cancer tissues or cells, and comprises an organic and biological molecule and the like, such as proteins or nucleic acid molecules, lipids, glycolipids, and glycoproteins that have increased or decreased in cancer tissues or cells compared to normal control samples. In the present disclosure, as markers for a hepatocellular cancer, glycoproteins the expression level or the extent of glycosylation of which are decreased or increased are employed.

In the present disclosure, the value determined in a normal control which is used to compare to that of a cancer sample may be a value determined during or before the method is performed.

Proteins undergo post translational modification (PTM) after translation to become functional. Among PTM, glycosylation plays an important role in various cellular process or properties of the proteins such as half-lives, cell-cell interaction and antigenic properties of the proteins. Glycosylation is the enzyme catalyzed process in contrast to non-enzymatic glycation process, and during the process sugars are added to proteins to form glycan chains.

Included in the types of Glycosylation are N-linked glycosylation, O-linked glycosylation, C-mannosylation and GPI (glycophosphatidyl-inositol) anchor attachment. Encompassed in the present disclosure is N-linked glycosylation.

By N-linked glycosylation, glycan is attached to Asparagine residue at the same time with a translation affecting protein folding. N-linked glycosylation occurs at a particular peptide motif including Asn-Xxx-Ser/Thr(N-X-S/T) or Asn-Xxx-Cys (N-X-C), in which Xxx refers to any amino acids except proline. The proteins comprised in the present biological sample comprise N-linked glycosylation motifs at all or part of which the proteins are glycosylated. As the amount of proteins expressed and/or the level of glycosylation are increased during a disease development or progression such as cancer, a particular type of sugar for example fucose is attached.

The biological samples employed in the present disclosure are a protein or a sample from a patient or a normal control that comprises a N-linked glycosylation motif. The samples to be tested are from patients who have a cancer or who are suspected of having cancer or who are in need of a cancer diagnosis or who are undergoing cancer therapy or who are cured of cancer. As a control, biological samples from a normal subject or a subject cured of cancer may be used. In the present disclosure, the subject includes mammals, particularly humans.

In accordance of the present disclosure, not only biological samples from appropriate patients but also proteins extracted from the sample are included. In one embodiment, the samples embodied in the present disclosure are a biological sample obtained from an organism including proteins from which information related to disease such as cancer development or progress or status can be determined or detected. Such samples include biological tissues, cell lines obtained by culturing biological tissues or media from the culture cells, cells, whole blood, serum, plasma, saliva, urine, cerebro-spinal fluid, **liquor folliculi,** milk and pancreatin, but are not limited thereto. Particularly glycoproteins related to cancer development or progress of the disease are released from the cells into the blood or extracellular fluids and thus bloods from the patient/subject to be tested or culture media in which cancer cells have been cultured can be advantageously used for detecting glycoproteins. In case of blood, the concentrations of proteins comprised therein varies widely among them. Thus, the samples may be pretreated to remove abundant proteins using a column such as MARS (Multiple Affinity Removal System) and the like. However, the pretreatment may be omitted if the sensitivity and reproducibility of the target protein detection is not affected.

Particularly, many kinds or monosaccharides present on the surface or the cell membrane move inside the cell membrane by a signal transduction and are enzymatically transferred to proteins in the membrane by N-acetylglucosaminyltransferase to produce glycosylated proteins. The glycoproteins then perform their cellular function. Many glycoproteins present on the cell surface undergo abnormal glycosylation by particular signals generated from such as oncogenes. It has been known that abnormal function of glycosyltransferases and glycolytic enzymes in response to the signal by oncogenes are involved in the cancer development (Kim, Y. J., et al., Glycoconj. J., 1997, 14, 569-576., Hakomori, S., Adv. Cancer Res.,1989, 52, 257-331., Hakomori, S., Cancer Res., 1996, 56, 5309-5318).

Thus the present methods may be applied to discover or screen biomarkers to detect or diagnose or monitoring various cancers such as a blood cancer, a liver cancer, a stomach cancer, a colon cancer, a lung cancer, a uterine cancer, a breast cancer, a prostate cancer, a thyroid cancer and a pancreatic cancer without being limited thereto.

The term diagnosis as used herein refers to determining susceptibility of a subject to a disease or disorder, determining whether a subject has a specific disease or disorder, determining the prognosis (for example, identification of transitional cancer status, stages or progression of a cancer or determining the response to cancer treatments) of a subject who has a particular disease or disorder, or therametrics (for example, monitoring the status of a subject to provide the information on the efficacy of treatment).

In accordance with the present methods, the level of de-glycosylation at the glycosylation motif and the level of non-glycosylation motif and their ratio are determined, which is then compared to the values obtained from a normal control. In comparison to the control, when the ratio is changed, i.e., decreased or increased, in the subject or in the sample, the ratios are used to select biomarkers. The levels may be determined as described hererinafter. When liquid chromatographic methods are used, the area of the peak corresponding to de-glycosylated fragments and the area of the peak corresponding to non-glycosylated fragments are determined, which are then used to calculate the ratios after normalization of each of the peak area above with that of the internal standard peptide, i.e., to calculate the normalized peak area of the de-glycosylated fragment/the normalized peak area of the non-glycosylated fragment.

Therefore, to de-glycosylate the glycosylation motif and fragment them, various de-glycosylation enzymes known in the art may be employed for the present methods. In the claimed invention PNGase-F (Peptide N Glycosidase F) is used. In the present methods, the proteins in the sample are fragmented into polypeptides of 6-24 amino acids in length. For this, various hydrolytic enzymes may be employed, which include for example trypsin that digest amide bond between lysine and arginine. Also lysine-C that hydrolyzes at a lysine residue, arginine-C that hydrolyzes at an arginine residue, an aspartic acid N that hydrolyzes at an aspartic acid may also be used as desired. In one embodiment, a trypsin is used.

The non-glycosylation motif employed in the present methods is an amino acid sequence which is not glycosylated and found in the same protein as the glycosylation motif is found. The non-glycosylation motif does not contain NxS/T motif, cysteine as well as methionine residues which may be modified. The length of the non-glycosylation motif may vary depending on the detection methods employed. For example, when the mass spectrometry is used, the peptide length of about 6 to about 24 amino acids may be used in consideration of the detection range which is about 15-1400m/z, average molecular weight and charges of amino acids, and a minimum length required for conferring specificity. But the length is not limited thereto.

In the present methods, the proteins comprising N-linked glycosylation motif may be from cancer tissues or cancer cells or liquids samples such as blood, or normal control samples or samples cured of cancer. Thus the proteins comprising N-linked glycosylation motif may be selected from the known glycoproteins.

In the present methods, N-linked glycosylation motif and non-glycosylation peptide fragments which do not comprise the N-linked glycosylation motif may be selected from the known glycosylated proteins and have a specific amino acid sequence for each protein or peptide fragments. Thus the proteins showing changes in the ratio of the de-glycosylated peptides to non-glycosylated peptides can be discovered as biomarkers for a cancer from which the protein samples are derived. The glycosylated proteins may be selected from the known database such as Plasma Proteome Database (PPD)(http://www.plasmaproteomedatabase.org/) or http://www.uniprot.org/

In one embodiment, PDD is used and glycosylated proteins which contain a conserved N-linked glycosylation motif NxS/T and are glycosylated at the motif may be selected.

For the quantification of de-glycosylated and non-glycosylation peptides and the ratio therebetween (the de-glycosylated/non-glycosylation peptide), it is preferred to employ a sensitive process particularly in normal and cancer samples or samples suspected of cancer. For this, abundant proteins which represent about 90% of plasma proteins such as albumin, IgG, IgA, Transferrin, Haptoglobin), Fibrinogen are removed. Or the proteins may be purified and concentrated using acetone precipitation or MWCO (molecular weight cutoff) methods to remove salts. In one embodiment of the present disclosure, the de-glycosylated peptide fragment in the N-linked glycosylation motif, NxS/T or NxC, is AsnXxxSer/Thr or AsnXxxSer/Cys in which asparagine in the motif is changed to aspartic acid by glycosylation. That is, the peptide fragments which are detected as a result of de-glycosylation in N-linked glycosylation motif are AspXxxSer/Thr or AspXxxSer/Cys.

In one embodiment of the present disclosure, a mass spectrometry is used for detecting the present markers, wherein the proteins are extracted from the appropriate samples and analyzed using the method such as described in the Examples of the present disclosure, or the literatures Kim, et al. 2010 J Proteome Res. 9: 689-99; Anderson, L et al. 2006. Mol Cell Proteomics 5: 573-88 may also be referred. In one embodiment Multiple Reaction Monitoring (MRM) technology utilizing Triple Quadrupole LC-MS/MS and QTRAP and the like may be used. MRM is a method for exactly quantifying multiple markers present in biological samples in minute amount. In MRM, by a first mass filter (Q1), parent or precursor ions are selected from the ion fragments generated in ionization source and transferred to a collision cell. And then the precursor ions arrived at the collision cell collide with internal collision gas, and are fragmented into products or daughter ions and transferred to a second mass filter (Q2), from which only the specific ions are delivered to a detector. In this way only the information of the desired target can be obtained with high selectivity and sensitivity. The literature Gillette et al., 2013, Nature Methods 10:28-34 and the like may be referred.

In other embodiment, liquid chromatography mass spectrometry is used. For example, Selected Ion Monitoring (SIM) or MRM is used, in which the peptides are not labelled and the data generated are analyzed based on the accurate MW of the peptides or proteins and the retention time of the peptides separated from the chromatography. In one embodiment, MRM is employed. In MRM analysis, peptide/transitions are monitored for analysis.

In MRM, a relative analysis without the use of labelling, or an absolute analysis using stable isotope labeled peptide standard which is injected before the analysis are used. Also for a more efficient quantification using multiple reaction monitoring, the database and programs such as TIQAM(targeted identification for quantitative analysis by MRM) may also be employed to select a unique peptide only detected in the candidate proteins and to generate and confirm MRM transition of the peptide (Anderson L, et al., Mol. Cell Proteomics. 2006, 5: 573-588).

In one embodiment, blood is obtained from a patient having a disease or suspected of a disease, which is then analyzed by LC/MS (Liquid Chromatography/Mass Spectrometer) to detect the glycosylation and de-glycosylation levels and the ratio therebetween in the appropriate proteins. The levels and/or ratios determined in the test samples are then compared to that of a control to diagnose and/or for selecting biomarkers.

As a way of example, cutoff value of a particular peptide at issue in the normal sample (upper or lower limit depending on increasing or decreasing, respectively) is determined. Then the ratio of de-glycosylation/non-glycosylation level determined in the samples from a patient having a disease or suspected of a disease is changed, i.e., decreased or increased, compared to the cutoff value, the patient is diagnosed to have a disease.

In one embodiment, the glycosylated proteins are concentrated from blood using hydrazide or lectin column, which are then treated with trypsin to collect non-glycosylated peptides. And the proteins are treated with PNGase-F to collect de-glycosylated peptides. All the peptides are then analyzed with LC-MS/MS to identify glycoproteins in blood.

In the present methods, the de-glycosylation and non-glycosylation can be determined at the same time or in separate for multiple proteins in one sample. For example, a maximum of about 1000 peptides which include de-glycosylated and non-glycosylated peptides may be analyzed at one time, which corresponds to detection of about 500 glycoproteins.

In other aspect, the present disclosure relates to biomarkers screened by the present methods for hepatocellular carcinoma and theirs uses for diagnosing or detecting HCC in samples or tissues. The markers are at least one, preferably at least two markers selected from a group consisting of Alpha-2-antiplasmin (SERPINF2), Alpha-2-macroglobulin (A2M), Apolipoprotein B-100 (APOB), Beta-galactosidase (GLB1), Bone morphogenetic protein 1 (BMP1), Corticosteroid-binding globulin (SERPINA6), Complement factor H (CFH), Cholinesterase (BCHE), Clusterin (CLU), Collagen alpha-1(XII) chain (COL12A1), Carboxypeptidase N subunit 2 (CPN2), Versican core protein (VCAN), Receptor tyrosine-protein kinase erbB-3 (ERBB3), Coagulation factor V (F5), Coagulation factor XI (F11), Follistatin-related protein 1 (FSTL1), N-acetylglucosamine-6-sulfatase (GNS), G-protein coupled receptor 126 (GPR126), Heparin cofactor 2 (SERPIND1), Hypoxia up-regulated protein 1 (HYOU1), Integrin alpha-2 (ITGA2), Integrin alpha-3 (ITGA3), Integrin alpha-6 (ITGA6), Integrin alpha-M (ITGAM), Integrin beta-2 (ITGB2), Plasma kallikrein (KLKB1), Kinectin (KTN1), Lysosome-associated membrane glycoprotein 2 (LAMP2), Galectin-3-binding protein (LGALS3BP), Plexin-A1 (PLXNA1), Periostin (POSTN), Inactive tyrosine-protein kinase 7 (PTK7), Roundabout homolog 4 (ROBO4), Tenascin (TNC) and Vitronectin (VTN).

In other aspect, the present disclosure relates to a composition or a kit for diagnosing or prognosis of HCC comprising agent(s) for detecting the present marker.

In one embodiment, the biomarkers which are used for diagnosing HCC showing changes, i.e., a decrease or an increase in the expression level or glycosylation level in HCC tissues or in blood are at least one markers selected from a group consisting of AFP, Alpha-2-antiplasmin (SERPINF2), Alpha-2-macroglobulin (A2M), Apolipoprotein B-100 (APOB), Beta-galactosidase (GLB1), Bone morphogenetic protein 1 (BMP1), Corticosteroid-binding globulin (SERPINA6), Complement factor H (CFH), Cholinesterase (BCHE), Clusterin (CLU), Collagen alpha-1(XII) chain (COL12A1), Carboxypeptidase N subunit 2 (CPN2), Versican core protein (VCAN), Receptor tyrosine-protein kinase erbB-3 (ERBB3), Coagulation factor V (F5), Coagulation factor XI (F11), Alpha-fetoprotein (AFP), Follistatin-related protein 1 (FSTL1), N-acetylglucosamine-6-sulfatase (GNS), G-protein coupled receptor 126 (GPR126), Heparin cofactor 2 (SERPIND1), Hypoxia up-regulated protein 1 (HYOU1), Integrin alpha-2 (ITGA2), Integrin alpha-3 (ITGA3), Integrin alpha-6 (ITGA6), Integrin alpha-M (ITGAM), Integrin beta-2 (ITGB2), Plasma kallikrein (KLKB1), Kinectin (KTN1), Lysosome-associated membrane glycoprotein 2 (LAMP2), Galectin-3-binding protein (LGALS3BP), Plexin-Al (PLXNA1), Periostin (POSTN), Inactive tyrosine-protein kinase 7 (PTK7), Roundabout homolog 4 (ROBO4), Tenascin (TNC) and Vitronectin (VTN).

In the present disclosure, the present markers may be used in alone or two or more markers may be used in combination to further improve the specificity and/or sensitivity. For example, two, three, four, five, six, seven or more markers may be combined. The person skilled in the art would be able to select the combination of markers that show a desired sensitivity and specificity using the methods such as Logistic regression analysis and/or analysis of the biological samples from the subjects including a normal person and patient using the methods such as described in the examples of the present disclosure.

The hepatocellular carcinoma (HCC) of the present disclosure is a primary malignant tumor of liver tissue developed in a subject who has risk factors such as alcoholic abuse, viral hepatitis and metabolic liver disease. HCC which usually does not develop fibrous stroma leads to a bleeding and necrosis, and thus resulting in a vascular invasion into hepatic portal vein system which may lead to a hepatorrhexis and abdominal plasma exudation in severe cases.

The biological sample of the present disclosure is a substance or a mixture of the substances that contain or is expected to contain/express one or more of the present biomarkers, and includes cells, tissues or bodily fluids from an organism, particularly human, for example, whole blood, urine, plasma, and serum, but is not limited thereto. Also the sample includes cells or tissues cultured in vitro as well as those derived directly from an organism. Various samples may be used for the detection of HCC markers according to the present disclosure. In one embodiment, urine, whole blood, plasma and/or blood serum can be used. In other embodiment, the liver tissues/cells or in vitro cell cultures from an organism of interest, where HCC has developed or HCC is plausible or likely to be developed, may be used, but the samples are not limited thereto. Also the fractions or derivatives of the blood, cells or tissues are included. When cells or tissues are used, lysates thereof may also be used.

The term detection or detecting as used herein refers to a determination in quantity, quality and/or changes in expression patterns and/or profiles of the expression. The detection includes a determination of the presence and/or absence as well as the levels of the present markers. The present markers may be detected using the methods known in the art, and the person skilled in the art would be easily able to select appropriate methods for the detection.

The markers according to the present disclosure can be detected to analyze the presence or absence of the markers and/or the expression level, the difference in the expression level or the changes in the expression level at mRNA and/or protein level through various quantitative and/or qualitative analysis methods known in the art.

The detection of the markers to diagnose HCC according to the present disclosure may be based on the functional and/or antigenic characteristics thereof. The markers can be detected using the agents that specifically interact with the markers at the nucleic acid level, particularly mRNA level and/or protein level, or the agents that specifically interact with the markers may also be used.

In other embodiment, the present biomarkers are based on its glycosylation characteristics. Thus each marker may be detected by quantifying the de-glycosylated level of the glycosylated peptides of each biomarker and the level of non-glycosylated peptide.

In this perspective, in the present disclosure there is provided a kit or composition which comprises nucleic acid sequences or the sequences complementary thereto or the fragments thereof, or antibodies or fragment thereof, an aptamer, an avidity multimer, a peptidomimetics specifically recognizing the biomarker or the protein encoded by the nucleic acid sequence; or a regent or agent to detect de-glycosylation or non-glycosylation peptides comprised in each of the biomarkers.

The quantitative and qualitative analysis of the makers for HCC diagnosis according to the present disclosure may be done by various methods known in the art that can detect the nucleic acid molecules or proteins quantitatively or qualitatively. For example, the methods include a western blot, an ELISA, a radioimmunoassay, an immunodiffusion, an immunoelectrophoresis, an immunostaining, an immunoprecipitation, a complement fixation assay, an array system such as a nucleic acid array or a protein array such as antigen array, a nucleic acid transcription and amplification system, eTag system, a system based on tailed beads, a binding with a tailed antibody in solution/suspension and a detection by flow cytometry, or a mass spectrometry, and the like. These methods are known and documents such as chip-based capillary electrophoresis: Colyer et al. 1997. J Chromatogr A. 781(1-2):271-6; mass spectroscopy: Petricoin et al. 2002. Lancet 359: 572-77; eTag systems: Chan-Hui et al. 2004. Clinical Immunology 111:162-174; microparticle-enhanced nephelometric immunoassay: Montagne et al. 1992. Eur J Clin Chem Clin Biochem. 30:217-22 may be referred.

In one embodiment of the present disclosure, a mass spectrometry is used for detecting the present markers, wherein the proteins are extracted from the appropriate samples and analyzed using the method such as described in the Examples of the present disclosure, and in the literatures Kim, et al. 2010 J Proteome Res. 9: 689-99; Anderson, L et al. 2006. Mol Cell Proteomics 5: 573-88 may also be referred. In one embodiment Multiple Reaction Monitoring (MRM) technology utilizing Triple Quadrupole LC-MS/MS and QTRAP and the like may be used. MRM is a method for exactly quantifying multiple markers present in biological samples in minute amount. In MRM, by a first mass filter (Q1), parent or precursor ions are selected from the ion fragments generated in ionization source and transferred to a collision cell. And then the precursor ions arrived at the collision cell collide with internal collision gas, and are fragmented into products or daughter ions and transferred to a second mass filter (Q2), from which only the specific ions are delivered to a detector. In this way only the information of the desired target can be obtained with high selectivity and sensitivity. The literature Gillette et al., 2013, Nature Methods 10:28-34 and the like may be referred.

The present biomarkers each comprise de-glycosylated peptides and non-glycosylated peptides as disclosed in Table 17-1 to 17-3, the amount or level of which or the ratio therebetween may be determined using the agents comprised in the present composition or kit.

In the present disclosure, the amount of non-glycosylated peptides determined may represent the expression level of the corresponding glycoproteins. The amount of the de-glycosylated peptides determined may represent the glycosylation level of the corresponding glycoproteins. In one embodiment, biomarkers from a normal sample and a HCC patient are analyzed in 3 types of peptides (non-glycosylated peptides, glycosylated peptides and ratio of glycosylated peptides/non-glycosylated peptides). This leads to an analysis with high discrimination power.

For example, when a particular protein shows a big difference in the protein expression level between a normal and HCC patient sample, then the protein expression level itself (non-glycosylated peptides) may be used alone to diagnose HCC. When the level of glycosylation (de-glycosylated peptide) or the ratio (de-glycosylated peptides/non-glycosylated peptides) shows a difference greater than the expression level, then the level related to glycosylation and de-glycosylation may be used alone or in combination with the expression level of the biomarkers. E no

For example, in case of SERPINF2, the non-glycosylated peptide (AUC=0.796) and the de-glycosylated peptide (AUC=0.799) show a decreased level in HCC patient sample. However, the ratio therebetween i.e., de-glycosylated peptide/non-glycosylated peptide (AUC=0.501) shows no difference. Thus, in this case, the detection may be performed only for the non-glycosylated peptide or the de-glycosylated pepti

In case of APOB, the non-glycosylated peptide (AUC=0.707) shows an increase in HCC samples and the de-glycosylated peptide (AUC=0.526) shows no difference. In this case, although the non-glycosylated peptide alone may be analyzed for detecting the marker to detect HCC, the ratio therebetween, i.e., de-glycosylated peptide/non-glycosylated peptide (AUC=0.781) is preferably used since it has a better discrimination power in differentiating cancer patients and normal subjects.

That is, a particular protein level may be increased in a HCC patient compared to a normal subject, however the glycosylation level of which may be decreased. Also a particular protein level may be decreased in a HCC patient compared to a normal subject, however the glycosylation level of which may be increased. Thus, the expression level of a biomarker may be determined by determining the non-glycosylated peptide level, and the glycosylation level may be determined by the de-glycosylated peptide level. When the trends are changed, the discrimination between a normal and HCC sample can be improved by determining the ratio, i.e., de-glycosylated peptide level/non-glycosylated peptide level.

In one embodiment, the biomarker SERPINF2, SERPINA6, CLU, COL12A1, ERBB3, F11, FSTL1, ITGA2, KLKB1, LAMP2, LGALS3BP, and PLXNA1 are detected by the amount of de-glycosylated peptide, and the biomarker AM2, GLB1, BMP1, BCHE, GNS, GPR126, SERPIND1, ITGA6, ITGAM, ITGB2, KTN1, PTK7, ROBO4 and VTN are detected by the amount of the non-glycosylated peptide, and the biomarker APOB, CFH, CPN2, VCAN, F5, HYOU1, ITGA3, POSTN and TNC are detected by the ratio of the amount of the de-glycosylated peptide to the non-glycosylated peptide.

In one embodiment, the present biomarkers are detected using agents specifically recognizing the present biomarkers or mRNAs encoding the present biomarkers or a microarray comprising the same.

In a still other embodiment, an immunoassay using sandwich system like ELISA (Enzyme Linked Immuno Sorbent Assay), or RIA (Radio Immuno Assay) and the like may be used for quantitative and/or qualitative detection of the present markers. In this system, the biological samples are reacted with a first antibody fixed to a solid substrate/support such as a glass, a plastic (for example, polystyrene), polysaccharides, a bead, a nylon or nitrocellulose membrane or a microplate well to form a complex and the complex is then allowed to react with an second antibody that is usually labeled with agents that can be detected directly or indirectly such as radioactive substances like ³H or ¹²⁵I, fluorescent materials, chemiluminescent substances, hapten, biotin, or digoxygenin and the like. In some cases, the labeling materials are conjugated with an enzyme such as horseradish peroxidase, alkaline phosphatase, or maleate dehydrogenase that is able to produce colors or color changes or illuminate in the presence of appropriate substrates.

Other methods based on immune reaction may also be used. In other embodiment, an Immuno Electrophoresis such as an Ouchterlony plate, a Western blot, a Crossed IE, a Rocket IE, a Fused Rocket IE, or an Affinity IE, which can detect the markers simply by antigen-antibody reaction may be used. The agents or materials that may be used in the methods described above are known the art. For example, the markers may be detected through an antigen-antibody reaction, or a reaction with a substrate, nucleic acid or peptide aptamers, receptors or ligands that specifically recognize the present markers, or cofactors or using mass spectrometry. The agents or materials that bind or interact specifically with the markers of the present disclosure can be utilized by means of chip or with nanoparticles. The immunoassay or immunostaining methods as described above are disclosed in the following literatures : Enzyme Immunoassay, E. T. Maggio, ed., CRC Press, Boca Raton, Florida, 1980; Gaastra, W., Enzyme-linked immunosorbent assay(ELISA), in Methods in Molecular Biology, Vol. 1, Walker, J.M. ed., Humana Press, NJ, 1984 etc. The intensities of the signals generated by the immunoassay mentioned above are then analyzed, namely compared with the signals from appropriate controls for the determination whether the sample has HCC or not.

Each marker of the present disclosure can be used for diagnosis or prognosis of HCC by detecting the marker(s) quantitatively and/or qualitatively at nucleic acid level, particularly at mRNA level. Various methods known in the relevant art may be used for this purpose. For example, for the quantitative or qualitative detection of the marker(s) or the expression pattern thereof at mRNA level, a polymerase chain reaction (PCR) combined with cDNA synthesis, a RT-PCR (reverse Transcription PCR), a competitive RT-PCR, a real-time RT-PCR, a Nuclease Protection Assay (NPA) such as a RNase or a S1 nuclease assay, an in situ hybridization assay, a DNA microarray or chip or Northern blot and the like may be used, the methods of which are known in the art and also can be performed using commercially available kits. The skilled person in the art would be able to select appropriate methods for practicing the present invention. For example, the transcriptome size that exists in cells can be determined by a northern blot, which has an advantage of being able to use diverse probes. A NPA is useful for multi-marker analysis. An in situ hybridization is useful for localizing the transcriptome such as mRNA in cells or tissues. A RT-PCR is particularly useful when small amount of samples are employed.

The reagents for detecting the presence markers in the amount or for determining the pattern of mRNA via RT-PCR comprise, for example, a primer set and/or probe specific to the marker(s) of the present disclosure. The terms "primer" or "probe" refers to a nucleic acid sequences that can bind to a template via complementarity and have free 3'-hydroxy group that can serves as a starting point for polymerization using the template by reverse transcriptase or DNA polymerases. The reagents used herein can also be labeled with appropriate materials for the signal detection such as color forming agent, illuminating or fluorescent materials. In one embodiment, a northern blot or a reverse-transcription PCR is used for the mRNA detection. The latter is a method to detect or determine the expression level of a specific gene expression in a sample, wherein the RNA, particularly mRNAs are isolated from the sample and cDNAs are synthesized from the isolated RNA, and then the cDNAs are amplified using a specific set of primers, or a combination of primers and probe. Such methods to detect the presence/absence or quantity of a specific gene or marker may be found in for example, Han, H. et al, 2002. Cancer Res. 62: 2890-6.

In other aspect, the present disclosure provides a kit, composition or system for diagnosing or prognosis of hepatocellular carcinoma comprising an agent to detect the expression level or presence of at least one biomarker of the present disclosure. The detection agents and the methods of using the agent are described hereinbefore. The agents for detecting the present marker(s) can be provided in a container separated in sections, or can be provided separately. In this regards, the present disclosure provides a device that provides the agents for detecting the present markers in separate sections in a container.

In one embodiment, the detecting agents are antibodies specifically binding to one or more markers of the present disclosure, and can detect the protein in a sample such as serum quantitatively and/or qualitatively. In other embodiment, the antibodies may be provided as conjugated to a substrate or support, for example to a well of the multi-well plates or a surface of the glass slides or nitrocellulose filters.

In other aspect, the detecting agents which may be used for the present disclosure may be provided as a type of array such as microarray or chip, and the references for the array preparation technology may be found in for example, Schena et al., 1996, Proc Natl Acad Sci USA. 93(20):10614-9; Schena et al., 1995, Science 270(5235):467-70; and U.S. Pat. Nos. 5,599,695, 5,556,752 and 5,631,734. The detecting agents that can be provided as attached to an array include, but are not limited to, antibodies specifically recognizing the present marker, or antibody fragments, or aptamers, avimers (avidity multimer) or peptidomimetics.

The detecting agents may also be labeled for the direct or indirect signal detection via sandwich forms. In the case of direct labeling method, biological samples such as serum to be used for array may be labeled with a fluorescent material such as Cy3 and Cy5. In the case of indirect labeling, unlabeled samples are allowed to bind to the detecting agent which is attached to the array, and then the target or marker proteins are detected by the labeled antibodies which specifically recognize the marker or target. In the case of sandwich methods, the sensitivity and specificity of the detection is usually high and able to detect the marker at the pg/mL level. In addition, labeling agents including such as radioactive materials, agents that produce visible colors under proper condition, magnetic particles and high-density electron particle and the like may also be used.

For the detection of fluorescence signals, a scanning confocal microscopy may be used, which are available from for example, Affymetrix, Inc. or Agilent Technologies, Inc. and the like.

The kits or compositions of the present disclosure may also include one or more additional components as needed for the detection of the present marker(s), which include, for example, binding buffers, reagents for preparing biological samples, syringes for blood collecting or negative and/or positive controls.

The kits or compositions of the present disclosure in which various agents as described above may be contained may be provided for an ELISA, a dip stick rapid kit, a MRM, microarray, a gene amplification, or an immunoassay. The reagents which may be included for a particular kit may be appropriately selected from the known agents in view the present disclosure.

In one embodiment, an ELISA or a dip stick rapid kit is used, and in this case, the antibodies to detect the present markers may be provided as conjugated to a substrate/support, for example to wells of a multi-well plate or surface of a glass slide or a nitrocellulose paper. A dip stick is a form widely used in POCT (point of care technology) in which the biomarkers of the present disclosure may be detected by employing one or more antibodies that are conjugated to a substrate such as nitrocellulose paper which is then contacted with a sample such as serum for example by dipping one end of the stick to the serum and then the sample is moved through the substrate by a capillary action, and the markers are detected by a color that is developed when the markers of interest are bound by the antibody attached to the substrate.

In other embodiment, a MRM kit which is based on peptide analysis is provided. MRM analysis may be performed or used as described above. The MRM is a method using the peptides that selectively recognize a particular protein, and can detect the markers in biological samples in a more stable manner compared to the existing methods that employ antibodies that are sensitive to environmental conditions such as temperature and humidity and the like.

Also, the kits of the present disclosure may also include instructions for using the kit to detect the markers according to the present disclosure.

In still other aspect, the present disclosure provides methods for diagnosing or prognosis of HCC, or detecting in vitro at least one maker of the present disclosure for diagnosis or prognosis of HCC, which comprises detecting the presence or measuring the amount at a nucleic acid and/or protein level, or the glycosylation level of at least one biomarker selected from the group consisting of AFP, SERPINF2, A2M, APOB, GLB1, BMP1, SERPINA6, CFH, BCHE, CLU, COL12A1, CPN2, VCAN, ERBB3, F5, F11, AFP, FSTL1, GNS, GPR126, SERPIND1, HYOU1, ITGA2, ITGA3, ITGA6, ITGAM, ITGB2, KLKB1, KTN1, LAMP2, LGALS3BP, PLXNA1, POSTN, PTK7, ROBO4, TNC and VT; and comparing the detection result to that of a corresponding marker from a control sample wherein the change in the concentration and/or presence of the biomarker at a nucleic acid and/or protein level or in the glycosylation level in the subject or the sample from the control indicates the presence of HCC in the subject or the sample. The subject or sample can be diagnosed accordingly based on the detection result.

Samples such as biological samples, control or reference groups employed in the present methods, methods for determining the present biomarkers and the reagent used therefor, and methods for data analysis required for the interpretation or diagnosis are as described hereinbefore and in the following Examples.

In one embodiment of the present disclosure, each the present biomarker comprises de-glycosylated and non-glycosylated peptides as disclosed in Table 17-1 to 17-3. Then, in the detection step, the glycosylation level is determined, which may be performed by determining at least one of the amount of the de-glycosylated peptides, the amount of the non-glycosylated peptides or the ratio therebetween. In the present methods, SERPINF2, SERPINA6, CLU, COL12A1, ERBB3, F11, FSTL1, ITGA2, KLKB1, LAMP2, LGALS3BP, and PLXNA1 are detected by determining the level of de-glycosylated peptides, and AM2, GLB1, BMP1, BCHE, GNS, GPR126, SERPIND1, ITGA6, ITGAM, ITGB2, KTN1, PTK7, ROBO4 and VTN are detected by determining the level of non-glycosylated peptides. And APOB, CFH, CPN2, VCAN, F5, HYOU1, ITGA3, POSTN and TNC are detected by determining the ratio therebetween.

In one embodiment, the level of the de-glycosylated peptides and the non-glycosylated peptides are determined by Liquid chromatography using MRM. The de-glycosylated peptides and the non-glycosylated peptides are as described hereinbefore. Also the de-glycosylated peptides and the non-glycosylated peptides for each biomarker of the present disclosure are as disclosed in Table 1. In one embodiment, for each biomarker, the level of the de-glycosylated peptides and the non-glycosylated peptides in Table 1 are determined using for example LC using MRM. Then the ratios therebetween are determined, which are then used to diagnose HCC based on the changes in the ratio compared to the value obtained from a control sample.

**[Table 1]**

| **Protein Marker** | **De-glycosylated peptide** | **Non-glycosylation peptide** |
|---|---|---|
| AFP | VDFTEIQ | GYQELLEK |
| SERPINF2 | NPDPSAPR | LGNQEPGGQTALK |
| A2M | VSDQTLSLFFTVLQDVPVR | AIGYLNTGYQR FEVQVTVPK IAQWQSFQLEGGLK NEDSLVFVQTDK VSVQLEASPAFLAVPVEK |
| APOB | FEVDSPVYDATWSASLK | LSLESLTSYFSIESSTK |
| GLB1 | NNVITLDITGK | VNY GAYINDFK |
| BMP1 | IILDFTSLDLYR | GIFLDTIVPK |
| SERPINA6 | AQLLQGLGFDLTER | ITQDAQLK WSAGLTSSQVDLYIPK |
| CFH | SPDVIDGSPISQK | SSIDIENGFISESQYTYALK |
| BCHE | WSDIWDATK | AILQSGSFNAPWAVTS- LYEAR lFFPGVSEFGK YLTLNTESTR |
| CLU | LADLTQGEDQYYL | ASSIIDELFQDR EIQNAVNGVK |
| COL12A1 | NVQVYDPTPNSLDVR | ITEVTSEGFR VQISLVQYSR VYDPSTSTLNVR |
| CPN2 | AFGSNPDLTK | LELLSLSK |
| VCAN | VVAEDITQTSR | LLASDAGLYR TDGQVSGEAIK |
| ERBB3 | NLDVTSLGFR | LAEVPDLLEK |
| F5 | TWDQSIALR | ASEFLGYWEPR |
| F11 | LSSDGSPTK | VVSGFSLK |
| FSTL1 | GSDYSEILDK | LSFQEFLK |
| GNS | YYDYTLSINGK | AFQNVFAPR |
| GPR126 | SLSSSSIGSDSTYLTSK | ISVVIQNILR VILPQTSDAYQVSVAK |
| SERPIND1 | DFVDASSK | EYYFAEAQIADFSDPAFISK NYNLVESLK SVNDLYIQK TLEAQLTPR |
| HYOU1 | VFGSQDLTTVK VIDETWAWK | DEPGEQVELK |
| ITGA2 | YFFDVSDEAALLEK | FGIAVLGYLNR |
| ITGA3 | DITIVTGAPR | TVEDVGSPLK |
| ITGA6 | LWDSTFLEEYSK | LPNAGTQVR |
| ITGAM | EFDVTVTVR | ILVVITDGEK |
| ITGB2 | LTDNSNQFQTEVGK | ALNEITESGR |
| KLKB1 | GVNFDVSK | DSVTGTLPK IAYGTQGSSGYSLR YSPGGTPTAIK |
| KTN1 | TEDSSLTK | LQTLVSEQPNK |
| LAMP2 | VQPFDVTQGK | GILTVDELLAIR |
| LGALS3BP | ALGFEDATQALGR | ELSEALGQIFDSQR SDLAVPSELALLK YSSDYFQAPSDYR |
| PLXNA1 | YDYTEDPTILR | LSLPWLLNK |
| POSTN | EVDDTLLVNELK | IIDGVPVEITEK |
| PTK7 | SADASFNIK | SSLQPITTLGK |
| ROBO4 | DLSQSPGAVPQALVAWR | GPDSNVLLLR |
| TNC | LLETVEYDISGAER | APTAQVESFR |
| VTN | DGSLFAFR | DVWGIEGPIDAAFTR FEDGVLDPDYPR |

The subject to be diagnosed by the present methods is a mammal, particularly human. The subjects include ones who are suspected of HCC or at high risk or others who need for HCC diagnosis or prognosis. In one embodiment, the present methods are applied for a subject who has symptoms indicative of liver related disease, such as an abdominal pain, hepatomegaly, ascites, jaundice, muscle wasting, hepatitis(for example HCV infection), or esophageal varix and the like. In other embodiment, the subject includes ones who appear to be normal and yet to develop HCC related symptoms.

In other embodiment, the subject may be a person whose concentration of serum AFP is low or in a normal range and thus not diagnosed as HCC by applying AFP criteria. In this case, the serum concentration of AFP may be about 0 µg/l (not detected) to 20 µg/l, e.g., 0 µg/l (not detected) to 5 µg/l, 5 µg/l to 10 µg/l, 10 µg/l to 15 µg/l, or 15 µg/l to 20 µg/l.

When the combinations of two or more markers are used for the present methods, a profile, namely a set of data including quantitative information in relation to the protein expressions in the sample of interest may be generated.

When the profiles are obtained using the present markers, HCC in the sample of a subject may be diagnosed or determined by comparing the result of the sample with that of the control or reference groups. The control or reference groups which may be used for the present methods include a negative control from a healthy or normal subject, or from a subject cured of HCC, and a positive control is from HCC patient diagnosed not by the present method but by the other methods, or from liver cirrhosis patient, or from hepatitis patient.

In one embodiment, as a control or reference groups, normal samples or samples from HCC-treated patient are used and compared with the acquired profile from the subject who needs diagnosis.

For comparing the profiles obtained, methods known in the art may be used. For example, comparison of the digital images of expression profiles, comparison using expression database, or US Patent NOs 6,308,170 and 6,228,575 may be referred.

The acquired profiles using the present markers may be processed by data analysis methods known in the art. For example, the methods include but are not limited to nearest neighbor classifier, partial-least squares, SVM, AdaBoost and clustering-based classification. Or the methods described in the literatures such as Ben-Dor et al (2007, J. Comput. Biol. 7: 559-83), Nguyen et al (2002, Bioinformatics 18:39-50), Wang et al (2003, BMC Bioinformatics 4:60), Liu et al (2001, Genome Inform. Ser. Workshop Genome Inform.12:14-23), Yeang et al (2001, Bioinformatics 17 Suppl 1:5316-22) and Xiong (2000, Biotechniques 29(6):1264-8, 1270) and the like may also be referred.

Further, various statistical analysis methods may be employed to calculate the significance of the data obtained using the present markers to diagnose or prognosis HCC. In one embodiment, a logic regression method is used for a statistical analysis and the literatures such as Ruczinski, 2003, Journal of Computational and Graphical Statistics 12:475-512 may be referred. A logic regression method is similar to CART method in which classifier is presented as a binary tree, but Boolean operators are used for each node that is more general and related to characteristics than "and" operator used in CART. There are other analysis methods such as nearest shrunken centroids (Tibshirani. 2002 PNAS. 99:6567-72), random forests (Breiman. 2001. Machine Learning 45:5-32 and MART (Hastie. 2001. The Elements of Statistical Learning, Springer).

In one embodiment, the confidence level of a significant difference between the test sample and the controls to diagnose the sample as HCC may be determined in a statistical analysis. The raw data used for a statistics are the values analyzed in duplicate, triplicate or more for each marker.

This statistical analysis is useful to decide the clinical significance of the clinical and genetic data as well as the data produced from the biomarkers.

Also the present methods may be used to determine the severity of the HCC. For example, it may be diagnosed as a mild HCC, a moderate HCC or a severe HCC by comparing the data obtained with the profiles form the positive and/or negative control groups. Further, the marker profiles obtained from a certain HCC groups may be classified according to a certain criteria. In this way, it is possible to find the disease at the early stage so that expensive test such as magnetic resonance imaging (MRI) may not be needed.

In other embodiment, the present method may be performed multiple times with a certain interval over a period of time for example over one year to monitor the changes in the expression pattern of the present markers. The increase or decrease in the expression depending on the markers can be used to relate the sample with a particular status or severity of HCC. Also in one subject, the data may be used to determine the development, progression, and/or aggravation of HCC over time in comparison to the previous data from the same subject or to the data from the controls. The changes in the expression pattern or levels over time thus can be used to treat HCC or to prevent the progression of HCC into a more advanced stage. Also the present methods may be used together with conventional diagnosis methods such as AFP test, a ultrasonography, a computerized axial tomography(CT scan) or a magnetic resonance imaging (MRI).

The present disclosure is further explained in more detail with reference to the following examples. These examples, however, should not be interpreted as limiting the scope of the present invention in any manner.

### EXAMPLES

### EXAMPLE 1. Analysis of de-glycosylated/non-glycosylated peptides using a standard glycoprotein

The following experiments were performed using a standard protein to confirm the possibility of discovering or developing markers based on the quantification of glycosylated peptides and de-glycosylated peptides using MRM technology and its use in diagnostics.

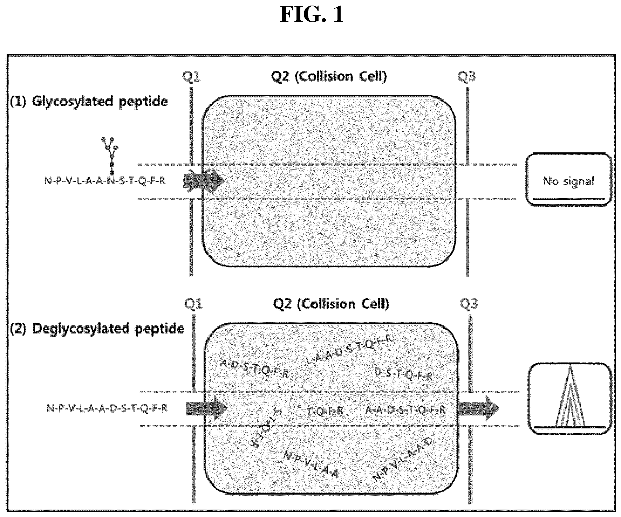

As shown in Fig. 1, MRM is a technology to quantify a relative or an absolute amount of proteins in biological fluids using Triple quadrupole as a mass spectrometry. MRM includes a first mass filter Quadruple 1 (Q1) filtering only the peptides with a specific m/z (mass/charge), Quadruple 2 (a collision cell) in which the peptides from Q1 are fragmented by electric energy and Quadruple 3 (Q3) which transmits only particular fragmented peptide ions. Then the ions transmitted through Q3 are shown as a peak of chromatogram at the detector. The area of the peak is calculated for the absolute or relative quantification of peptides. In case of glycosylated peptides, there are changes in the original mass of the peptide due to the glycan present in the glycosylated peptides. As a result, the glycosylated peptides cannot pass through a Q1 filter at the m/z value of the corresponding peptide and fails to enter into Q2 collision cell. Thus, the glycosylated peptides are not detected. In contrast, when the glycosylated peptides are de-glycosylated by treating them with a de-glycosylating enzyme such as PNGase-F, the glycans are removed at the N-glycosylation site (NxS/T) by which Asn (Asparagine, N) is changed to a deaminated form, i.e., Asp (Aspartic acid, D). The de-glycosylated peptides can thus be detected as a deaminated form of the peptide based on such principle.

### EXAMPLE 1-1 Selection of standard glycoproteins

Among the commercially available proteins which are purified and lyophilized, a protein in which both the glycosylated peptide having NxS/T motif and the non-glycosylated peptide without the motif are detected as predictable transitions in Skyline program when selecting theoretical Q1/Q3 transition has been used as a standard.

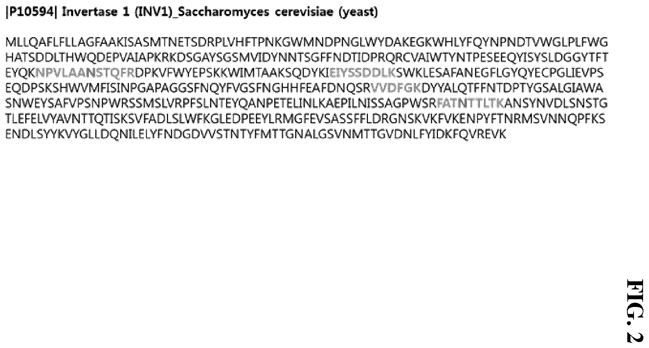

In the present example, Invertase-1 protein was used as a standard and the sequence is as shown in Fig. 2 in which the green indicates the sequence used in the analysis. The sequence of the standard glycosylated peptides 1 and 2 employed are NPVLAANSTQFR and FATNTTLTK, respectively. When the peptides are de-glycosylated, Asn residues are converted to Asp resulting in the peptide sequence of NPVLAADSTQFR and FATDTTLTK, respectively. The standard non-glycosylated peptides 1 and 2 employed are IEIYSSDDLK and VVDFGK, respectively.

### EXAMPLE 1-2 Selection of the theoretical transition (Q1/Q3) of the standard glycoprotein.

The native form of the sequence of the standard protein and the conversion form thereof in which N is changed to D at NxS/T motif were imported into Skyline (https://brendanx-uw1.gs.washington.edu/labkey/project/home/software/Skyline/begin.view) program to select a theoretical transition value. At the same time, synthetic peptides with the same sequence except that ¹²C and ¹⁴N atoms in Arg (R) and Lys (K) residues at the C-terminal region were heavy labelled with ¹³C and ¹⁵N were used to confirm that the peptides detected are actually from the peptides of interest to be detected.

That is, the heavy labelled peptides and the endogenous peptides share the same sequence and thus have the identical hydrophobicity. Thus they can be detected on LC-column (C18) since they are eluted at the same retention time.

As a result of the selection (Q1/Q3), Q1 difference of 0.49 Da and Q3 difference of 0.98 Da between the native and conversion sequence have been found. The difference between the endogenous peptide and the heavy labelled peptide were found to be 4.00 Da (5.00 Da) in Q1 and 8.01 Da (10.01 Da) in Q3. The transition analysis results are as below in Table 2.

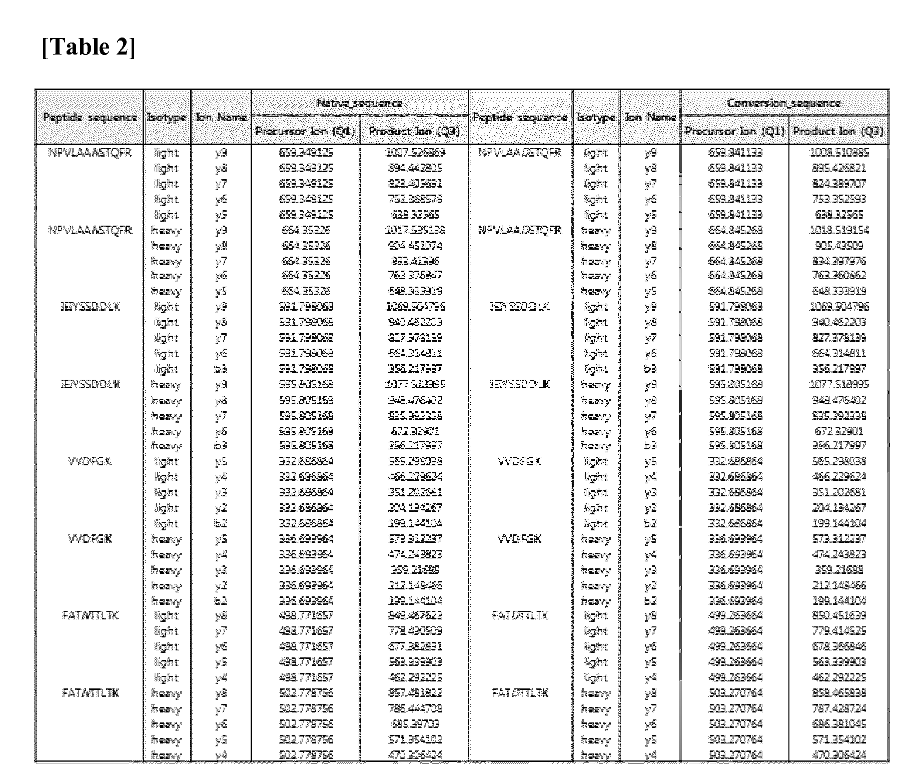

### EXAMPLE 1-3 MRM analysis of the standard glycoprotein

### 1-3-1 Preparation of the standard glycoprotein

Hundred µg of standard protein was treated with urea and DTT at the final concentration of 6M urea/20mM DTT(dithiothreitol) in Tris pH 8.0 and reduced at 37°C for 60min. Then the product was alkylated with IAA (iodoacetamide) at the final concentration of 50mM at RT for 30 min. Then the product was diluted with 100mM Tris pH 8.0 to bring the concentration of Urea not more than 0.6M. Then the de-glycosylated peptides were treated with 2µl (500,000 units/ml) of PNGase-F (Peptide N Glycosidase) (NEW ENGLAND BioLabs Inc. P0704L) and incubated at 37 °C for 16 hrs, which were then treated with trypsin at a ratio of 1:50 (w/w) trypsin to peptides and incubated at 37 °C for 12 hrs. Then the resulting products were treated with a formic acid solution at the final concentration of 5%. And as a control the glycosylated peptides was treated with 2µl of water under the same condition. Then desalting reaction was performed as follows using OASIS cartridge (Waters, USA) as suggested by the manufacturer's instruction. The desalted peptides were dissolved in Sol A buffer (97% D.W, 3% ACN, 0.1% formic acid) followed by a centrifugation at 15,000rpm for 60 min, and then used for MRM analysis.

### 1-3-2 Preparation of sample for MRM analysis

To confirm the possibility of the quantification, experiments to confirm the linearity of the heavy labelled synthetic peptide to the target peptide were performed as follows. For this, a serious dilution of 0, 4, 13, 40, 120, 370 fmol of the heavy labelled synthetic peptide was prepared, to which a 370nmol of the target peptide corresponding to the standard glycoprotein was added for the analysis. For the standard glycoprotein sample, heavy labelled synthetic peptide of N-form having Asn residue was used. For the de-glycosylated standard glycoprotein sample, heavy labelled synthetic peptide of D-form having Asp residue was used. All the experiments were repeated 3 times.

### 1-3-3 Condition for MRM analysis

Liquid chromatography (LC) 1260 capillary LC system from Agilent was used. For the peptide separation, Capillary RR 0.3 x 150, 3.5µm (Cat.N 5064-8261) was used. Five microliter of peptide sample was directly injected into the column without passing through a trap column and eluted at a flow rate of 20L/min.

Column was equilibrated with SolA (97% Distilled Water, 3% acetonitrile, 0.1% formic acid) for 10 min and eluted with SolB (3% Distilled Water, 97% acetonitrile, 0.1% formic acid) in 45 min and then on a linear gradient of 5% to 60% and of 85% in 5 min.

Mass spectrometry 6490-Triple quadrupole (QQQ) from Agilent technology was used to monitor the transition of the selected protein under MRM mode. The settings were as follows: gas temperature of 200°C, gas flow of 14L/min, nebulizer at 20psi, sheath gas temperature of 250°C and sheath gas flow of 11L/min. The voltage applied for the capillary and nozzle was 3000V.

The unit resolution of 0.7Da was used for Quadruple 1(Q1) and Quadruple 3 (Q3). The dwell time was set to 2 sec for a total cycle in an unscheduled MRM mode. Then the retention time was selected after the analysis for all the target peptides were completed, based on which the analysis were repeated 3 times in MRM scheduled at the window size 3 min.

### 1-3-4 MRM analysis results

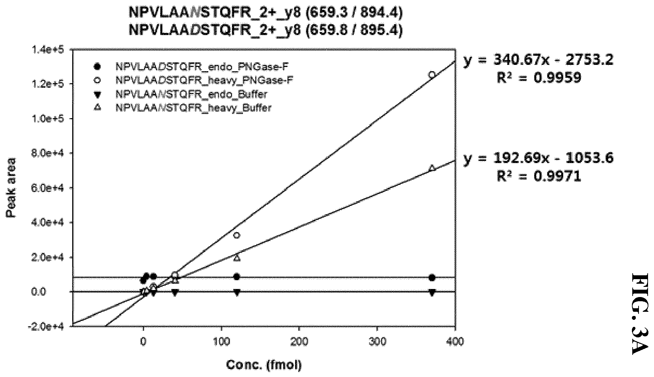

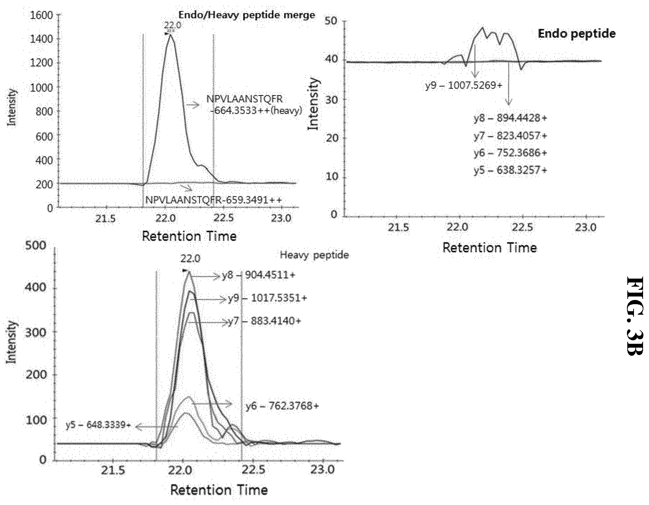

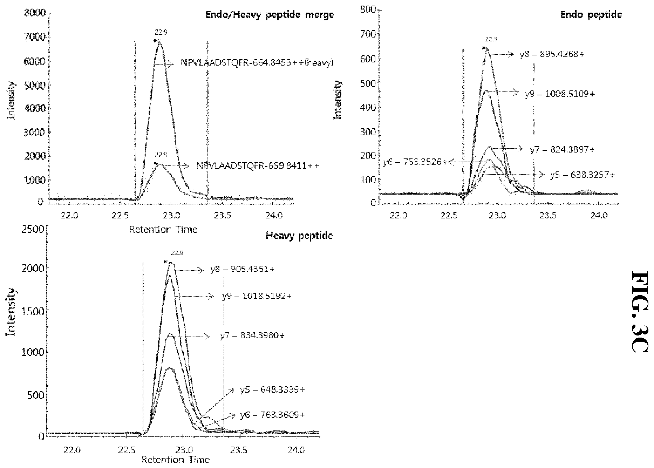

The results of MRM analysis for the standard glycoprotein are shown in Figs. 3 to 7.

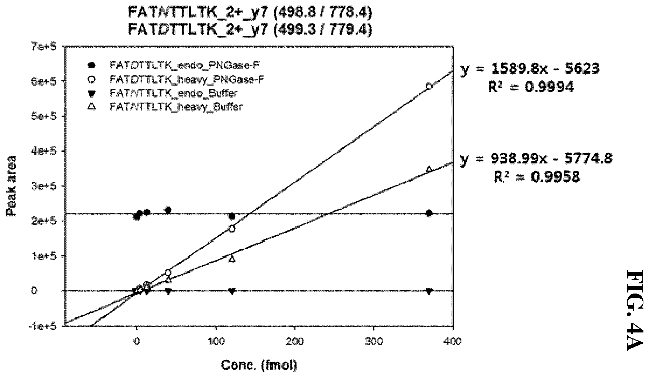

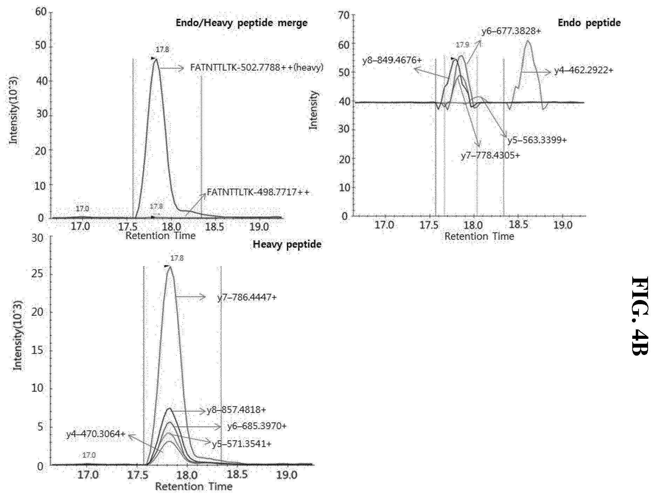

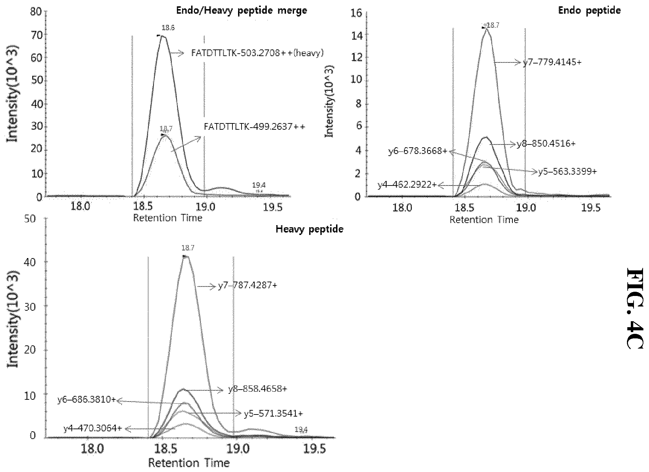

Results of the analysis performed on NPVLAANSTQFR (the glycosylated peptide 1) / FATNTTLTK (the glycosylated peptide 2) are shown in FIGs. 3 and 4, Tables 3-1 and 3-2 and Tables 4-1 and 4-2. When the serially diluted heavy labelled synthetic peptide in D-form was added to the de-glycosylated sample, it was confirmed that the endogenous peptide from the standard glycoprotein and the heavy labelled peptide were co-eluted at the identical time. Also the strength of five product ion types were confirmed to be identical. And the linearity (R^2=0.9959, 0.9994) of the heavy labelled synthetic peptide was confirmed. When the serially diluted heavy labelled synthetic peptide in N-form was added to the glycosylated sample, the endogenous peptide from the standard glycoprotein was not observed and only the heavy labelled peptide was detected. The linearity of R^=0.9971, 0.9958 was found.

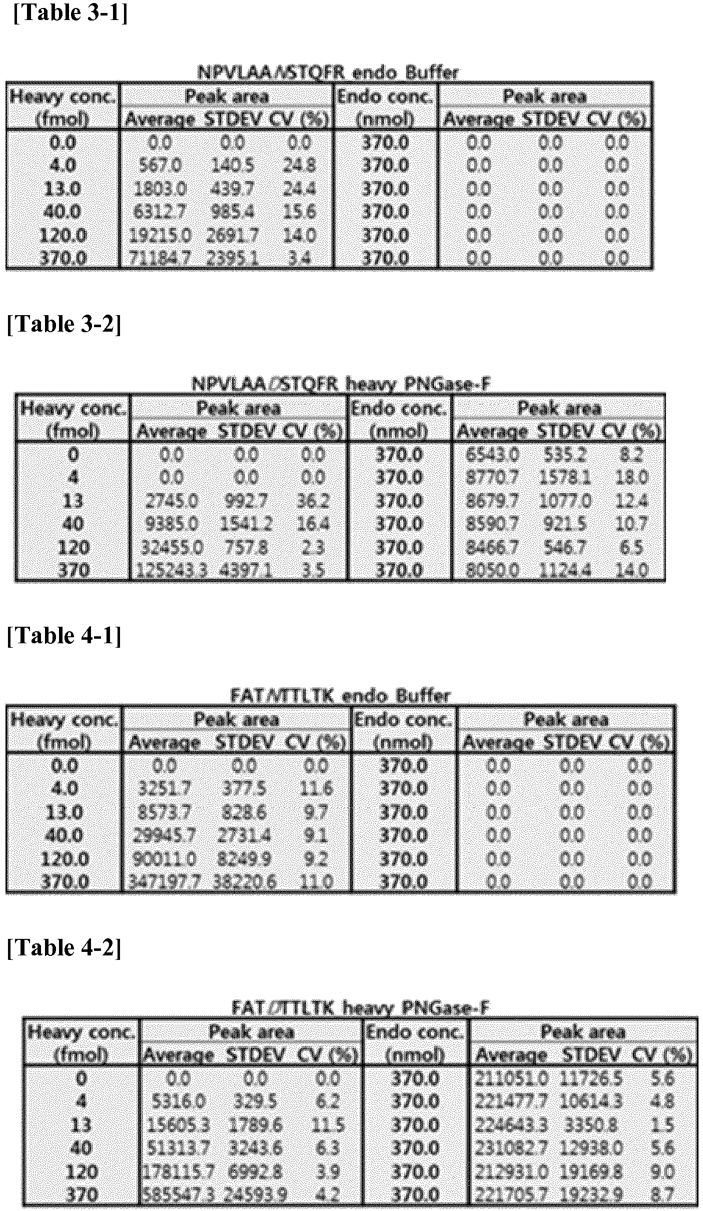

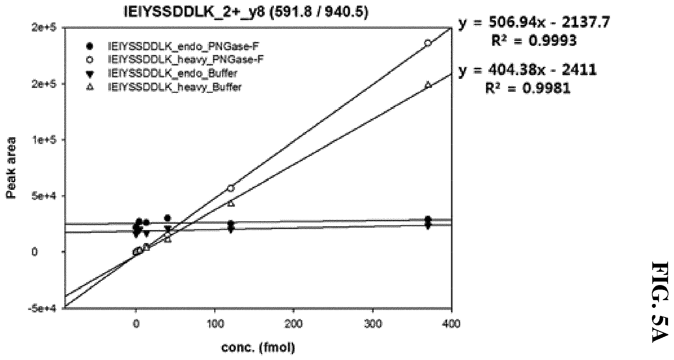

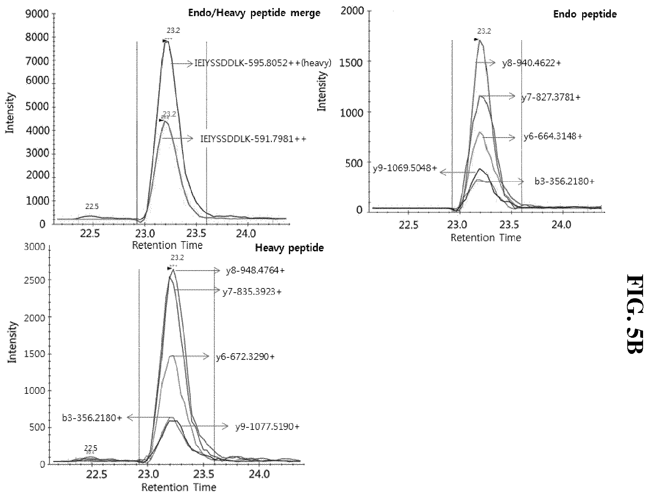

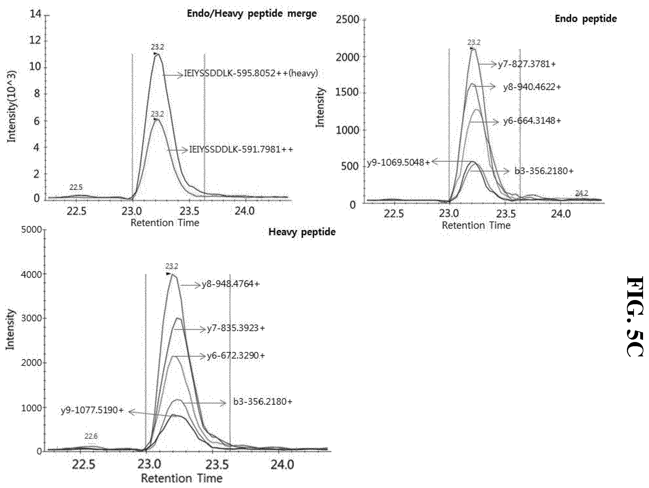

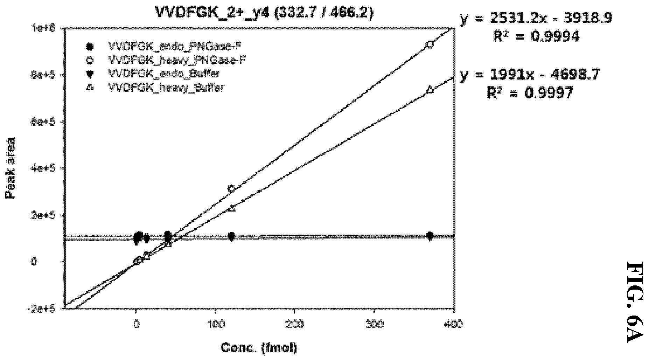

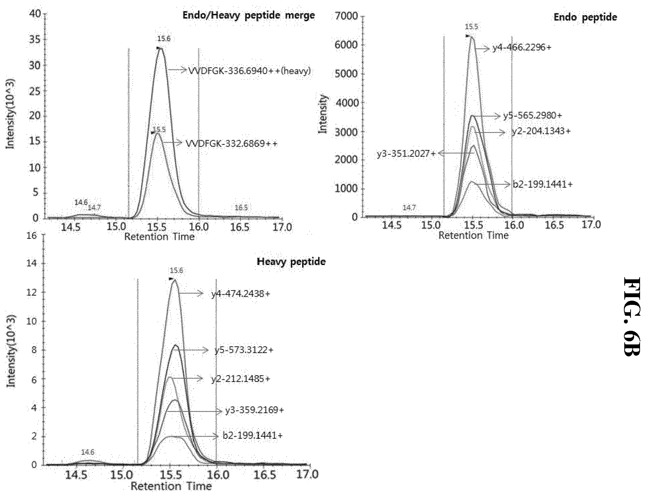

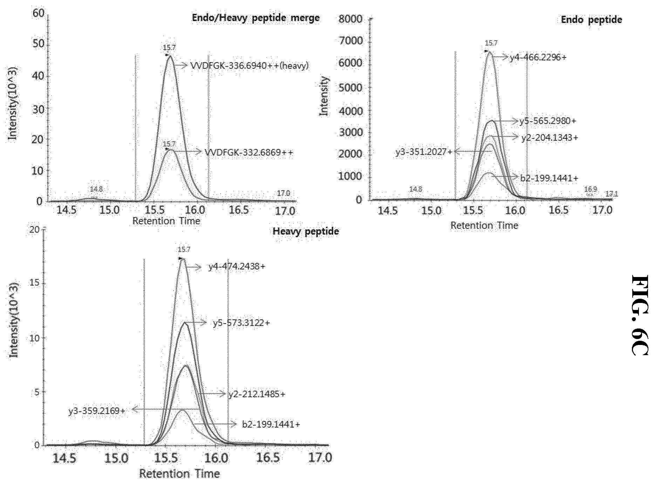

Results of the analysis performed on the non-glycosylated peptides IEIYSSDDLK / VVDFGK in which the de-glycosylated sample were prepared by PNGase-F treatment and the non-glycosylated peptides were prepared by water (control) are shown in Figs. 5 and 6, Tables 5-1 and 5-2, and Tables 6-1 and 6-2.

When the serially diluted heavy labelled peptide was added to the de-glycosylated and the glycosylated samples, the endogenous peptide from the standard glycoprotein and the heavy labelled peptide were co-eluted at the identical time. Also the strength of five product ion type was confirmed to be identical. The linearity of the heavy labelled synthetic peptide was confirmed to be R^2=0.9993, 0.9994 / R^2=0.9981, 0.9997. Further the endogenous peptides was found to have a strength that is lower than the de-glycosylated sample treated with PNGase-F.

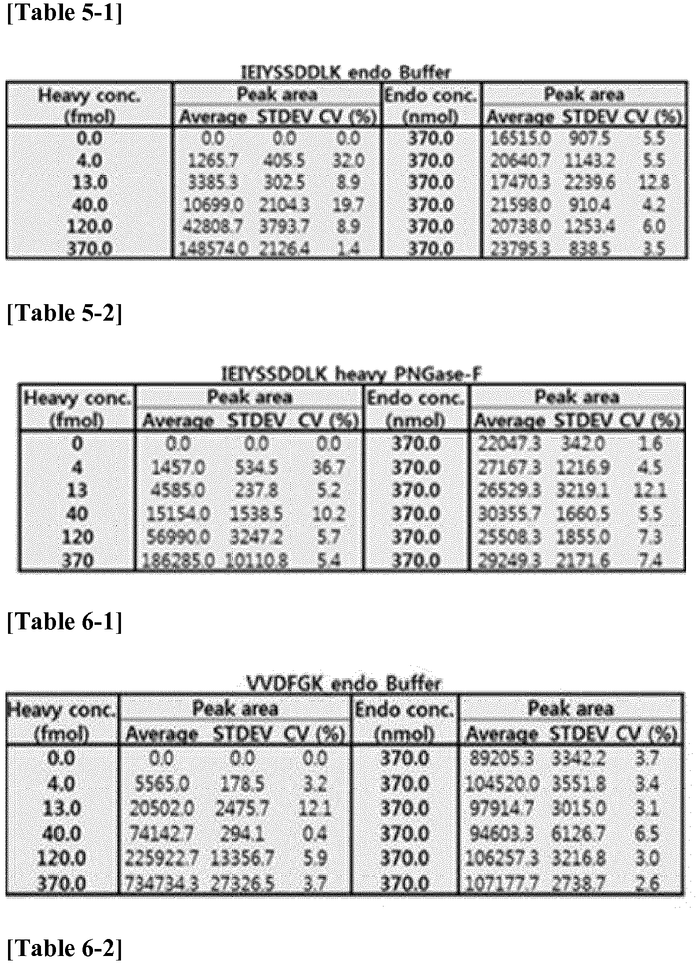

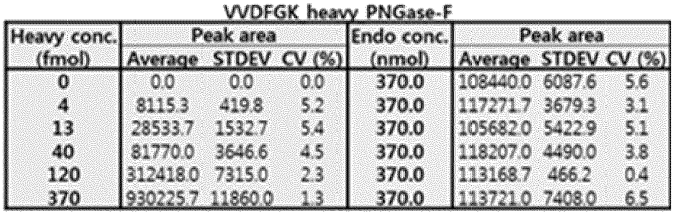

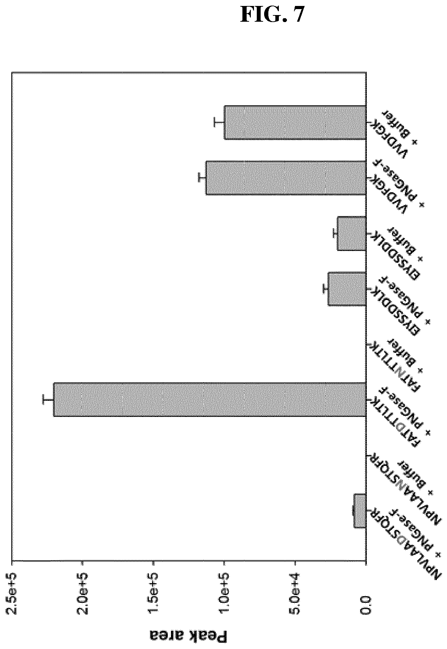

Summarizing the results of the experiments using the standard glycoprotein, the de-glycosylated peptide can be detected only when analyzed in D-form in MRM analysis as shown in Fig. 7; however, the glycosylated peptide cannot be detected in a native N-form because of the mass changed due to the presence of glycan. This indicates that the glycosylated peptides can be used successfully to quantify the glycoproteins in biological samples. In case of the non-glycosylated peptides, the peak intensity was found to be increased in the de-glycosylated sample treated with PNGase-F compared to the glycosylated sample. This is due to that the steric hindrance by the glycans was disappeared by PNGase-F which removes the glycans from the peptides thus facilitating the access of trypsin to the target peptides.

### EXAMPLE 2. MRM analysis of the glycoproteins in liver cancer sample.

### EXMAPLE 2-1 Clinical information of the sample

The institutional review board of Seoul National University Hospital approved the protocol of the present invention, and the written informed consent was obtained from each patient or their legally authorized representative. The clinical characteristics of the patients are as in Table 7.

In the present examples, 60 normal and 60 liver cancer samples were used. The samples were selected to include more samples from men than women in consideration of the higher ratio of liver cancer found in men than women. Although liver cancers are classified into virus origin (HBV, HCV) and alcoholic origin, only the liver cancer samples of HBV origin were selected in consideration of the fact that HBV is the highest cause of liver cancer in Asia and Africa.

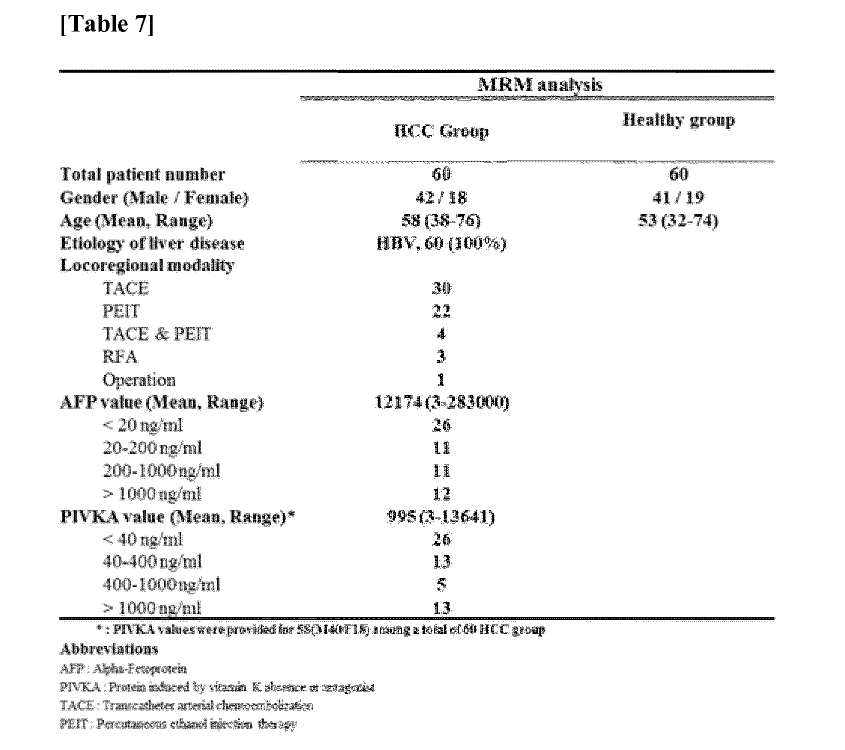

### EXAMPLE 2-2 Selection of biomarkers using clinical samples

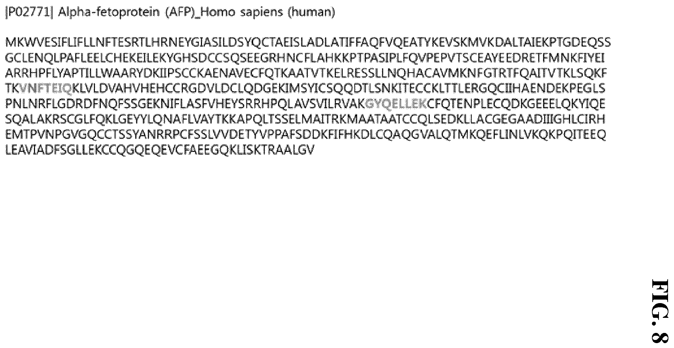

In case of alpha-fetoprotein (AFP) known as a biomarker for liver cancer, the peptide sequence in which the NxS/T motif is glycosylated is VNFTEIQ. The glycosylated peptide comprising NxS/T motif and the non-glycosylated peptide without the motif were analyzed using Skyline program to determine the possible transition (refer to Example 2-3). The sequence of the full-length is shown in Fig. 8, in which the green indicates the sequence used in the analysis.

To further discover the potential glycosylated protein markers specific for liver cancer, 495 glycosylated proteins which contain NxS/T motif(s) and are known to be N-glycosylated at the motif were selected from Plasma Proteome Database (PPD). The transition was determined using Skyline program for the peptides containing the motif and being glycosylated and for the non-glycosylated peptide without the motif. Through this process, a total of 406 proteins, 1637 peptides, and 9821 transitions (Q1/Q3) were selected for the non-glycosylated peptides. For the glycosylated peptides, a total of 240 proteins, 363 peptides, and 4111 transitions (Q1/Q3) were selected.

### EXMAPLE 2-3 Determination of theoretical transition (Q1/Q3) of the glycosylated proteins including AFP from liver cancer

As described in Example 1 for the analysis of standard glycoprotein, AFP protein in a native form and conversion form in which N is substituted with D were imported into Skyline program to determine the theoretical transition (in silico prediction). As a result, Q1 and Q3 differences between the two types of peptides were found to be 0.49 Da and 0.98 Da, respectively. Results are shown in Table 8. Other glycoproteins in Example 2-2 from liver cancer were analyzed in the same way.

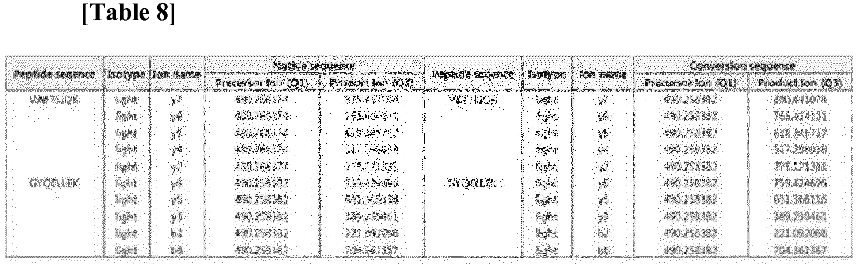

### EXAMPLE 2-4 Preparation of pooled clinical samples and MRM analysis

Each of sixty normal control and HCC patient samples were pooled into three groups by twenty samples. Major six proteins in serum (albumin, IgG, IgA, transferrin, haptoglobin, alpha-1-antitrypsin) were removed using MARS (Part #5185-5984, multiple affinity removal system, Agilent Technologies, USA) according to the manufacturer's instruction.

Then the serum proteins were concentrated using a filter (3K Amicon, USA) and quantified using BCA (bicinchoninic acid (BCA) assay, Sigma-Aldrich, USA) kit according to the manufacturer's instruction. Then 100µg of the protein was treated either with water (control) or PNGase-F to de-glycosylate the protein followed by treatment with trypsin.

For quality control of the data obtained and the stability confirmation of the instrument, peptides in which C and N atoms of arginines are heavy labelled were used as an internal standard. The peptide sequence used is LNVENPK from *E.coli* and thus not present in human serum, which was used at the concentration of 5 fmol per analysis.

Experiments were repeated 3 times per group and then the data obtained were imported into Skyline and converted into the transition area for each peptide. Then the peak area obtained from the AFP target peptide were normalized by the peak area obtained from the heavy labelled internal standard.

### EXAMPLE 2-5 Results of MRM analysis of pooled clinical samples

### 2-5-1 AFP peptide (GYQELLEK / VDFTEIQ) and results of analysis for discovering potential liver specific glycosylated protein markers

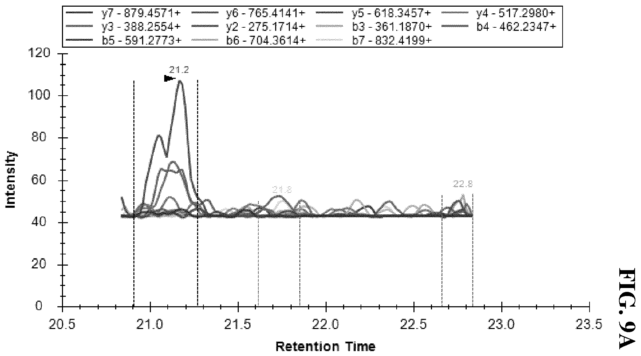

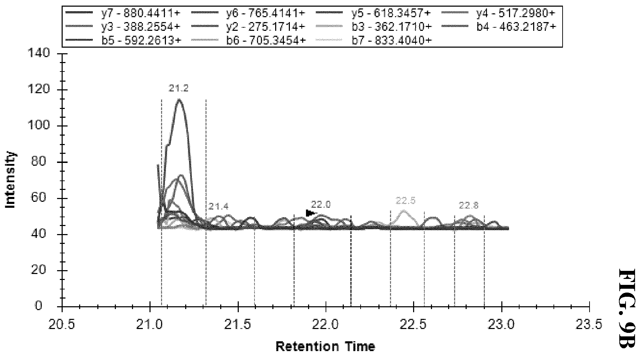

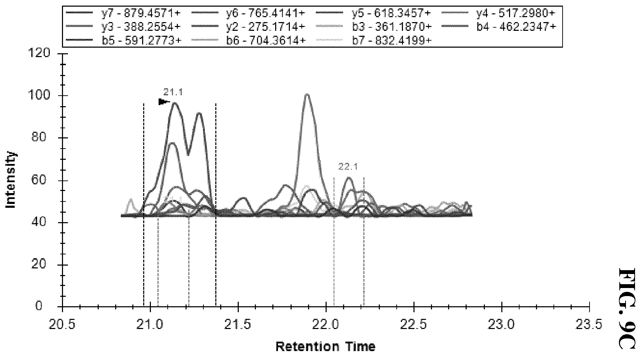

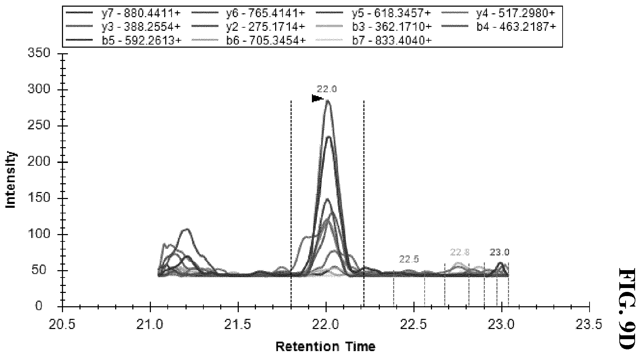

Results from the experiments in which the control glycosylated sample treated only with water (control) and the de-glycosylated sample treated with PNGase-F were analyzed for VNFTEIQ (VDFTEIQ) peptides are shown in Figs. 9a to 9d. In the pooled normal control samples, both N-form V**N**FTEIQ used for the glycosylated samples and D-form V**D**FTEIQ used for the de-glycosylated samples were not detected.

In contrast, with HCC samples, when N-form V**N**FTEIQ was used for the glycosylated samples and D-form V**D**FTEIQ was used for the de-glycosylated samples, the de-glycosylated samples analyzed for D-form were only detected.

This is due to the fact that the expression level of AFP is increased in HCC samples compared to the control and thus comes within the level to be detected by Mass spectrometry in contrast to the glycosylated samples in which case the mass of the peptide is changed due to the glycosylation and thus the peptide is not detected and only the de-glycosylated samples are detected.

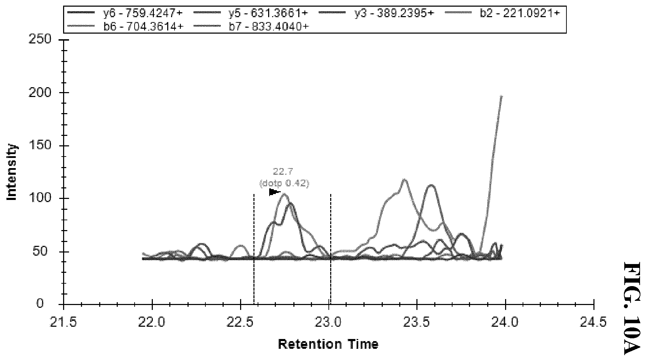

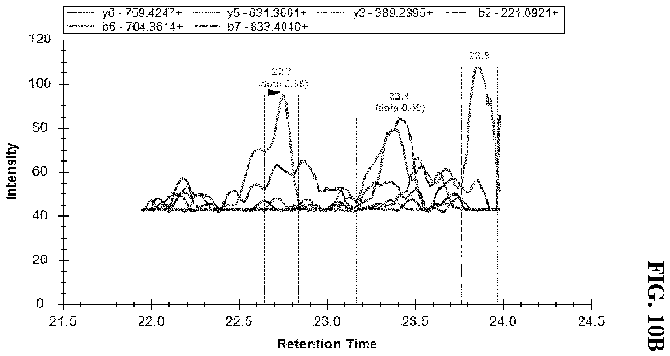

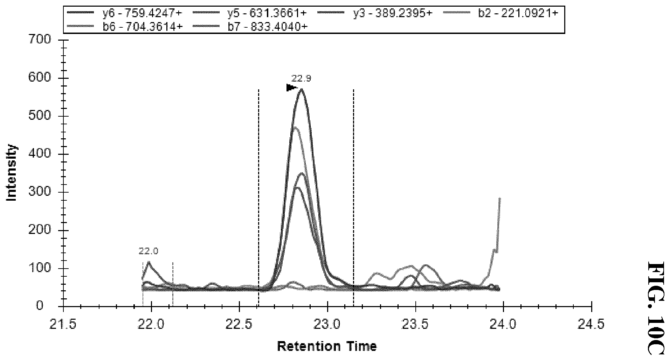

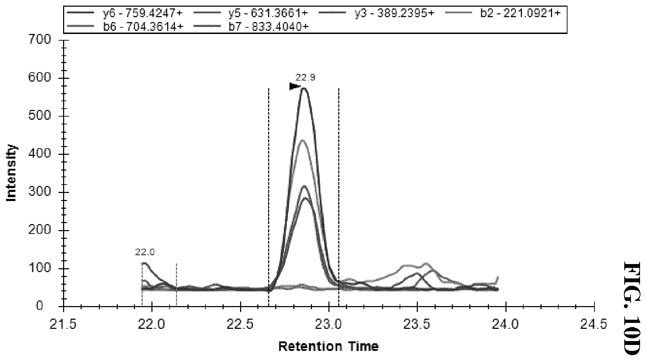

Results from the experiments in which the control glycosylated sample treated only with water (control) and the de-glycosylated sample treated with PNGase-F were analyzed for the non-glycosylated GYQELLEK peptide are shown in Figs. 10a to 10d. When the glycosylated and de-glycosylated samples were analyzed in the pooled normal control sample, none were detected. When the glycosylated and de-glycosylated samples were analyzed in the pooled HCC sample, both were detected. That is, the level of AFP protein was increased in HCC samples compared to the normal sample and thus comes within the level to be detected by Mass spectrometry.

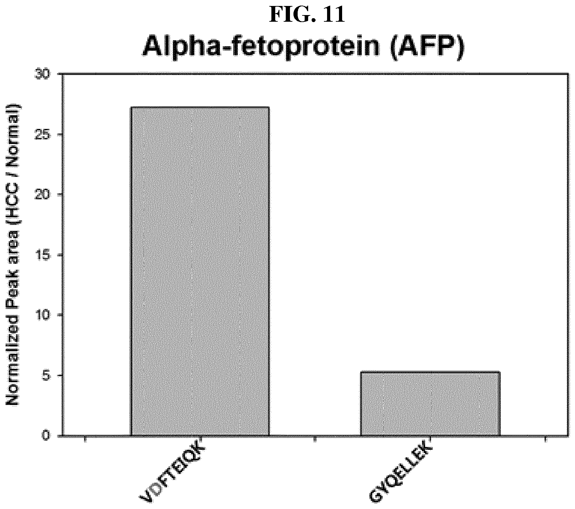

In summary, when the serum from the pooled normal control sample and HCC sample were treated with PNGase-F/trypsin and the de-glycosylated samples were analyzed for the de-glycosylated peptide V**D**FTEIQK and the non-glycosylated peptide GYQELLEK, a 27.3 fold difference was found between HCC group and the normal group when analyzed for V**D**FTEIQK in comparison to a 5.3 fold difference when analyzed for GYQELLEK as shown in Tables 9 and 10, and FIG. 11. This indicates that the glycosylation analysis is far superior in detecting the difference to the protein expression analysis.

**[Table 9]**

| | **Normal group** | | | **Cancer group** | | |
|---|---|---|---|---|---|---|
| **Set** | **Average** | **STDEV** | **CV (%)** | **Average** | **STDEV** | **CV (%)** |
| **1** | 0.0011 | 0.0004 | 38.5054 | 0.0731 | 0.0054 | 7.3393 |
| **2** | 0.0030 | 0.0012 | 40.6544 | 0.0124 | 0.0011 | 8.9907 |
| **3** | 0.0015 | 0.0015 | 104.7847 | 0.0652 | 0.0076 | 11.7206 |

**[Table 10]**

| | **Normal group** | | | **Cancer group** | | |
|---|---|---|---|---|---|---|
| **Set** | **Average** | **STDEV** | **CV (%)** | **Average** | **STDEV** | **CV (%)** |
| **1** | 0.0158 | 0.0035 | 22.3136 | 0.1020 | 0.0051 | 5.0402 |
| **2** | 0.0129 | 0.0065 | 50.2251 | 0.0077 | 0.0034 | 44.6222 |
| **3** | 0.0093 | 0.0031 | 32.9208 | 0.0907 | 0.0037 | 4.0869 |

In addition to AFP, further analysis have been done to further discover the candidates of glycosylated protein markers, as a result, a total of 354 proteins and 1000 peptides therefrom were detected in the liver cancer in comparison to the normal sample. From them, 145 proteins as glycoproteins with NxS/T motif, and 182 peptides therefrom as the de-glycosylated peptide after being treated with PNGase-F were determined. The de-glycosylated peptides and glycosylated peptides used for the detection are listed in Table 16.

### EXAMPLE 3. MRM analysis of an individual sample

### EXAMPLE 3-1 Preparation of individual clinical sample and MRM analysis

The preparation and MRM analysis of the individual samples from 60 normal samples and 60 HCC samples were prepared as described in Example 2.

For normalization, the synthetic heavy labelled peptide for the de-glycosylated peptide and the non-glycosylated peptide were used at the concentration of 7.3 fmol and 10.3 fmol, respectively.

All the individual samples were analyzed once and the data were imported into Skyline and converted into the area of the peptide transition. The peak area to AFP target peptide was normalized to the peak area value of the corresponding heavy labelled synthetic peptide.

In addition to AFP, the peptides or proteins which have shown at least 3 times signal to noise (S/N) ratio in the pooled clinical sample and which have been confirmed to flow in at least 3 product ions at the same retention time.

### EXAMPLE 3-2 AFP target peptide and optimization of collision energy

The present Example was performed to optimize the collision energy to improve the extent of the detection.

As a result of selecting the transition (Q1/Q3) of the target peptide, the difference between the endogenous peptide and the heavy labelled peptide difference was found to be 4.00 Da (5.00 Da) for Q1 and 8.01 Da (10.01 Da) for Q3.

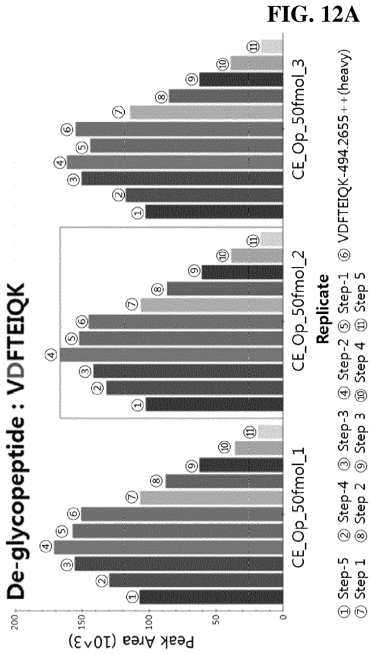

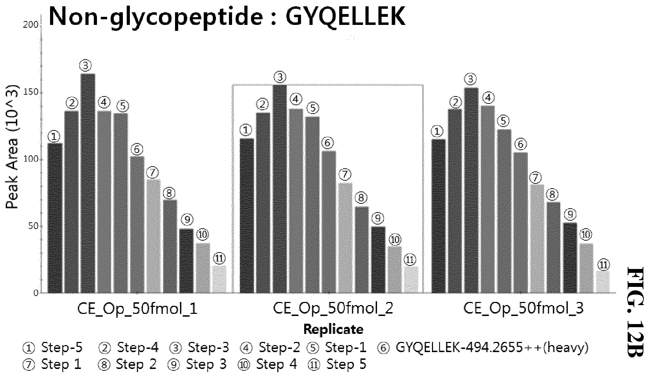

To determine the optimized collision energy (CE) for the heavy labelled synthetic peptide of AFP, a total of 11 points of CE including 2 units before and after the default CE value were analyzed for 3 times and the CE with the highest peak area was confirmed. The results are shown in Fig. 12 and Table 11. Table 11 shows the mass value (m/z) and the optimized CE value for AFP target peptide (endo/heavy).

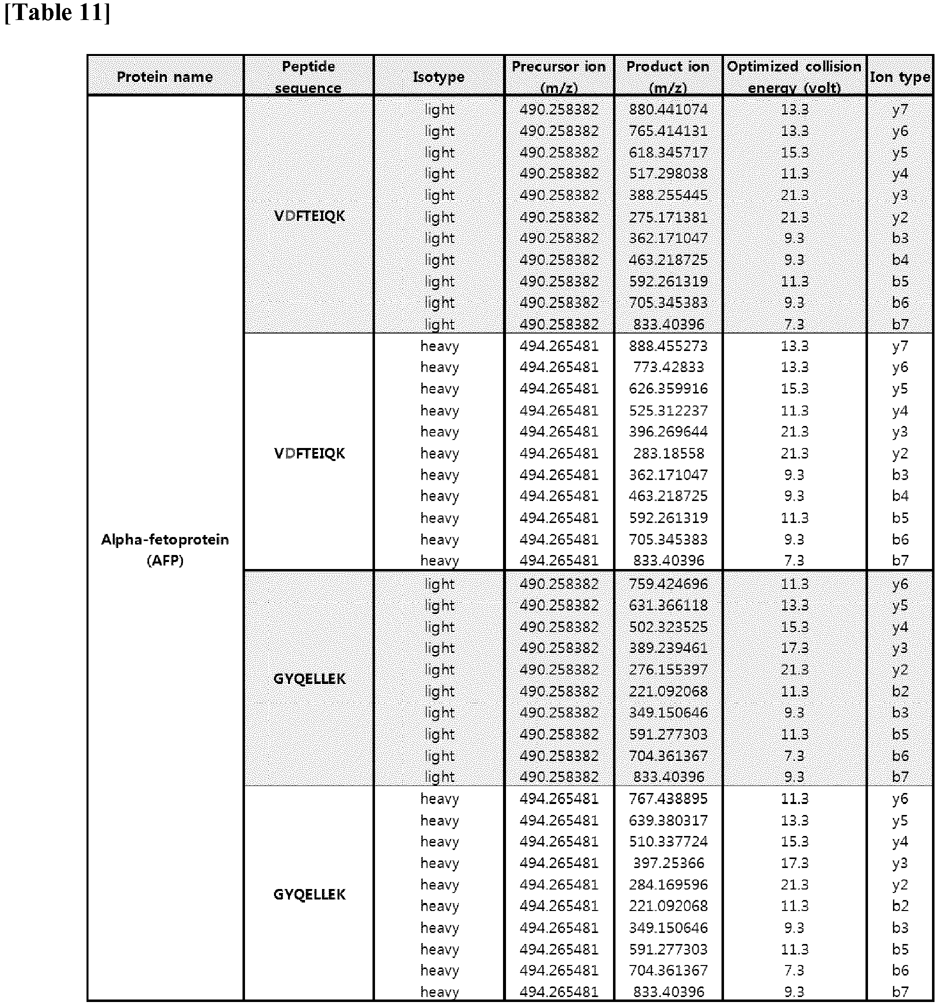

### EXAMPLE 3-3 Determination of endogenous AFP target peptide

Using a heavy labelled synthetic peptide with the same sequence as AFP target peptide (De-glycopeptide, Non-glycopeptide) except that ¹²C and ¹⁴N atoms in Arg (R) and Lys (K) residues at the C-terminal region were heavy labelled with ¹³C and ¹⁵N were used to confirm that the peptides detected are actually the endogenous peptide present in serum.

That is, the heavy labelled peptide and the endogenous peptide share the same sequence and thus have the identical hydrophobicity. Thus they can be detected on LC-column (C18) since they are eluted at the same retention time.

The de-glycosylated peptide V**D**FTEIQK and the non-glycosylated peptide GYQELLEK were analyzed on the de-glycosylated peptide obtained by treatment with

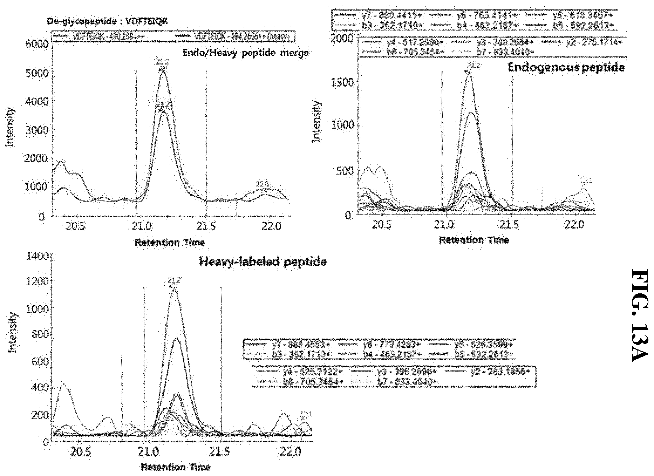

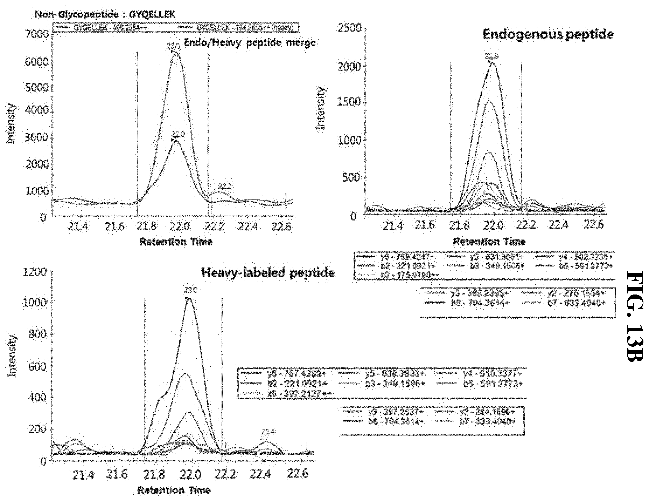

PNGase-F/trypsin together with the heavy labelled synthetic peptide. As a result, the endogenous peptides from serum and the heavy labelled synthetic peptide were eluted at the same retention time. Also the strength of the product ion type was determined to be identical. Results are shown in FIGs. 13a and 13b.

### EXAMPLE 3-4 Reaction curve of the heavy labelled synthetic peptide

To confirm the quantifiable property of the heavy labelled synthetic peptides to AFP target peptide, experiments to confirm the linearity of the reaction curve was performed as follows. The heavy labelled synthetic peptide for the de-glycosylated peptide VDFTEIQK was serially diluted to 0, 0.8, 1.6, 3.1, 6.3, 12.5, 25, 50, 100 fmol. The heavy labelled synthetic peptide for the non-glycosylated peptide GYQELLEK was serially diluted to 0, 1.6, 3.1, 6.3, 12.5, 25, 50, 100, 200 fmol. Then 5µg of serum from the pooled sample was added to each of the diluted peptides.

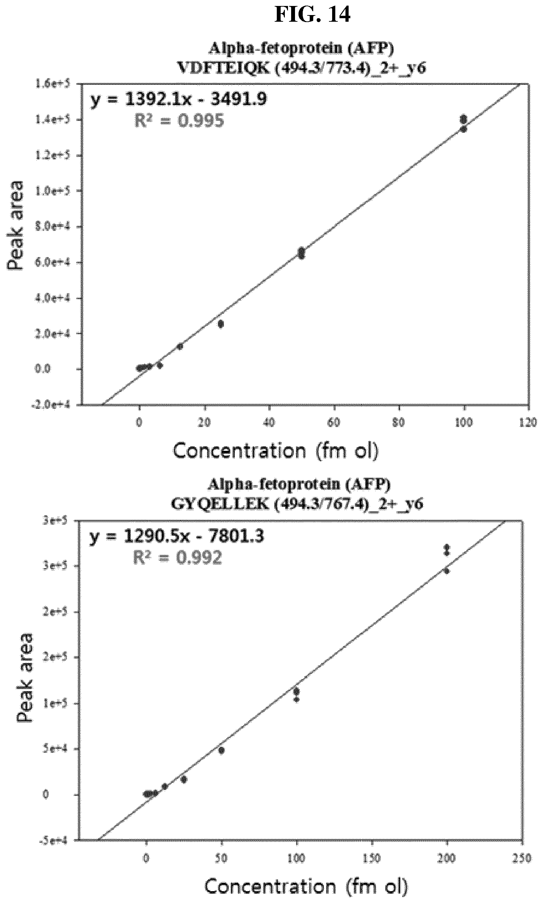

Experiments were repeated 3 times for each concentration. As a result, both the synthetic peptides were found to have a linearity (R^2=0.995, 0.992). Results are shown in FIG. 14 and Table 12.

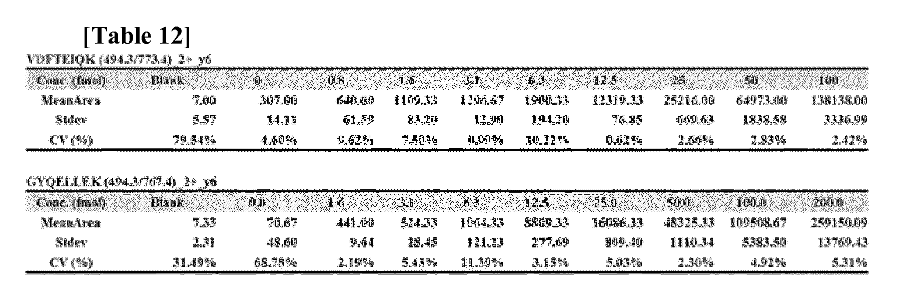

### EXAMPLE 4. Determination of correlation between clinical results and MRM data

### EXAMPLE 4-1 Classification 1 according to the detection (normal control)

MRM analysis were performed on 60 normal samples as described in Example. As shown in Table 13-1 to 13-3, the de-glycosylated peptide V**D**FTEIQK was detected in two samples (3.3%) out of sixty. The non-glycosylated peptide GYQELLEK was detected in seven samples out of sixty samples (11.7%). Based on this, the specificity with which the liver cancer can be differentiated from the normal person was found to be 96.7% for the de-glycosylated peptide and 88.3% for the non-glycosylated peptide.

**[Table 13-1]**

| | | **Set 1** | | | **Detection** | **(Normal group)** |
|---|---|---|---|---|---|---|
| **N.** | **T. #** | **Test Date** | **Sex** | **Age** | **Deglycopeptide** | **Non-glycopeptide** |
| **1** | N12-051 | 2012-03-23 | M | 53 | **Not detected** | **Not detected** |
| **2** | N12-052 | 2012-03-23 | M | 43 | **Not detected** | **Not detected** |
| **3** | N12-055 | 2012-03-23 | M | 59 | **Not detected** | **Not detected** |
| **4** | N12-057 | 2012-03-23 | M | 59 | **Not detected** | **Not detected** |
| **5** | N12-059 | 2012-03-23 | M | 42 | **Not detected** | **Not detected** |
| **6** | N12-061 | 2012-03-23 | M | 61 | **Not detected** | Detected |
| **7** | N12-062 | 2012-03-23 | M | 60 | **Not detected** | Detected |
| **8** | N12-069 | 2012-03-23 | M | 47 | **Not detected** | **Not detected** |
| **9** | N12-081 | 2012-03-23 | M | 51 | **Not detected** | Detected |
| **10** | N12-082 | 2012-03-23 | M | 44 | **Not detected** | **Not detected** |
| **11** | N12-085 | 2012-03-26 | M | 42 | **Not detected** | **Not detected** |
| **12** | N12-086 | 2012-03-26 | M | 51 | **Not detected** | **Not detected** |
| **13** | N12-088 | 2012-03-26 | M | 54 | **Not detected** | **Not detected** |
| **14** | N12-095 | 2012-03-26 | M | 69 | **Not detected** | **Not detected** |
| **15** | N12-054 | 2012-03-23 | F | 66 | **Not detected** | **Not detected** |
| **16** | N12-060 | 2012-03-23 | F | 64 | **Not detected** | **Not detected** |
| **17** | N12-075 | 2012-03-23 | F | 55 | Detected | **Not detected** |
| **18** | N12-084 | 2012-03-26 | F | 53 | **Not detected** | **Not detected** |
| **19** | N12-087 | 2012-03-26 | F | 39 | **Not detected** | **Not detected** |
| **20** | N12-108 | 2012-03-26 | F | 51 | **Not detected** | **Not detected** |

**[Table 13-2]**

| | | **Set 2** | | | **Detection** | **(Normal group)** |
|---|---|---|---|---|---|---|
| **N.** | **T. #** | **Test Date** | **Sex** | **Age** | **Deglycopeptide** | **Non-glycopeptide** |
| **21** | N12-096 | 2012-03-26 | M | 48 | **Not detected** | **Not detected** |
| **22** | N12-097 | 2012-03-26 | M | 55 | **Not detected** | **Not detected** |
| **23** | N12-101 | 2012-03-26 | M | 48 | **Not detected** | **Not detected** |
| **24** | N12-109 | 2012-03-26 | M | 69 | **Not detected** | **Not detected** |
| **25** | N12-112 | 2012-03-26 | M | 70 | **Not detected** | **Not detected** |
| **26** | N12-120 | 2012-03-26 | M | 45 | **Not detected** | **Not detected** |
| **27** | N12-122 | 2012-03-26 | M | 52 | **Not detected** | **Not detected** |
| **28** | N12-125 | 2012-03-26 | M | 59 | **Not detected** | **Not detected** |
| **29** | N12-126 | 2012-03-26 | M | 47 | **Not detected** | **Not detected** |
| **30** | N12-127 | 2012-03-26 | M | 66 | **Not detected** | Detected |
| **31** | N12-130 | 2012-03-26 | M | 53 | **Not detected** | **Not detected** |
| **32** | N12-131 | 2012-03-26 | M | 43 | **Not detected** | **Not detected** |
| **33** | N12-1S.8 | 2012-03-28 | M | 43 | **Not detected** | **Not detected** |
| **34** | N12-199 | 2012-03-28 | M | 56 | **Not detected** | **Not detected** |
| **35** | N12-110 | 2012-03-26 | F | 62 | **Not detected** | **Not detected** |
| **36** | N12-117 | 2012-03-26 | F | 49 | **Not detected** | **Not detected** |
| **37** | N12-119 | 2012-03-26 | F | 37 | **Not detected** | **Not detected** |
| **38** | N12-128 | 2012-03-26 | F | 42 | **Not detected** | **Not detected** |
| **39** | N12-189 | 2012-03-28 | F | 58 | **Not detected** | **Not detected** |
| **40** | N12-202 | 2012-03-28 | F | 65 | **Not detected** | **Not detected** |

**[Table 13-3]**

| | | **Set 3** | | | **Detection** | **(Normal group)** |
|---|---|---|---|---|---|---|
| **N.** | **T. #** | **Test Date** | **Sex** | **Age** | **Deglycopeptide** | **Non-glycopeptide** |
| **41** | N12-213 | 2012-03-29 | M | 59 | **Not detected** | **Not detected** |
| **42** | N12-216 | 2012-03-29 | M | 46 | **Not detected** | **Not detected** |
| **43** | N12-217 | 2012-03-29 | M | 61 | **Not detected** | **Not detected** |
| **44** | N12-219 | 2012-03-29 | M | 43 | **Not detected** | Detected |
| **45** | N12-220 | 2012-03-29 | M | 58 | **Not detected** | **Not detected** |
| **46** | N12-225 | 2012-03-29 | M | 58 | **Not detected** | **Not detected** |
| **47** | N12-228 | 2012-03-29 | M | 53 | **Not detected** | **Not detected** |
| **48** | N12-229 | 2012-03-29 | M | 52 | **Not detected** | **Not detected** |
| **49** | N12-233 | 2012-03-29 | M | 53 | **Not detected** | **Not detected** |
| **50** | N12-239 | 2012-03-29 | M | 57 | **Not detected** | **Not detected** |
| **51** | N12-249 | 2012-03-29 | M | 57 | **Not detected** | **Not detected** |
| **52** | N12-254 | 2012-03-30 | M | 56 | **Not detected** | **Not detected** |
| **53** | N12-258 | 2012-03-30 | M | 51 | **Not detected** | **Not detected** |
| **54** | N12-261 | 2012-03-30 | M | 51 | **Not detected** | **Not detected** |
| **55** | N12-204 | 2012-03-28 | F | 44 | **Not detected** | Detected |
| **56** | N12-218 | 2012-03-29 | F | 59 | **Not detected** | **Not detected** |
| **57** | N12-221 | 2012-03-29 | F | 35 | **Not detected** | **Not detected** |
| **58** | N12-226 | 2012-03-29 | F | 54 | **Not detected** | **Not detected** |
| **59** | N12-235 | 2012-03-29 | F | 53 | Detected | Detected |
| **60** | N12-241 | 2012-03-29 | F | 50 | **Not detected** | **Not detected** |

### EXAMPLE 4-2 Classification 2 according to the detection (HCC group)

MRM analysis were performed on 60 HCC samples as described in Example 3. As shown in Tables 14-1 to 14-3, the de-glycosylated peptide (VDFTEIQK) was detected in 39 samples out of 60 (65.0%). The non-glycosylated peptide (GYQELLEK) was detected in 32 samples out of 60 samples (53.3%). Based on this, it was determined that the sensitivity to determine the cancer as cancer is 65.0% for the de-glycosylated peptide and 53.3% for the non-glycosylated peptide.

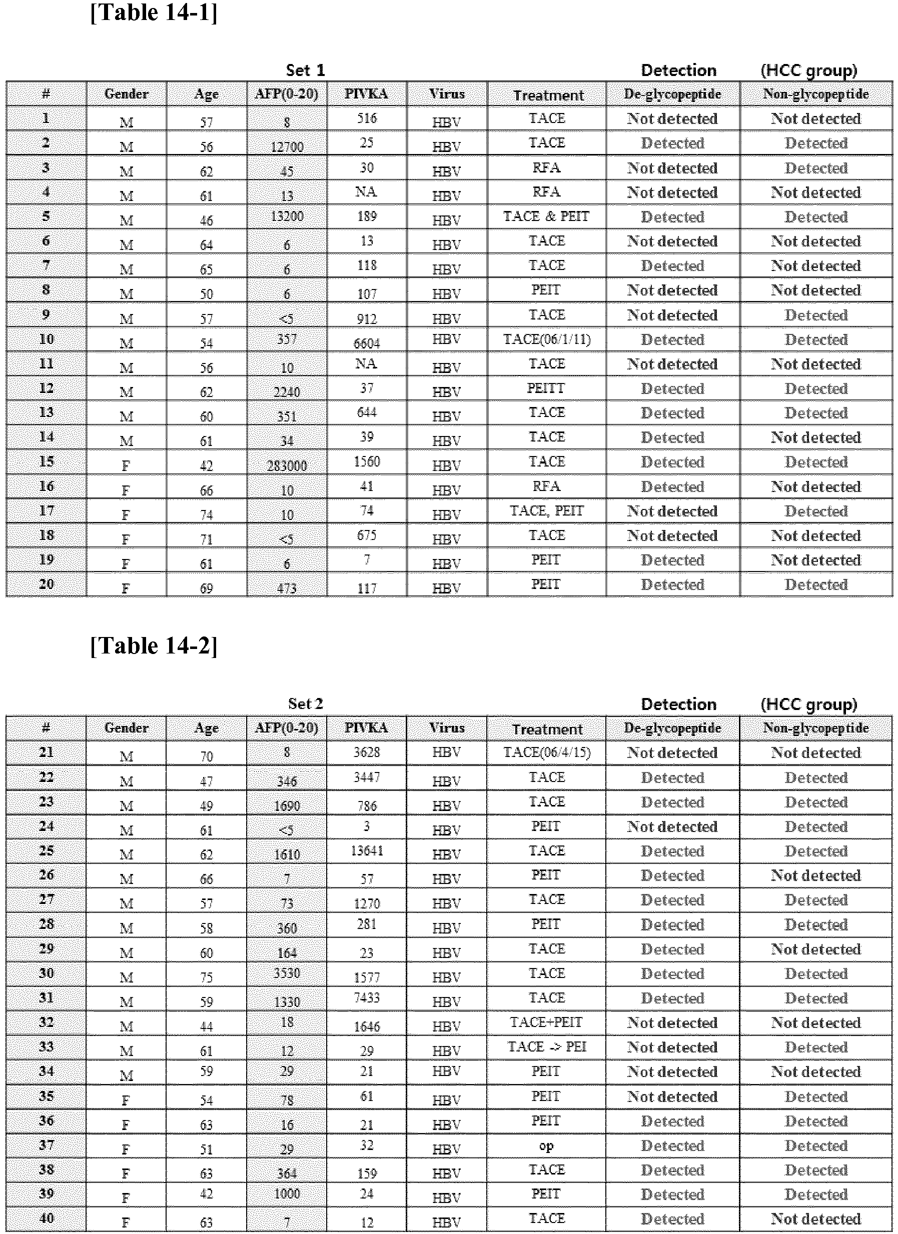

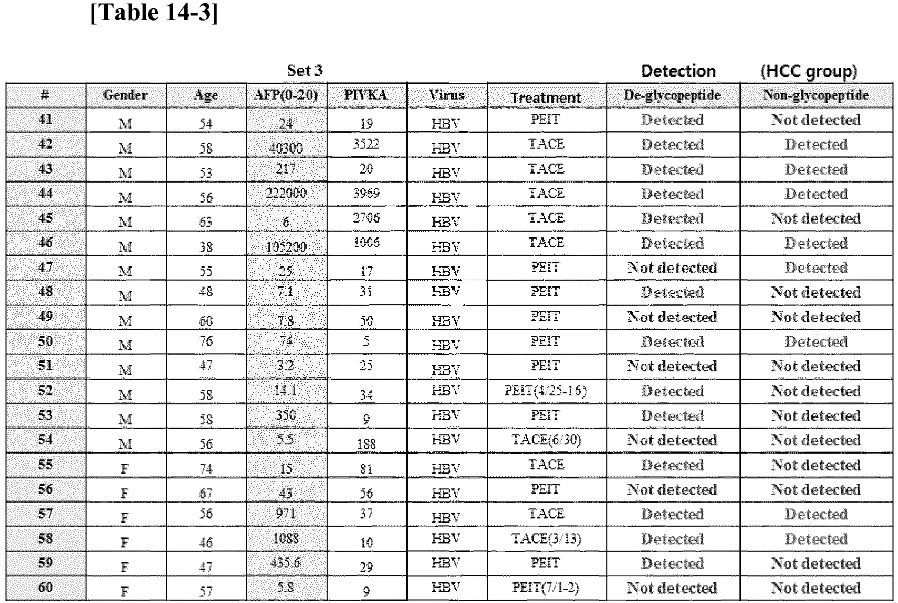

### EXAMPLE 4-3 Correlation between clinical results and MRM results

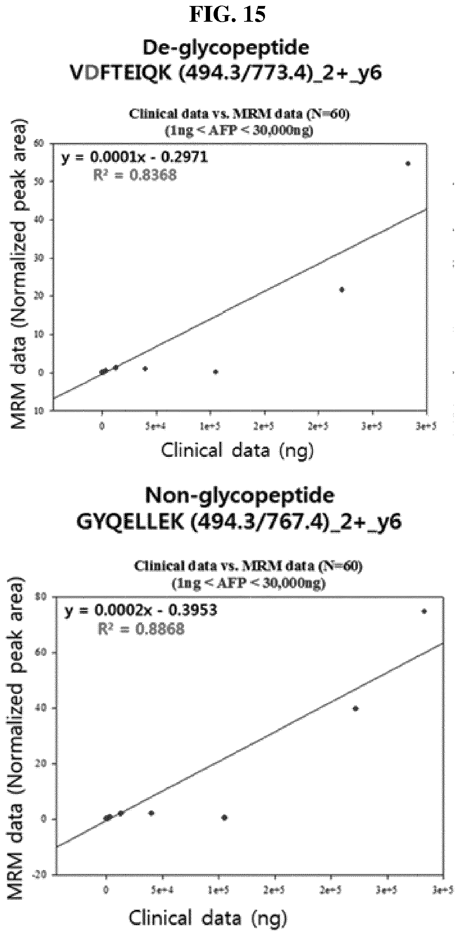

The level of AFP was measured in 60 HCC patients using commercially available AFP kit (Bioland; NanoSign AFP, Nanoentech; AFP quantification kit) commonly used in clinics according to the manufacturer's instruction (clinical results). Then the data obtained using AFP kit were compared to the MRM data as described in Example 3 to determine the correlation between the two data set. As shown in FIG. 15, de-glycosylated peptide of AFP protein (V**D**FTEIQK) was found to have a R^2 value of 0.8368, the non-glycosylated peptide (GYQELLEK) was found to have a R^2 value of 0.8868. This indicates that the two data set, i.e., AFP data and MRM data are correlated to each other in a quantitative manner.

Further to confirm the efficiency of the diagnosis using the present method, ROC (Receiver-Operating Characteristic) curve was determined on 60 normal controls and HCC patients to obtain AUC (Area Under Curve). As a result, it was found that the non-glycosylated peptide (GYQELLEK) was found to have an AUC value of 0.734, and the de-glycosylated peptide (V**D**FTEIQK) was found to have an AUC value of 0.811.

Then the non-glycosylated and de-glycosylated peptides data from AFP protein were combined into one panel using logistic regression model. As a result, it was found that 58 normal people out of 60 were determined as being normal and 2 were determined as having cancer; and 41 liver cancer patients out of 60 were determined as having cancer, and 19 patients were determined as being normal. Thus the accuracy was 82.5%. That is, as shown in Table 15, as a result of comparison of AUC value using the non-glycosylated peptide, de-glycosylated peptide and the combination thereof, the two-peptide panel was found to have a higher value (AUC=0.852) to differentiate liver cancer from normal patients compared to each of the peptide.

This indicates that by monitoring both the non-glycosylated peptide (GYQELLEK) and the de-glycosylated peptide (V**D**FTEIQK) of AFP which were obtained by treating the blood sample of patients with PNGase-F/trypsin, the liver cancer can be differentiated from normal sample with high specificity.

**[Table 15]**

| **Group** | **Predicted group** | | **Percent correct** |
|---|---|---|---|
| | **Normal group** | **HCC group** | |
| **Normal group** | 58 | 2 | 96.67% |
| HCC group | 19 | 41 | 68.33% |
| **Percent of cases correctly classified** | | | 82.50% |

### Example 5. MRM analysis to discover additional glycoprotein markers in addition to AFP

Three hundred fifty four proteins corresponding to the non-glycosylated peptides selected in Example 2-5, 145 proteins corresponding to 1000 peptides and de-glycosylated peptides, and 182 peptides were applied to individual samples. Then the proteins showing the difference between normal and patient groups were selected as final target protein marker.

In the analysis, normal and patient samples were analyzed alternatively, that is, normal sample No. 1, liver cancer sample No.1, normal sample No. 2 followed by liver cancer sample No. 2 and the like. The data obtained were fed into Skyline software and analyzed using MedCalc (version 12.2). As a result, 35 proteins showing the difference between the normal and liver cancer sample were selected as follows: Alpha-2-antiplasmin (SERPINF2), Alpha-2-macroglobulin (A2M), Apolipoprotein B-100 (APOB), Beta-galactosidase (GLB1), Bone morphogenetic protein 1 (BMP1), Corticosteroid-binding globulin (SERPINA6), Complement factor H (CFH), Cholinesterase (BCHE), Clusterin (CLU), Collagen alpha-1(XII) chain (COL12A1), Carboxypeptidase N subunit 2 (CPN2), Versican core protein (VCAN), Receptor tyrosine-protein kinase erbB-3 (ERBB3), Coagulation factor V (F5), Coagulation factor XI (F11), Follistatin-related protein 1 (FSTL1), N-acetylglucosamine-6-sulfatase (GNS), G-protein coupled receptor 126 (GPR126), Heparin cofactor 2 (SERPIND1), Hypoxia up-regulated protein 1 (HYOU1), Integrin alpha-2 (ITGA2), Integrin alpha-3 (ITGA3), Integrin alpha-6 (ITGA6), Integrin alpha-M (ITGAM), Integrin beta-2 (ITGB2), Plasma kallikrein (KLKB1), Kinectin (KTN1), Lysosome-associated membrane glycoprotein 2 (LAMP2), Galectin-3-binding protein (LGALS3BP), Plexin-Al (PLXNA1), Periostin (POSTN), Inactive tyrosine-protein kinase 7 (PTK7), Roundabout homolog 4 (ROBO4), Tenascin (TNC), and Vitronectin (VTN).

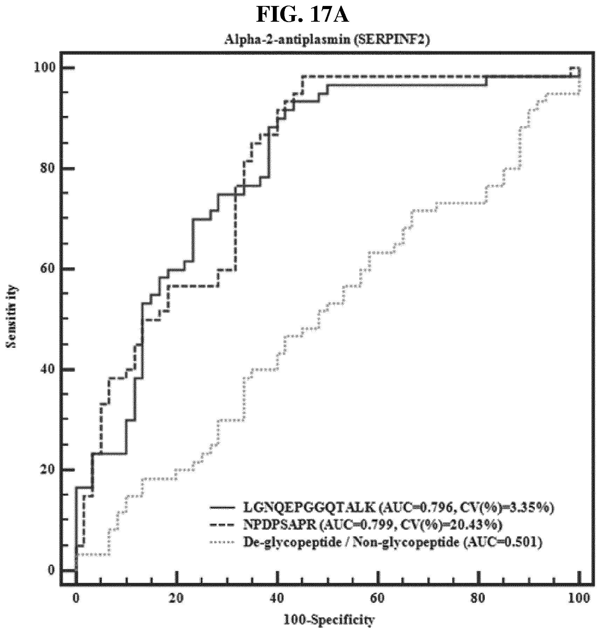

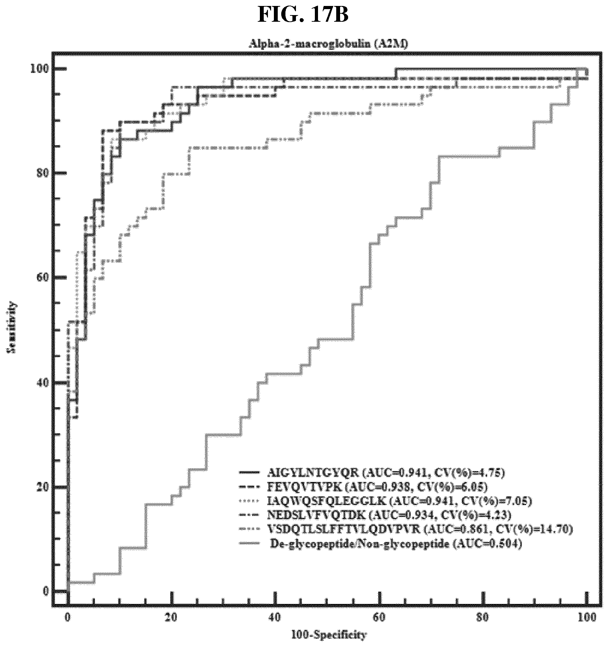

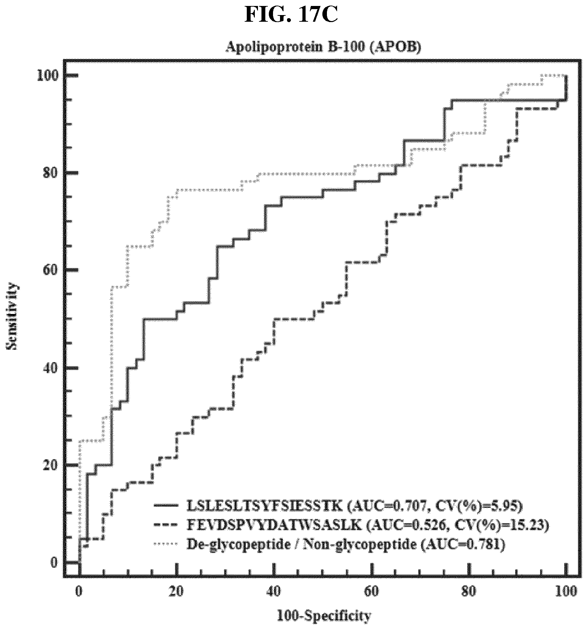

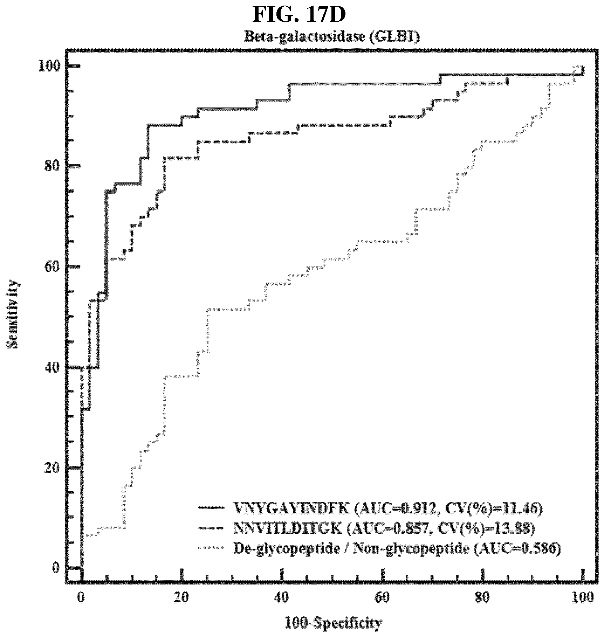

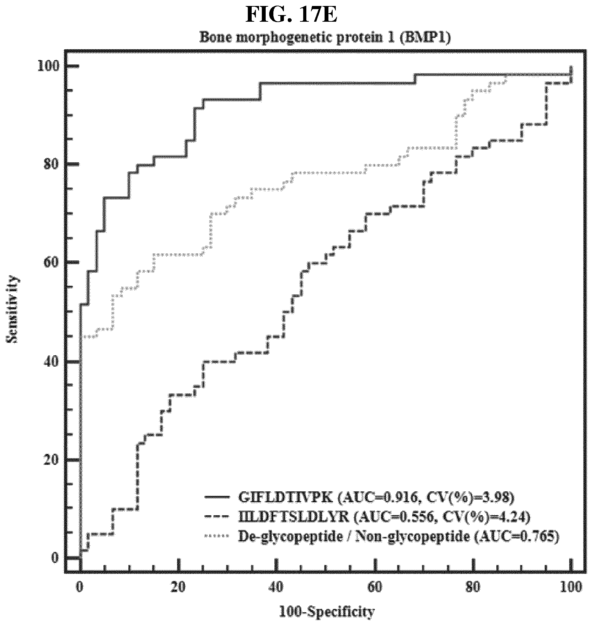

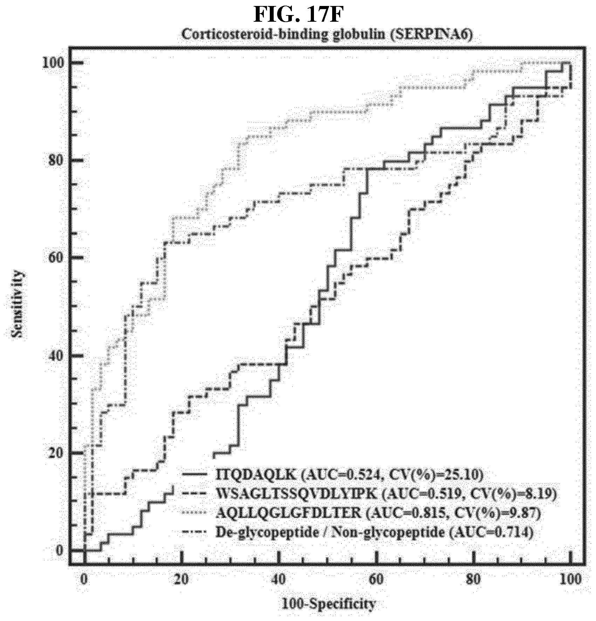

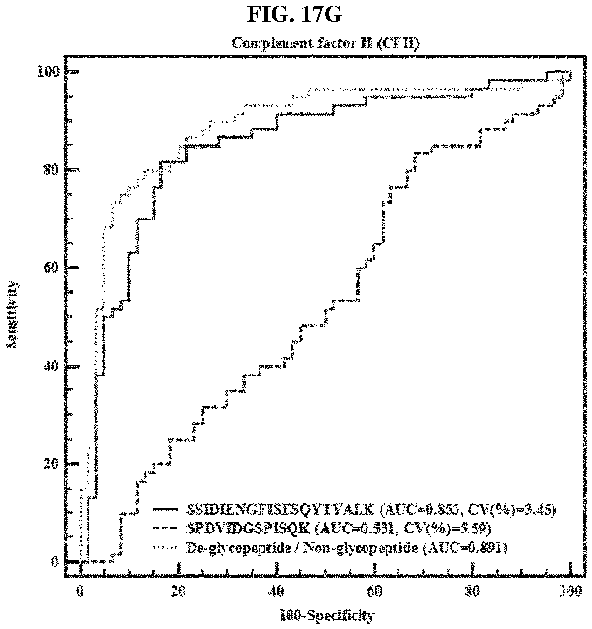

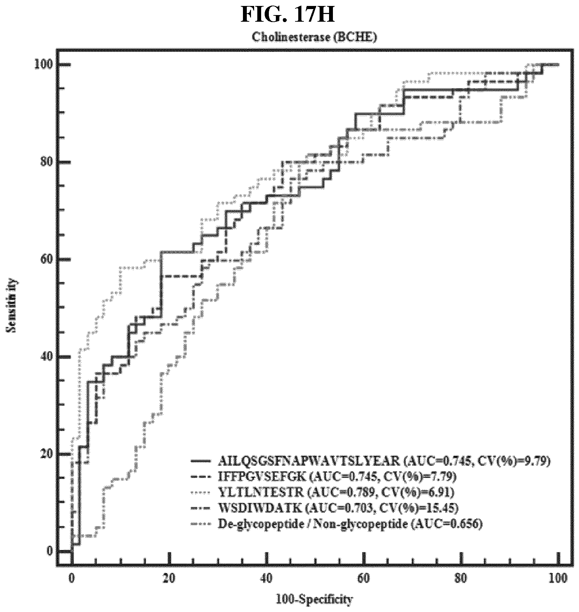

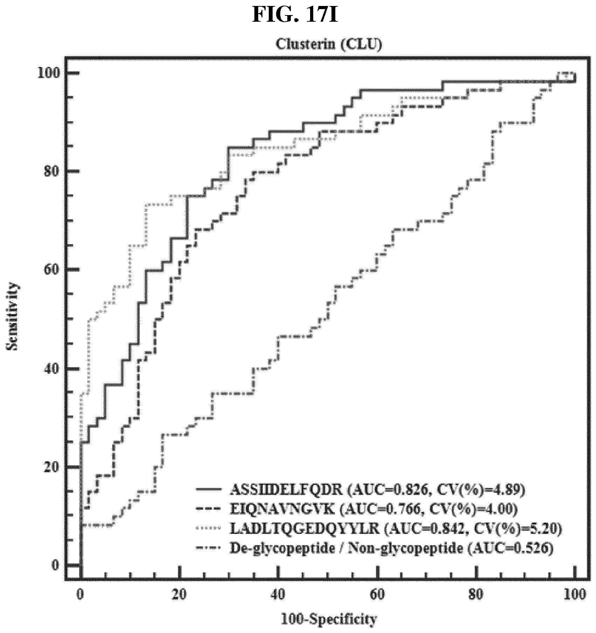

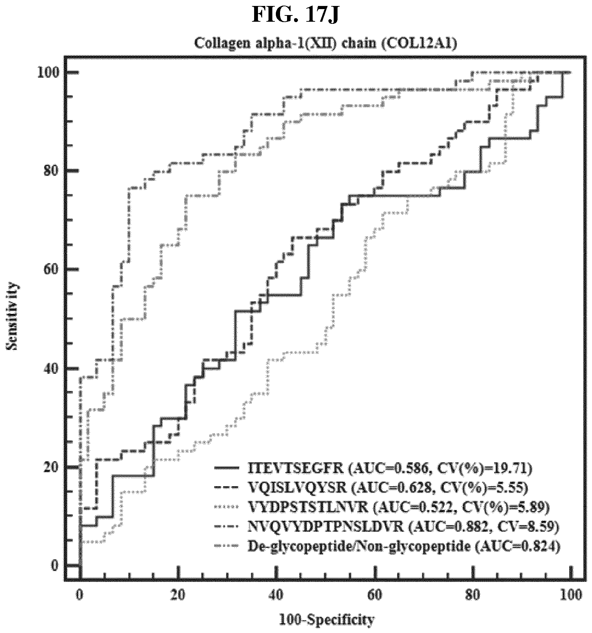

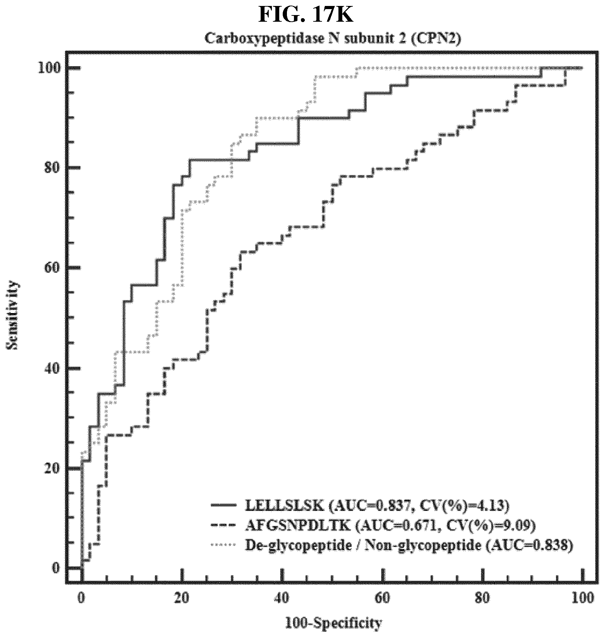

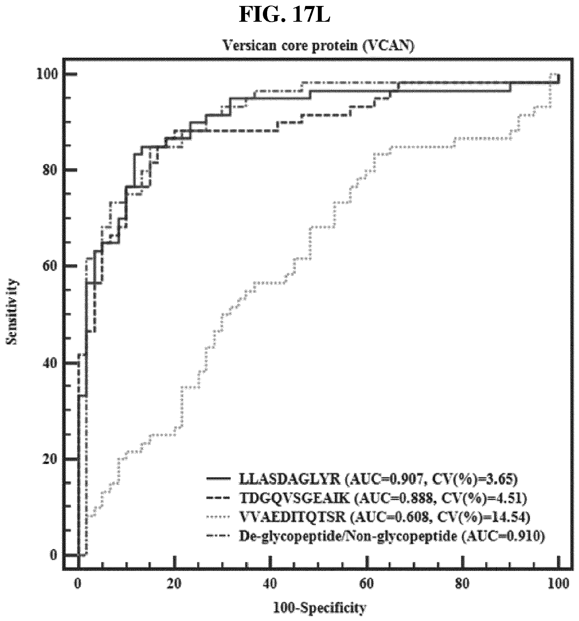

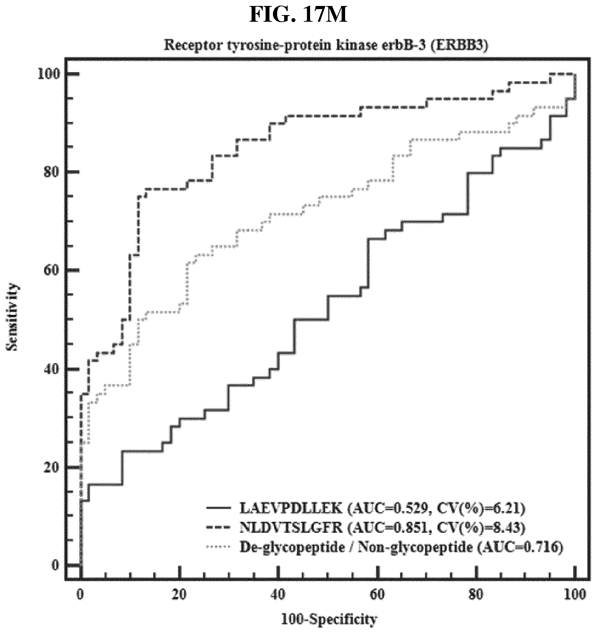

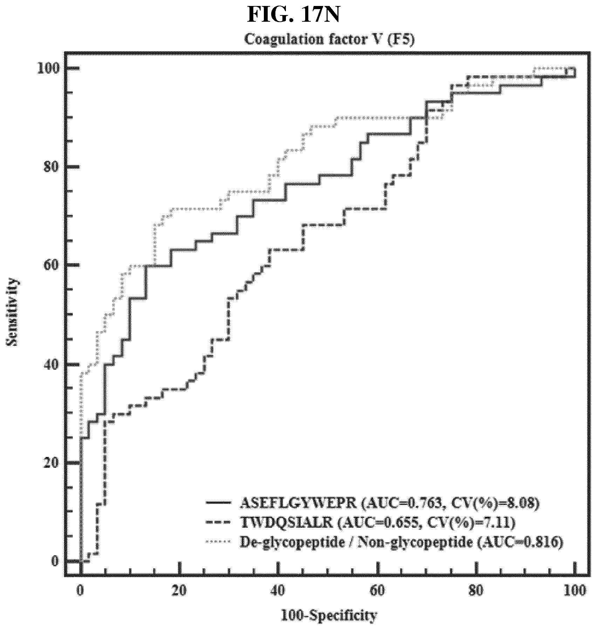

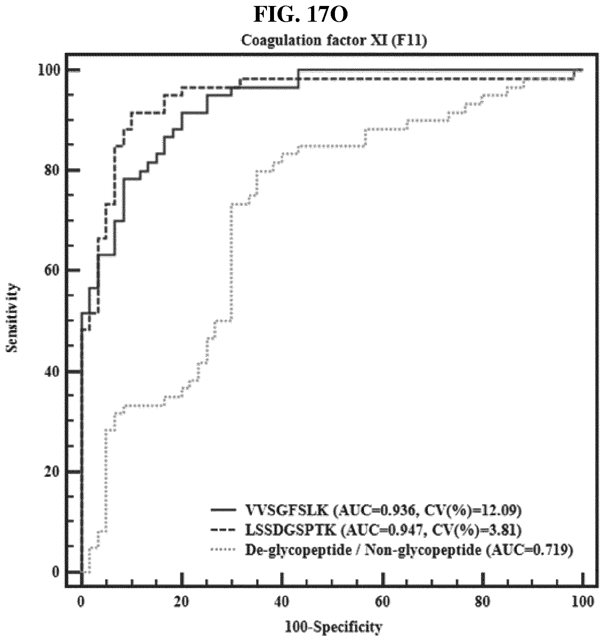

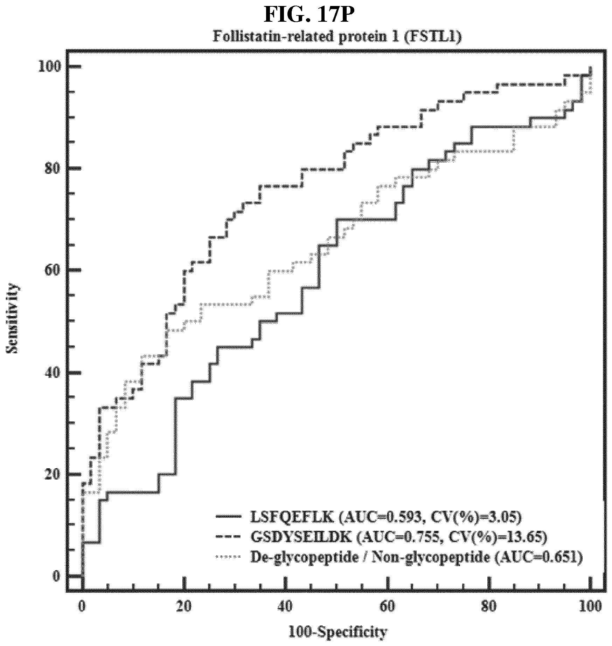

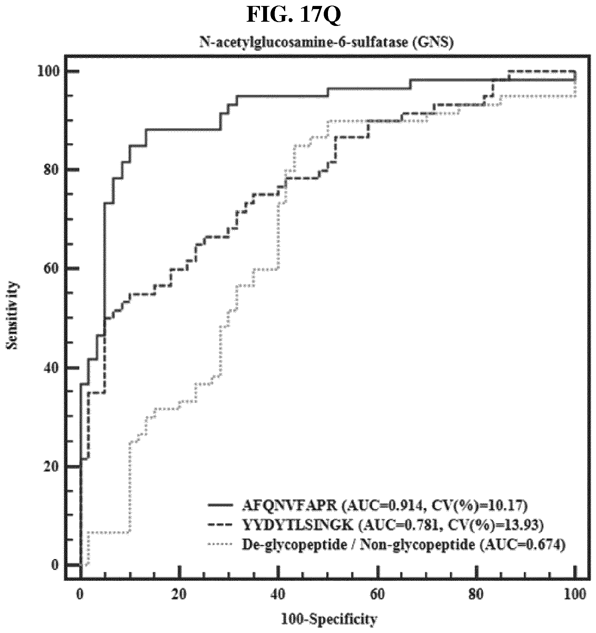

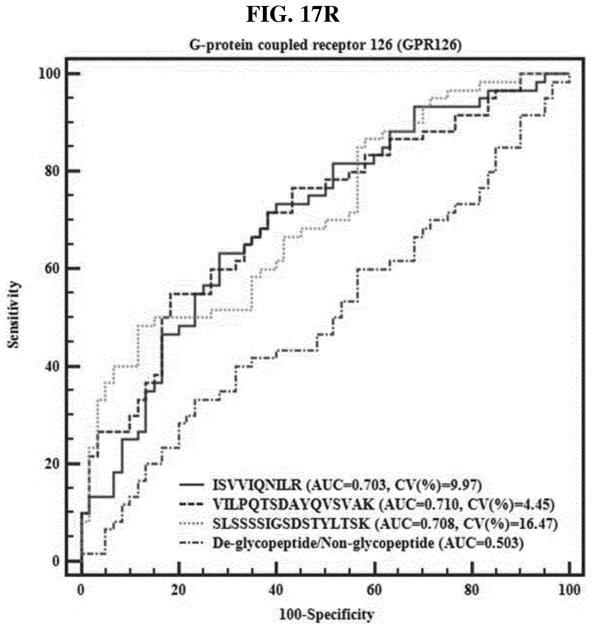

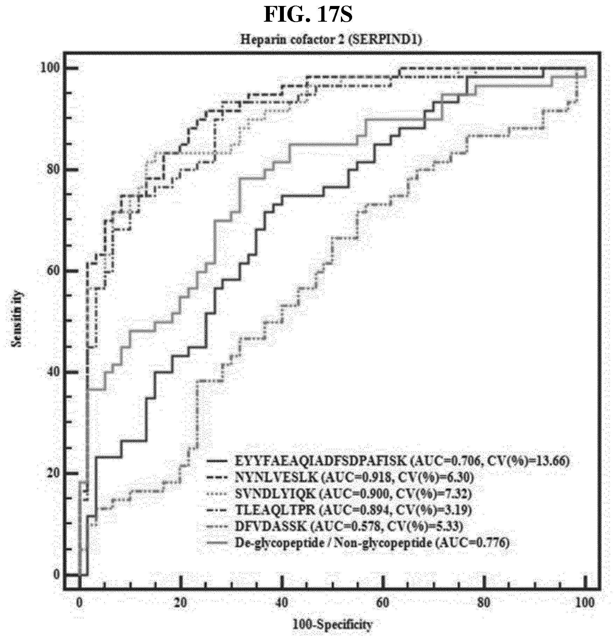

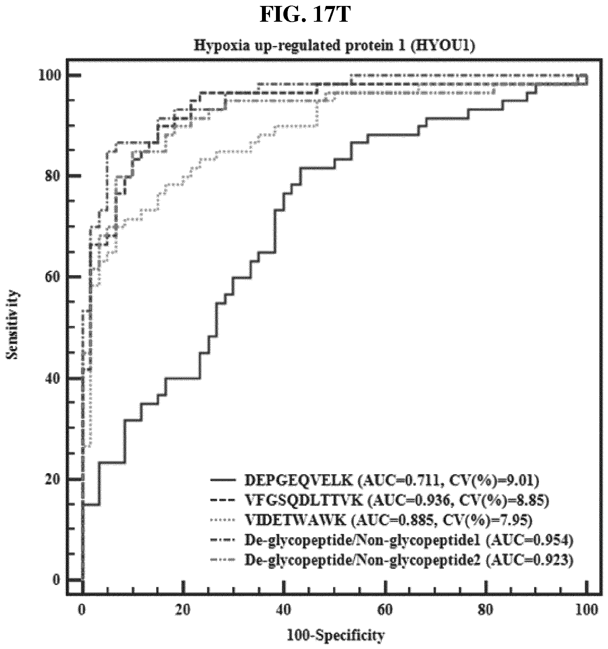

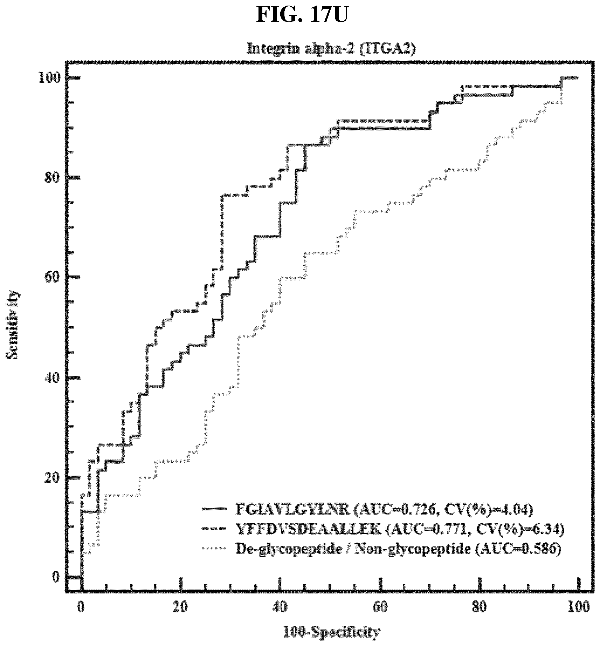

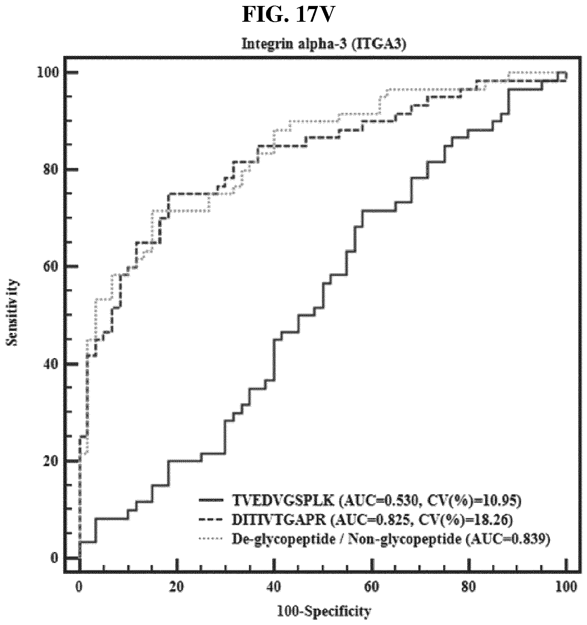

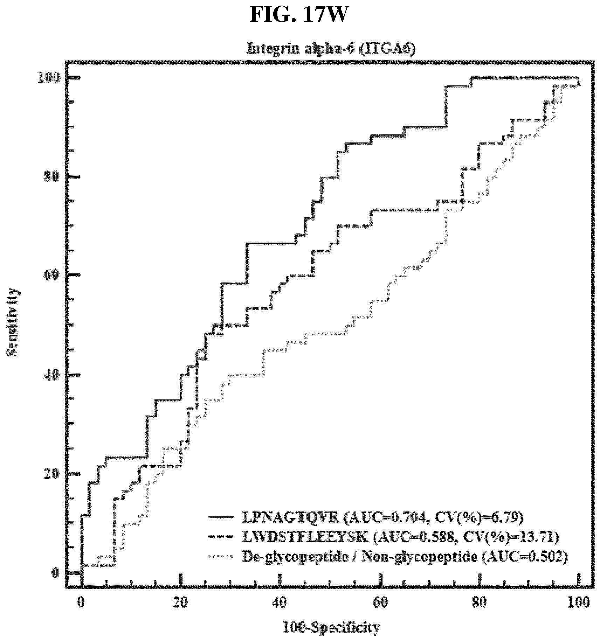

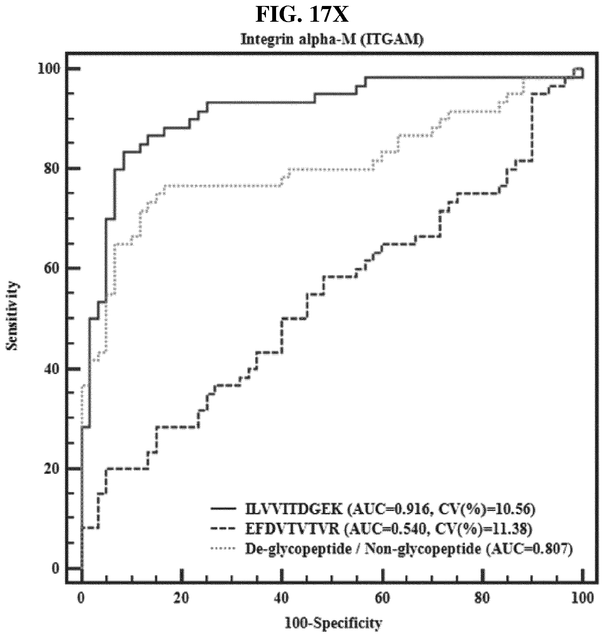

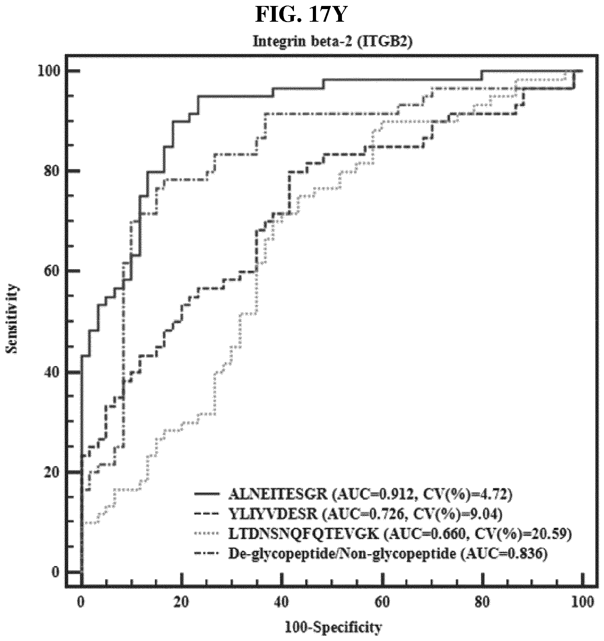

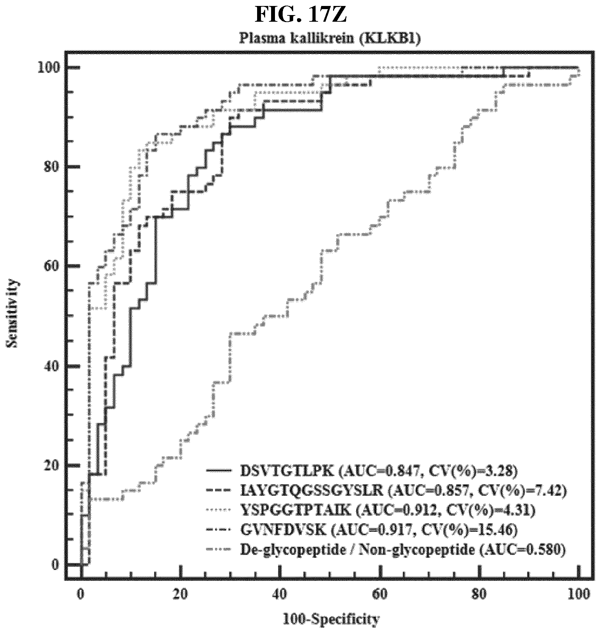

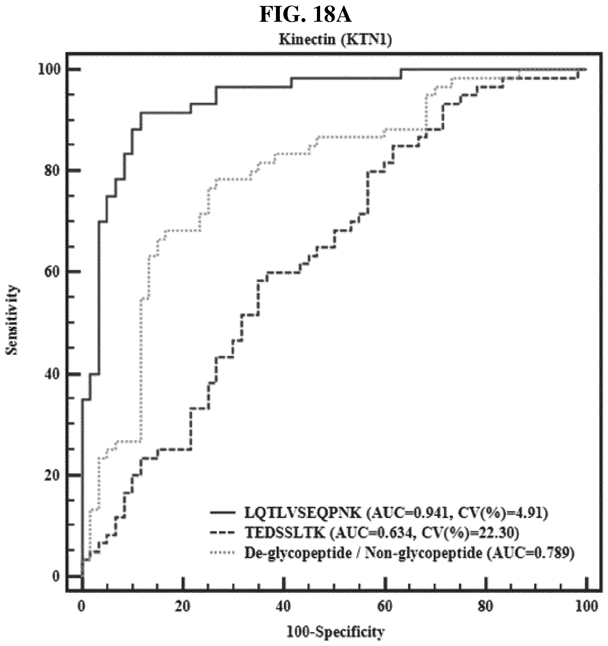

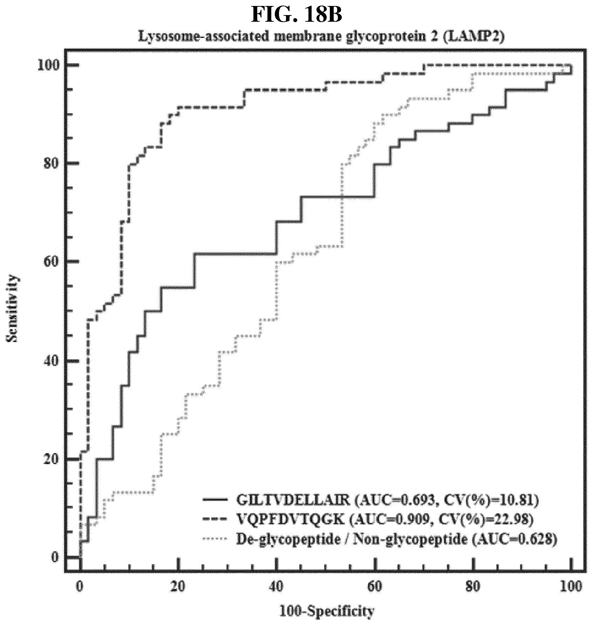

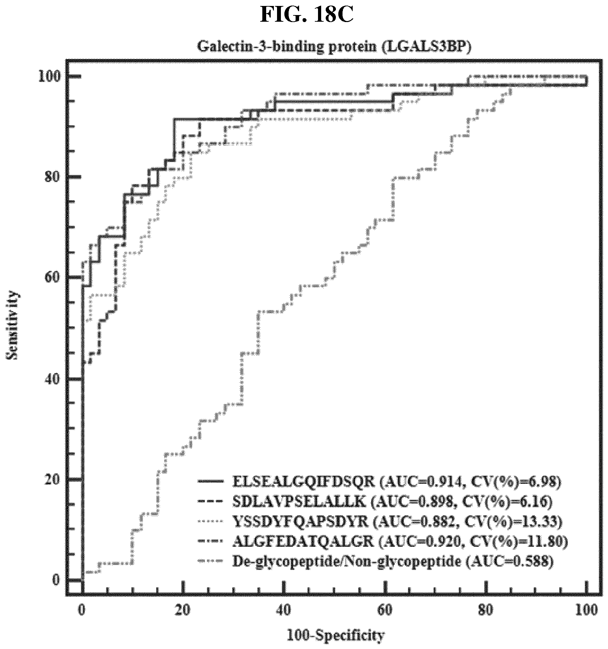

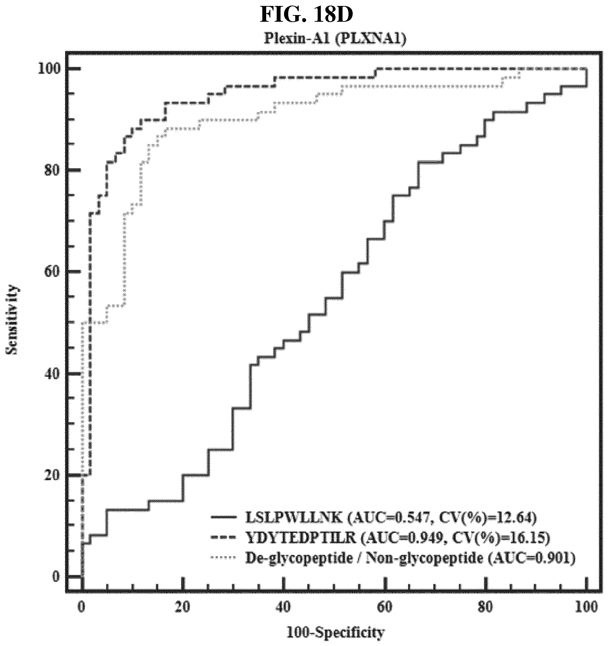

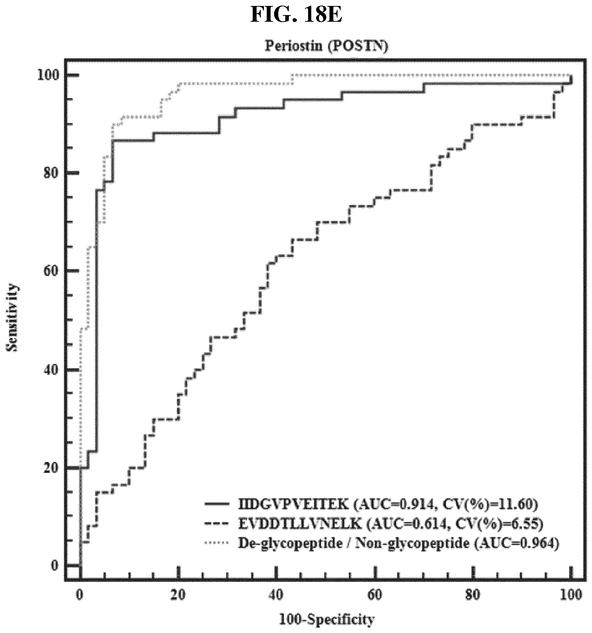

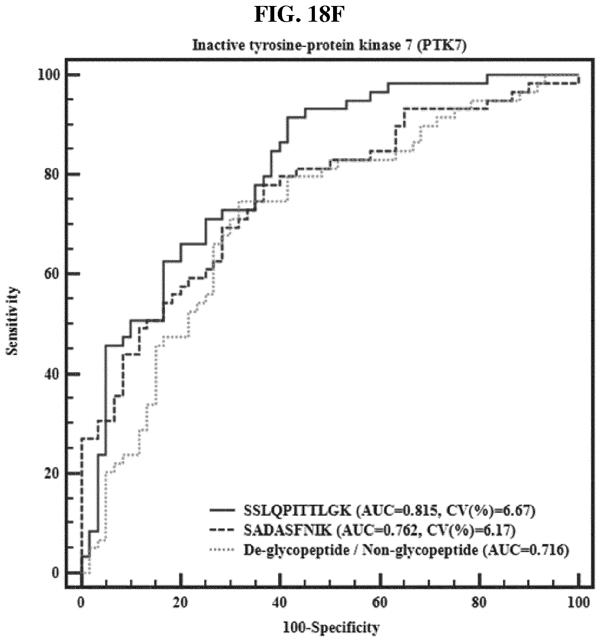

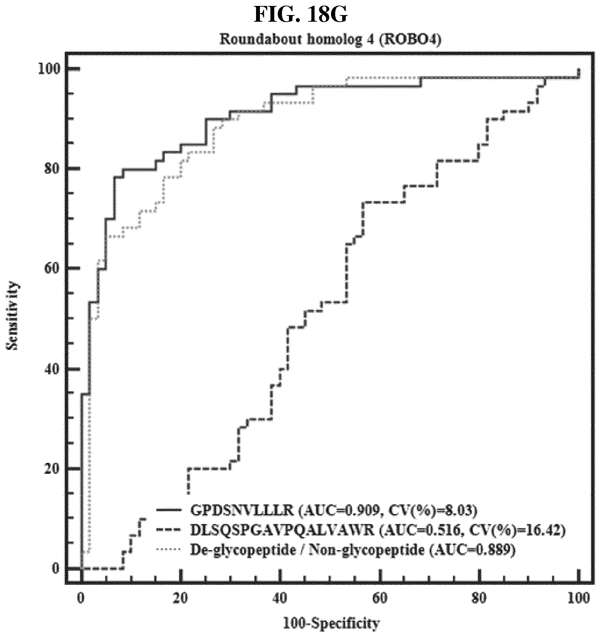

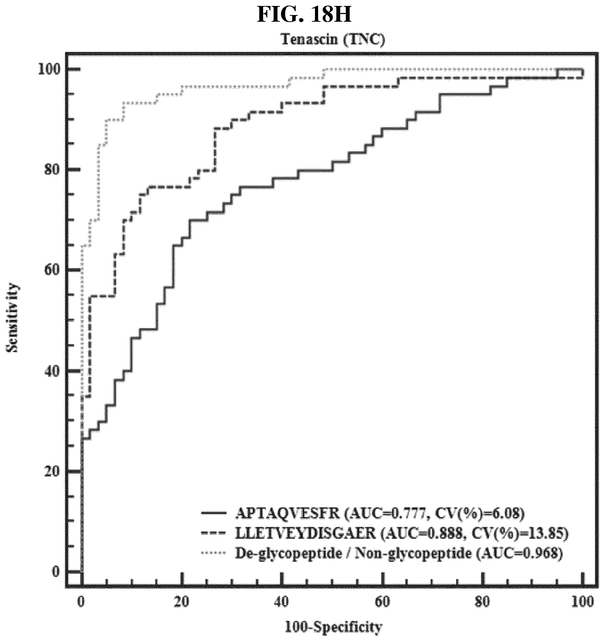

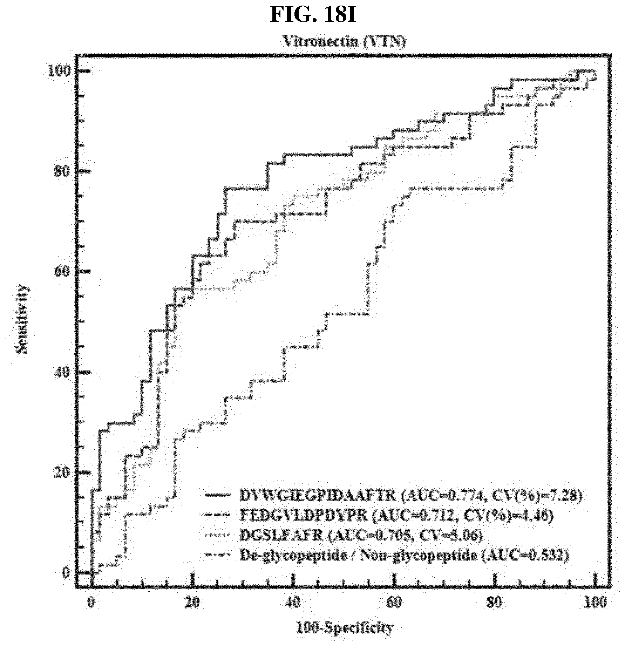

The ROC curves were determined for each of the 35 target proteins. A ROC curve is a graphical plot that illustrates the changing relationship between the specificity and sensitivity. In ROC curves, bigger AUC (area under curve) value indicates better diagnosis ability. The AUC values determined for 35 markers were listed in Tables 16-1 to 16-3. ROC curve and the correlation plot were prepared using MedCalc (version 12.2) statistical program which are shown in FIGs. 17a to 17z and FIGs. 18a to 18i.

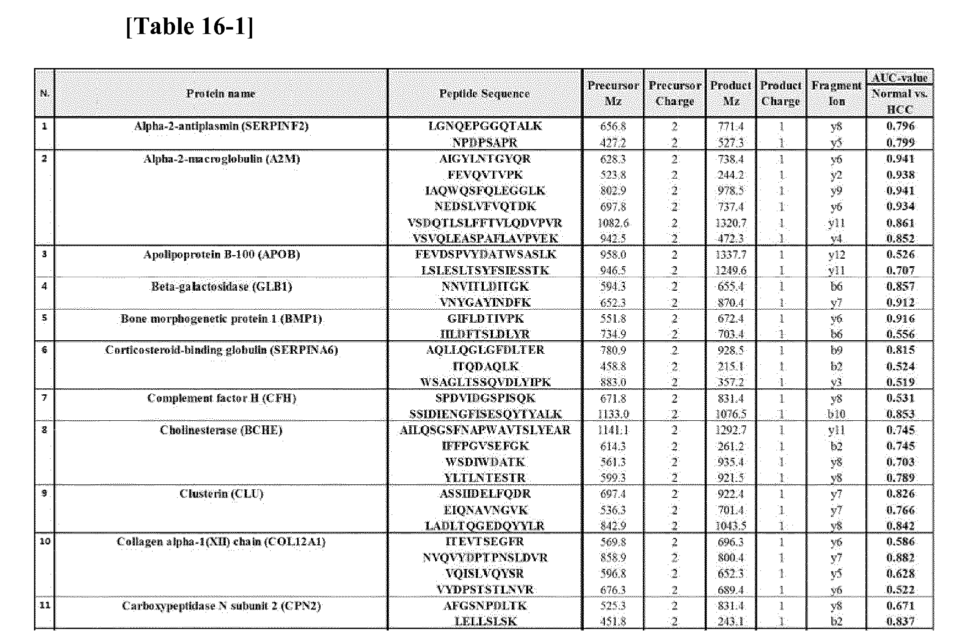

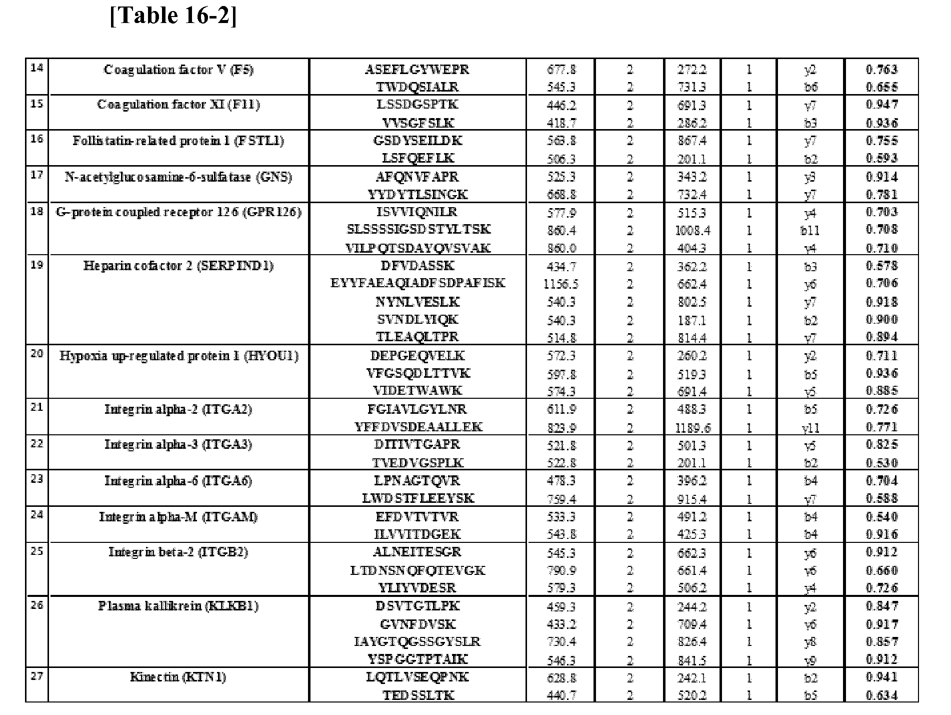

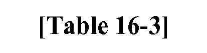

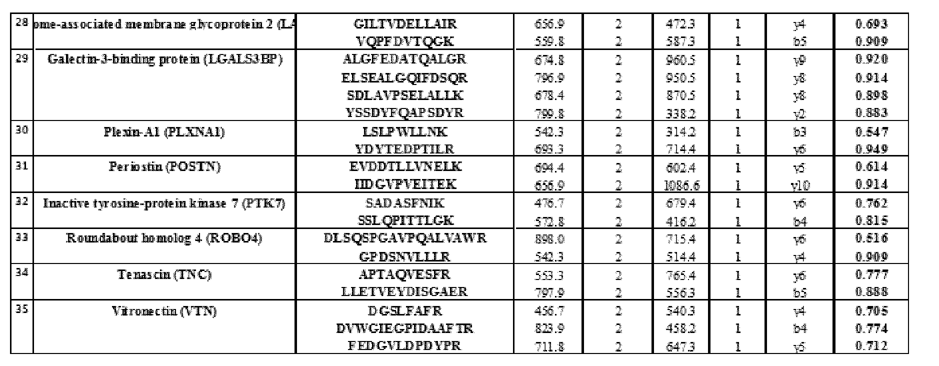

### Example 6. Determination of liver cancer biomarkers

To determine the efficiency of the liver diagnosis using the biomarkers of the present disclosure, the amount of de-glycosylated peptides and the amount of the non-glycosylated peptides of the biomarkers screened by the present method as in Example 4 were determined in the cancer and normal samples using an interactive plot.

The amount of de-glycosylated peptides and the amount of the non-glycosylated peptides of the biomarkers were determined by Liquid chromatography (1260 capillary LC system, Agilent, USA) on 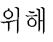 Capillary RR 0.3 x 150, 3.5um (Cat.N 5064-8261) column.

Five microliter of the peptide samples were directly injected into the column without passing through a trap column and eluted at a flow rate of 20L/min. Column was equilibrated with SolA (97% Distilled Water, 3 vol% acetonitrile, 0.1 vol% formic acid) for 10 min and eluted with SolB (3% Distilled Water, 97% acetonitrile, 0.1% formic acid) over 45 min on a linear gradient of 5% to 60% and over 5 min on a gradient of 85%.

Mass spectrometry 6490-Triple quadrupole (QQQ) from Agilent technology was used to monitor the transition of the selected protein under MRM mode. The settings were as follows: gas temperature of 200°C, gas flow of 14L/min, nebulizer at 20psi, sheath gas temperature of 250°C and sheath gas flow of 1 1L/min. The voltage applied for the capillary and nozzle was 3000V.

The resolution of 0.7Da was used for Quadruple 1(Q1) and Quadruple 3 (Q3). The dwell time was set to 2 sec for a total cycle in an unscheduled MRM mode to complete analysis of all the target peptides. Then the retention time for elution was selected, based on which the scheduled MRM analysis were repeated 3 times at the window size 3 min.

Experiment was performed once for each individual sample and then the data obtained were imported into Skyline and converted into the transition area for each peptide. Then the peak area obtained from each target peptide were normalized by the peak area obtained from the internal standard peptide.

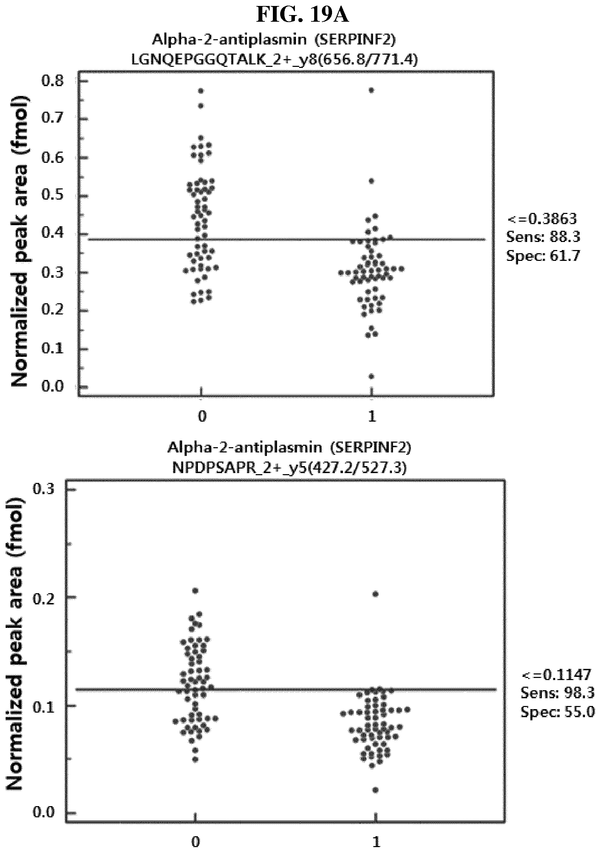

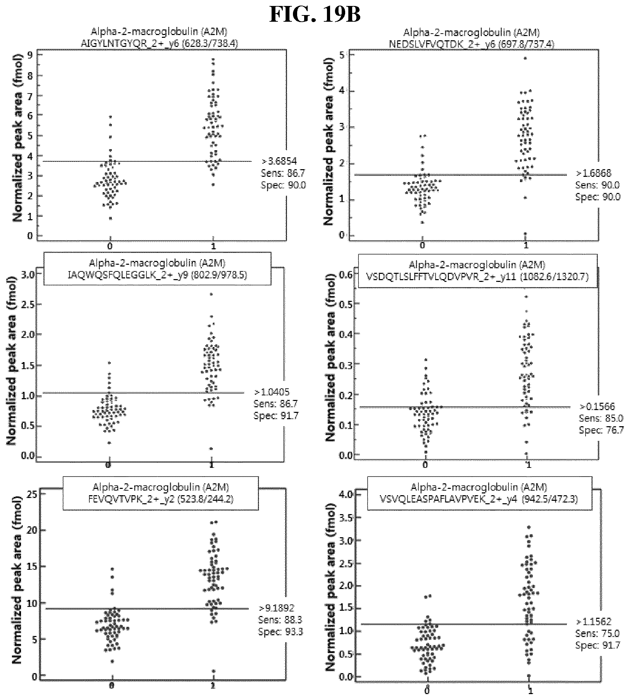

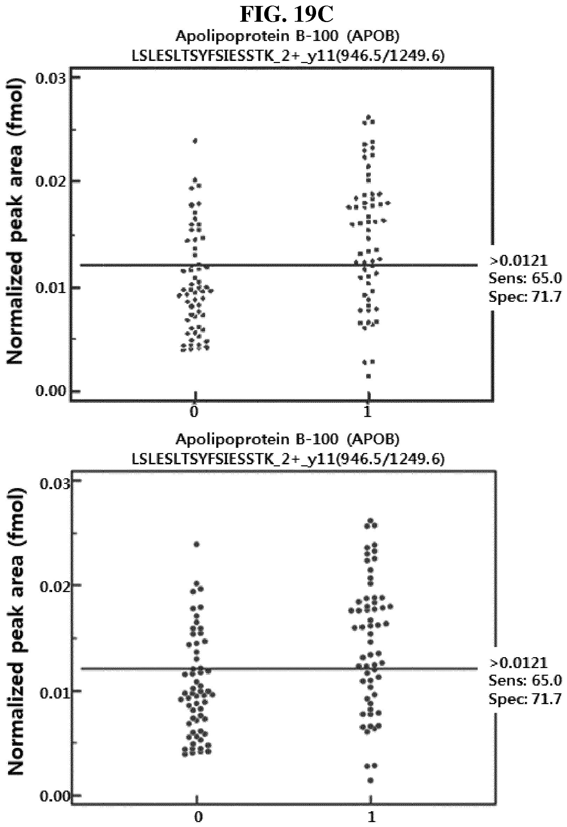

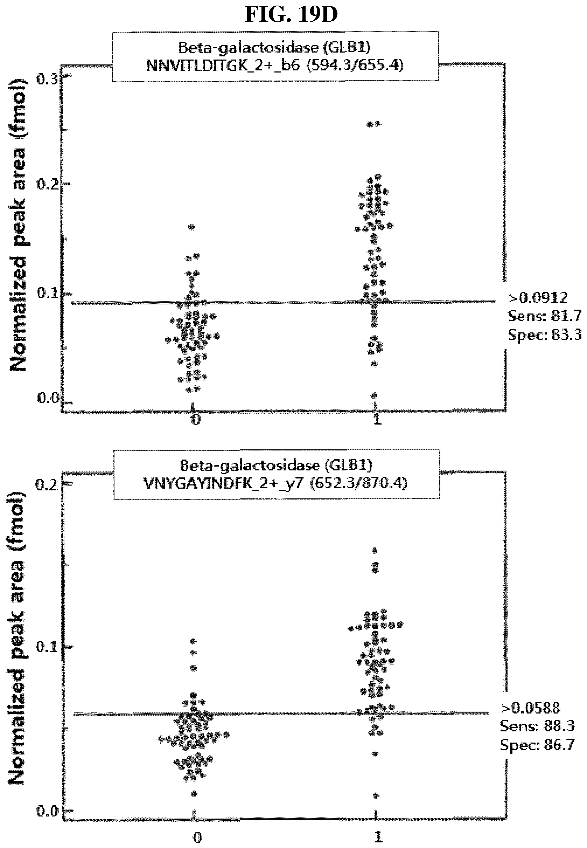

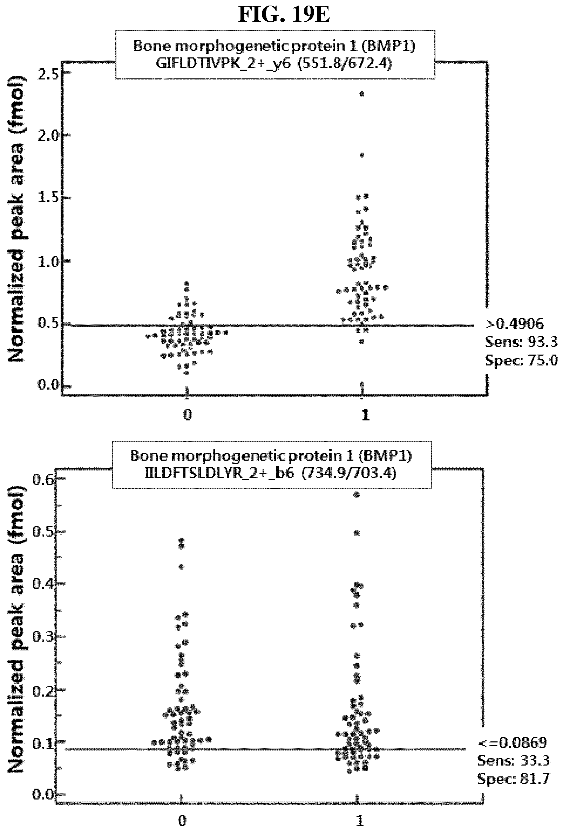

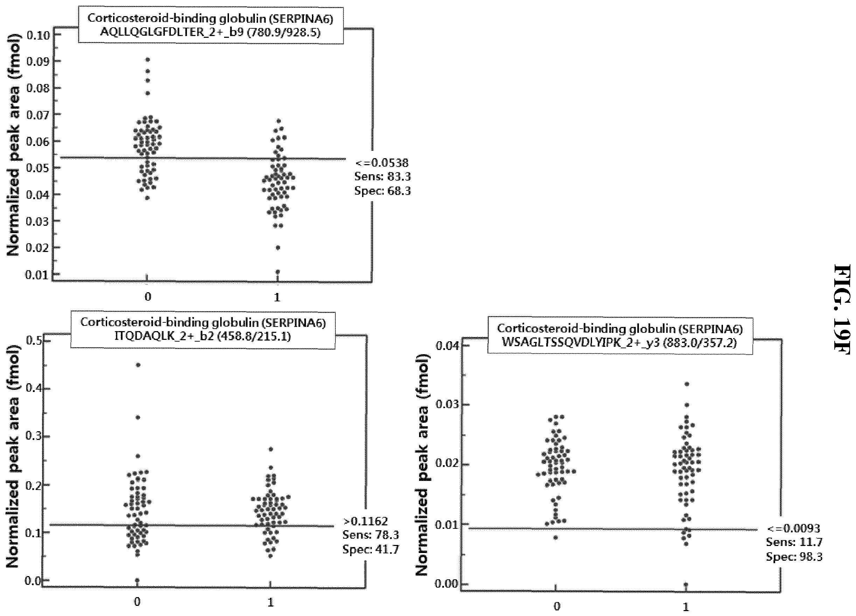

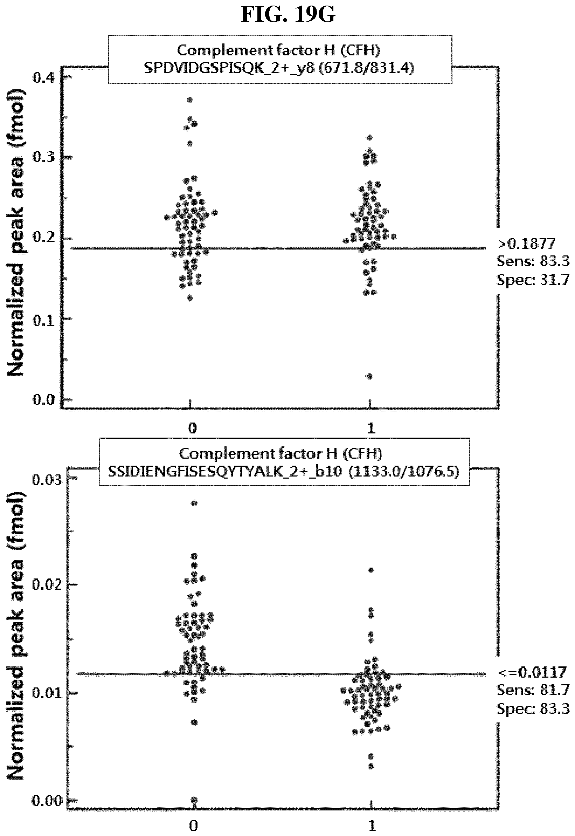

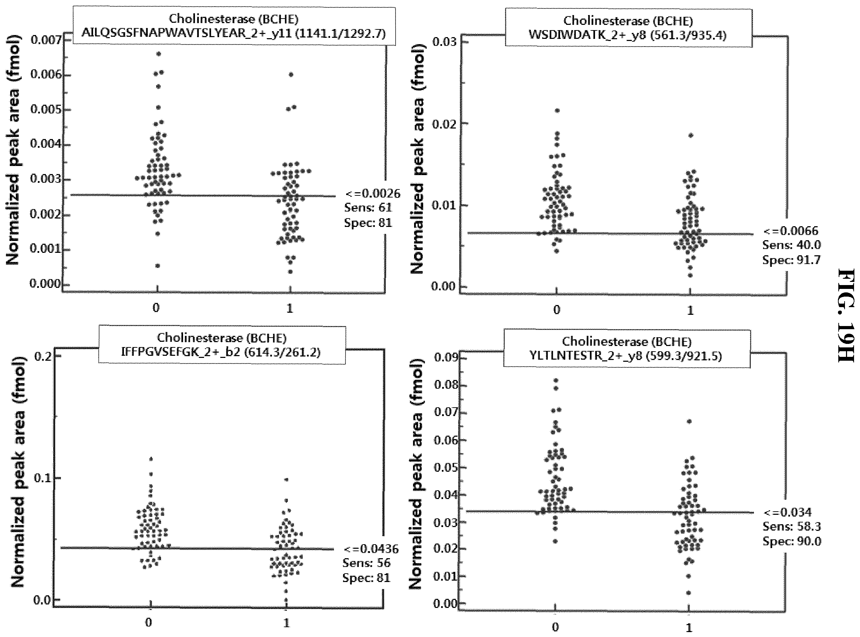

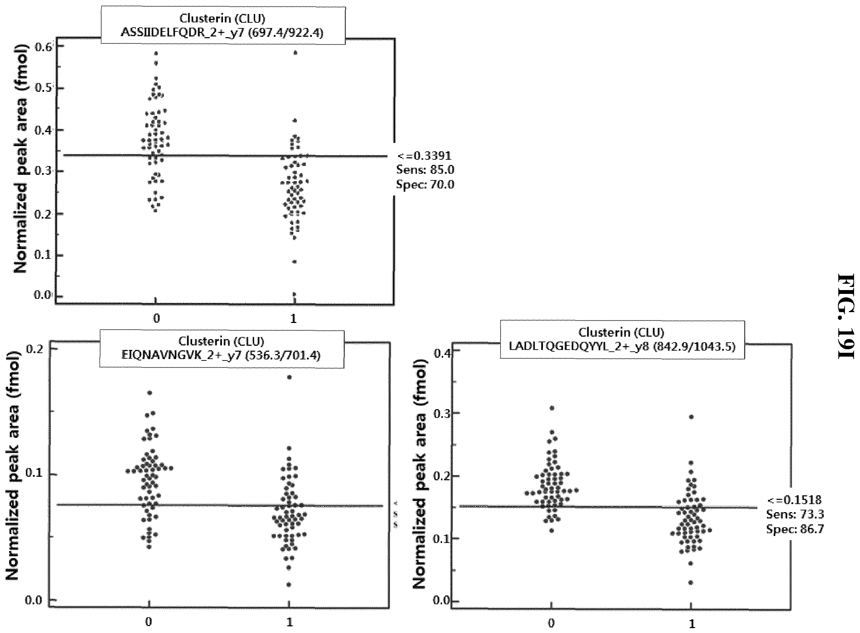

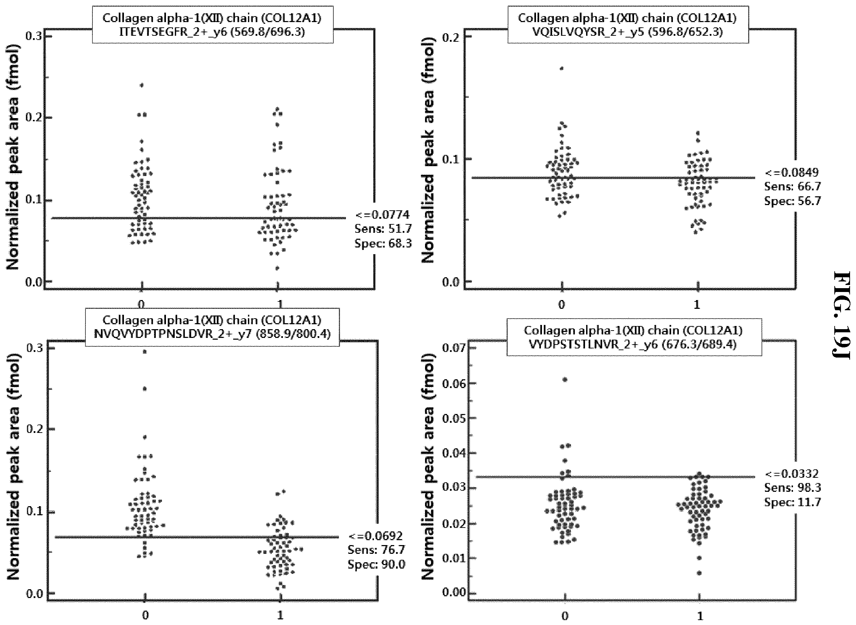

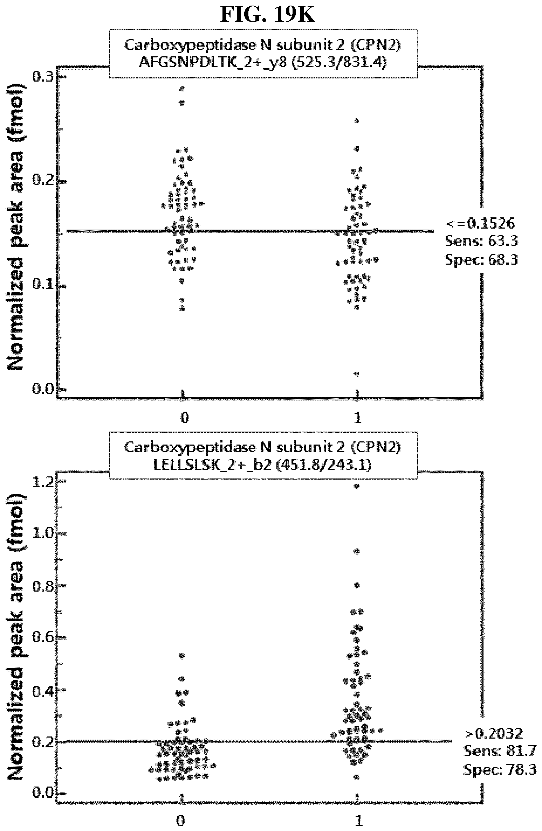

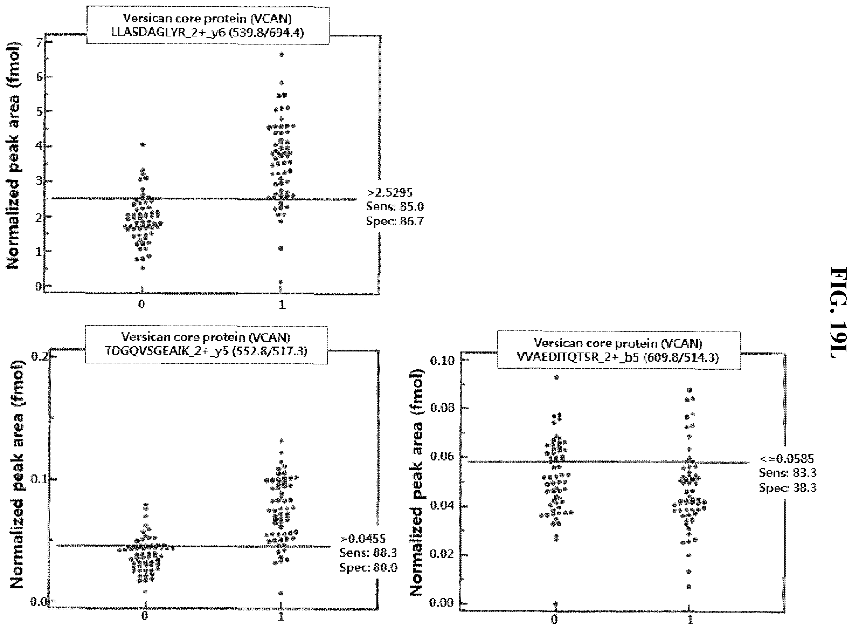

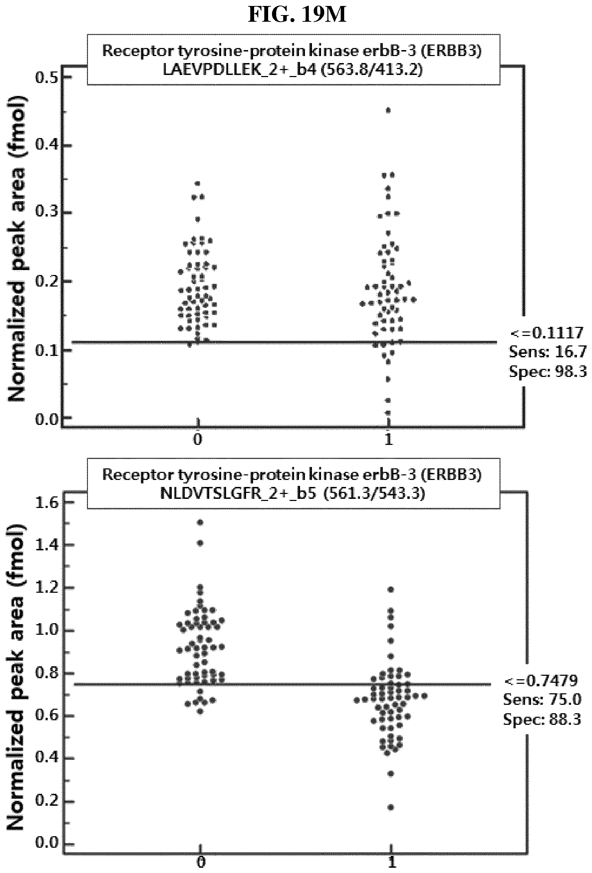

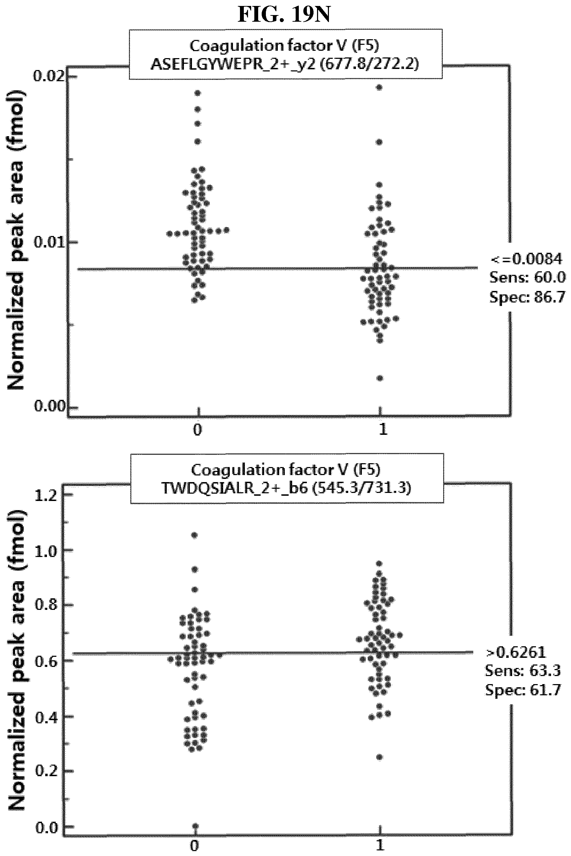

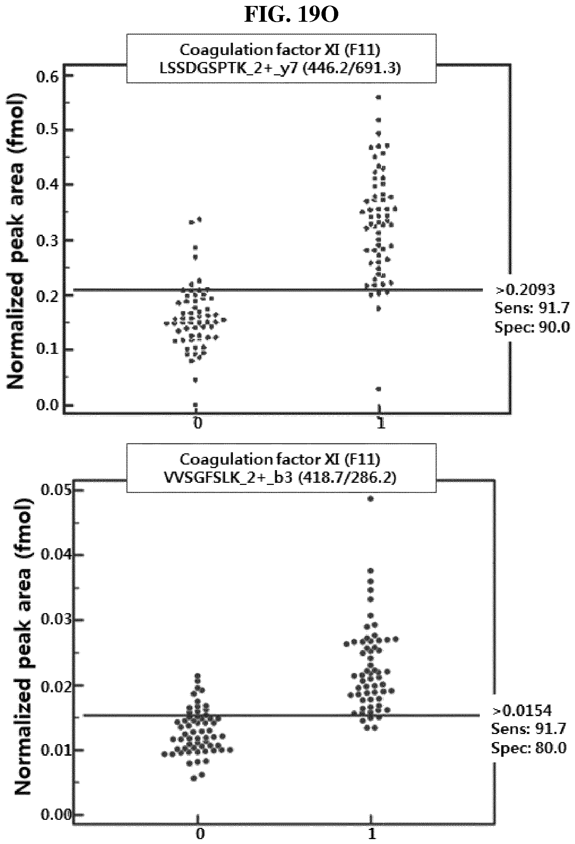

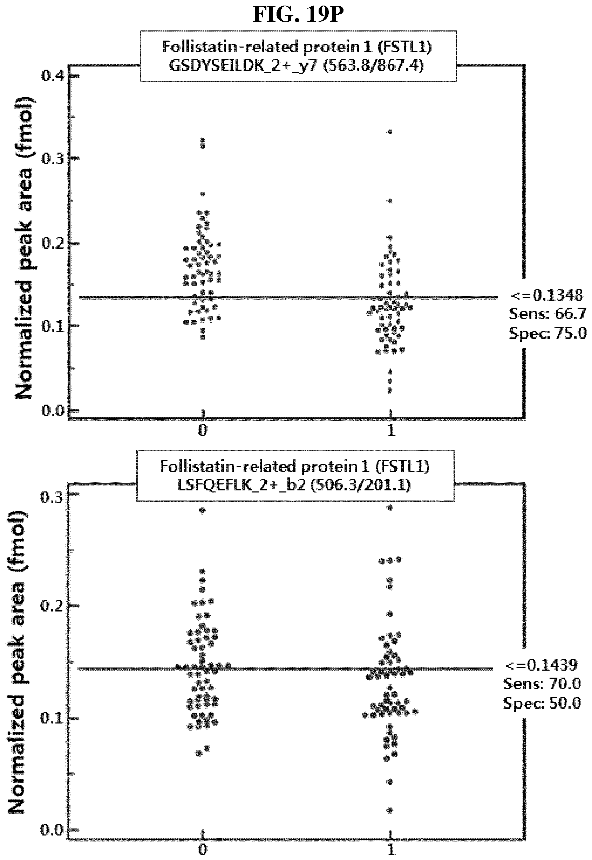

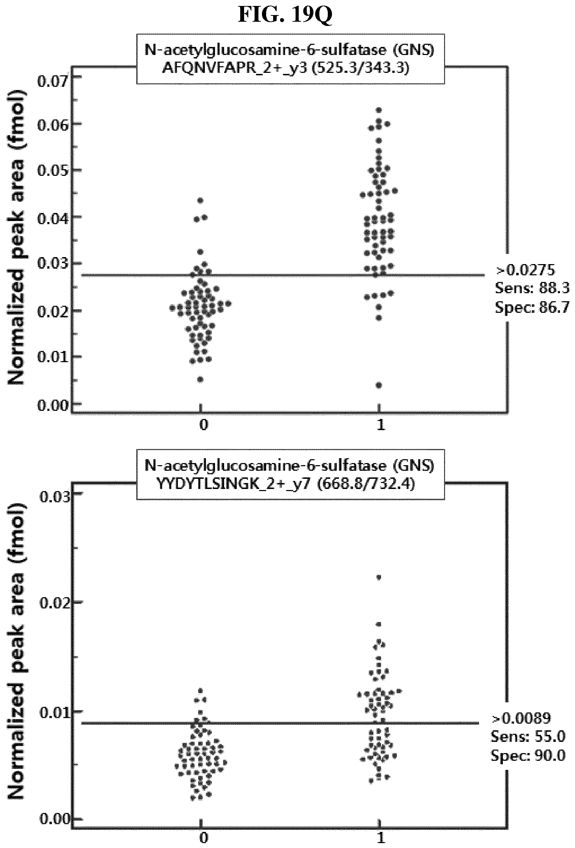

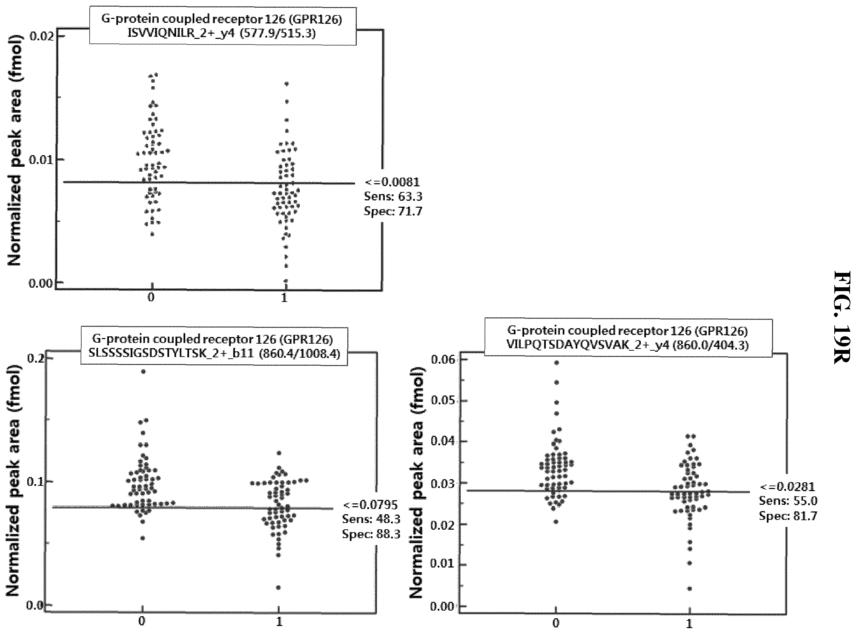

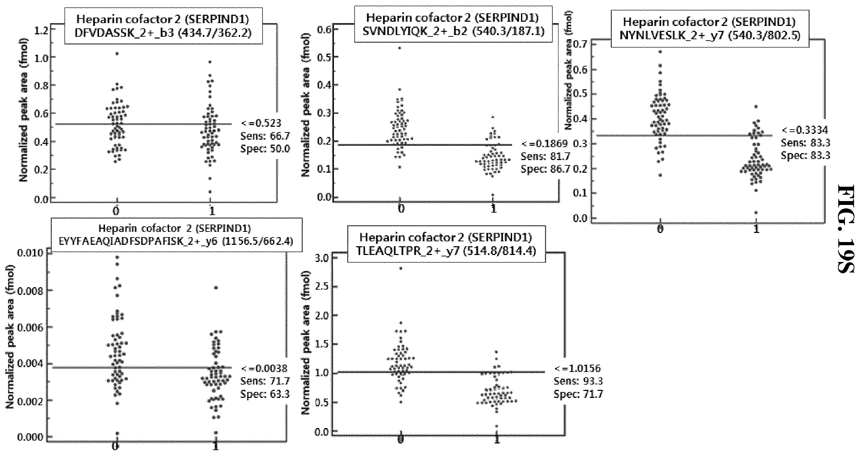

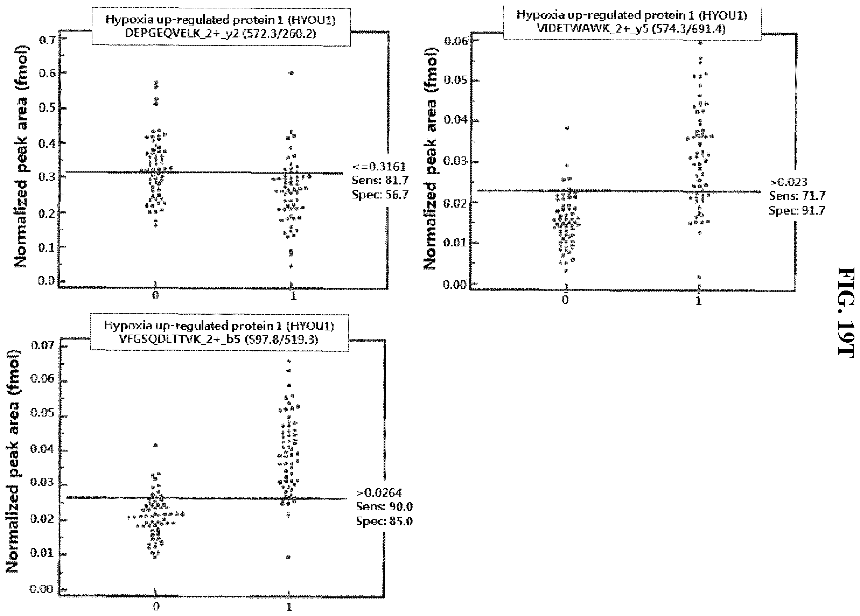

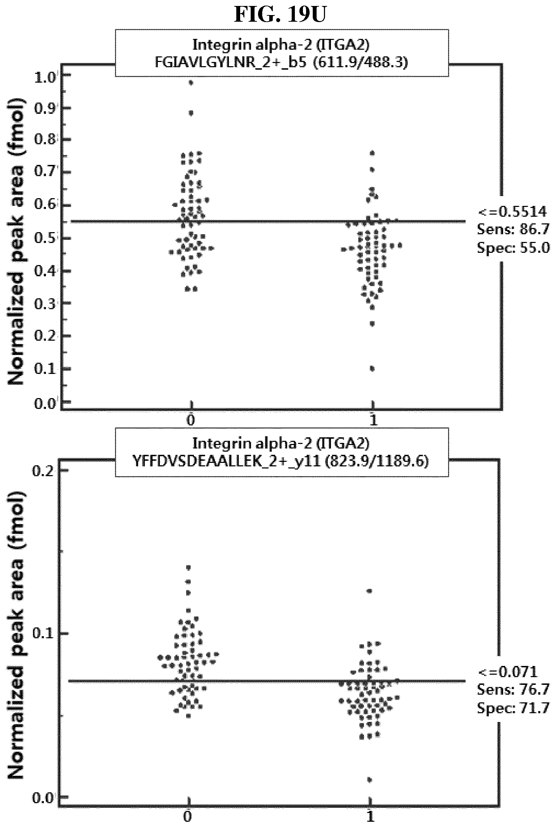

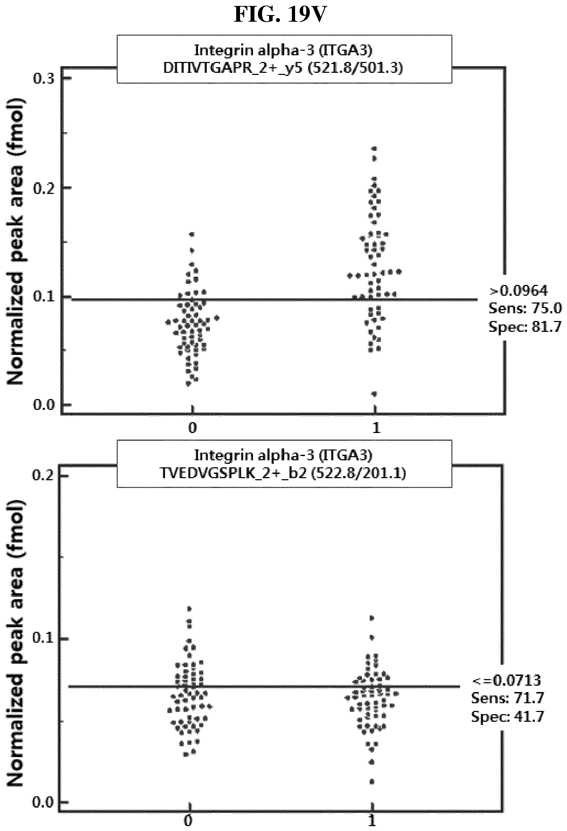

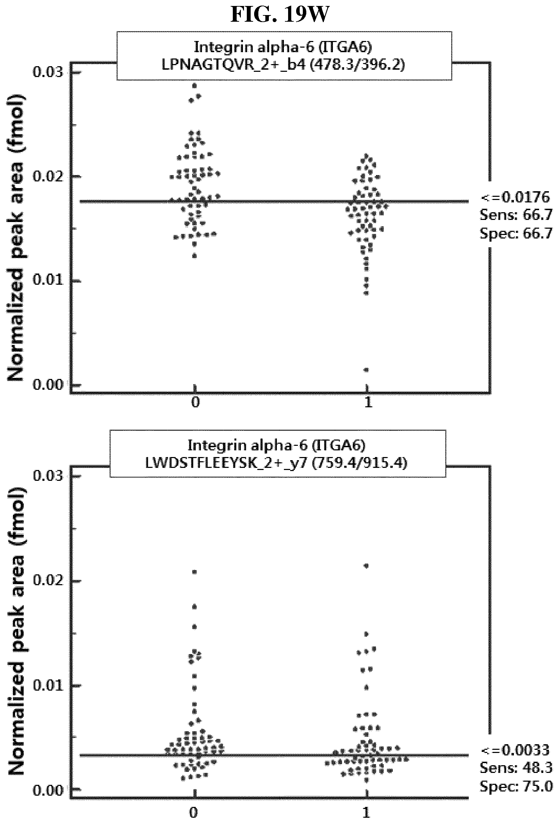

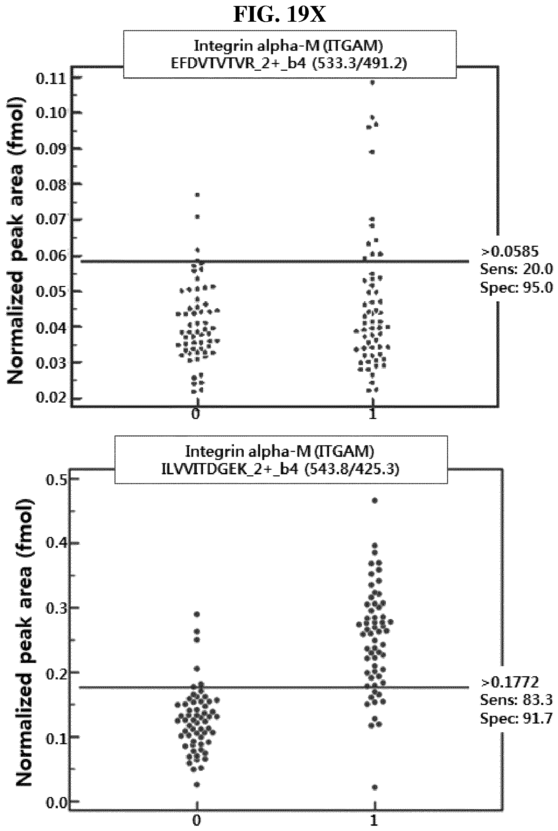

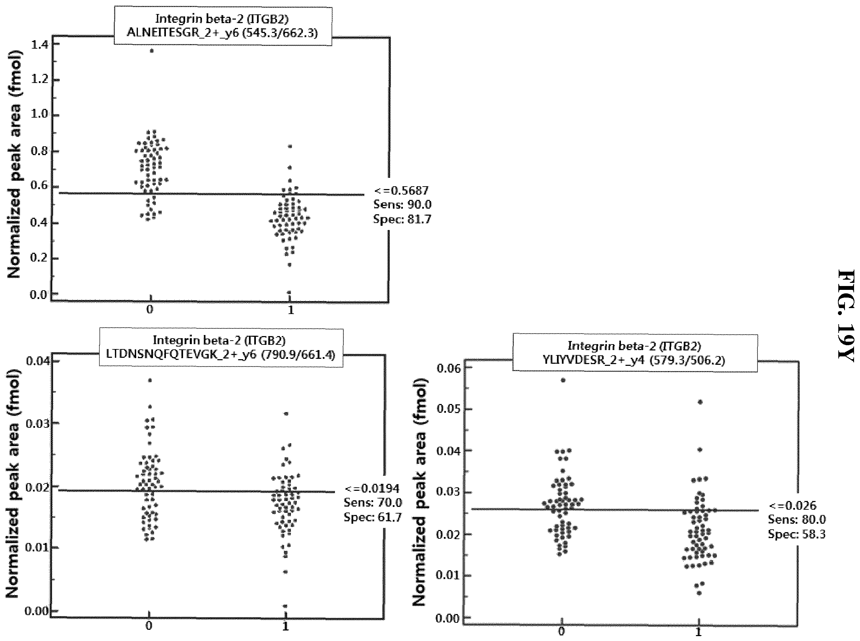

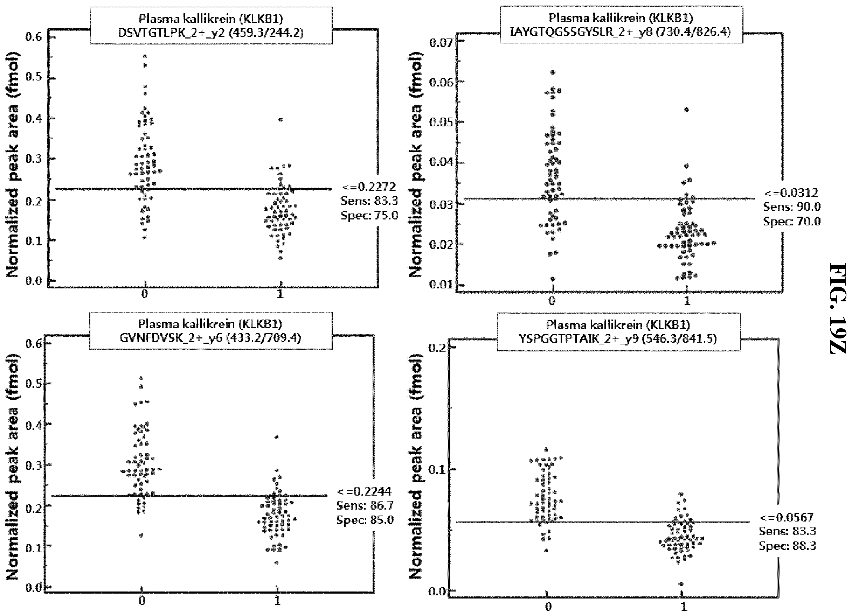

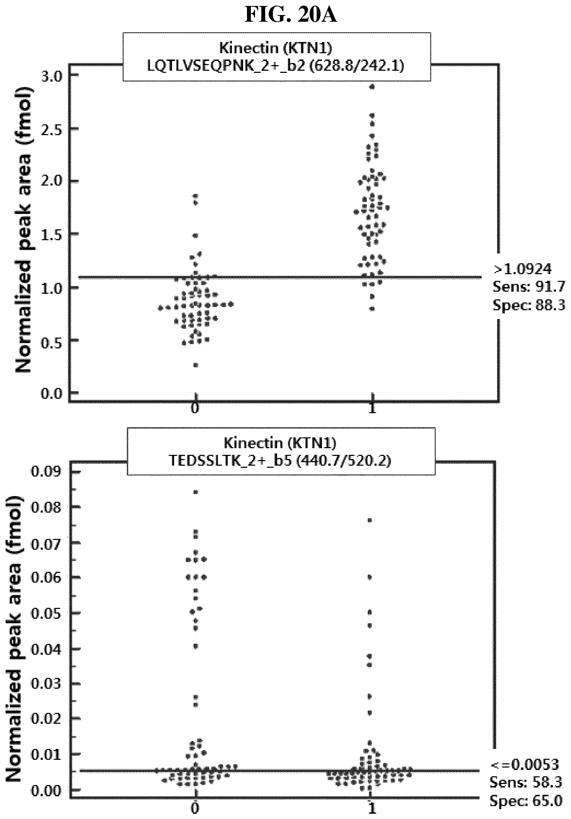

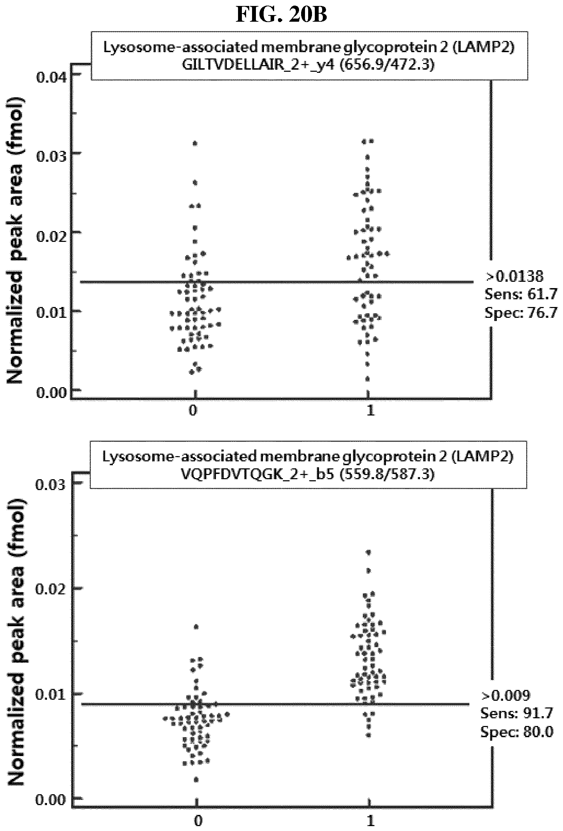

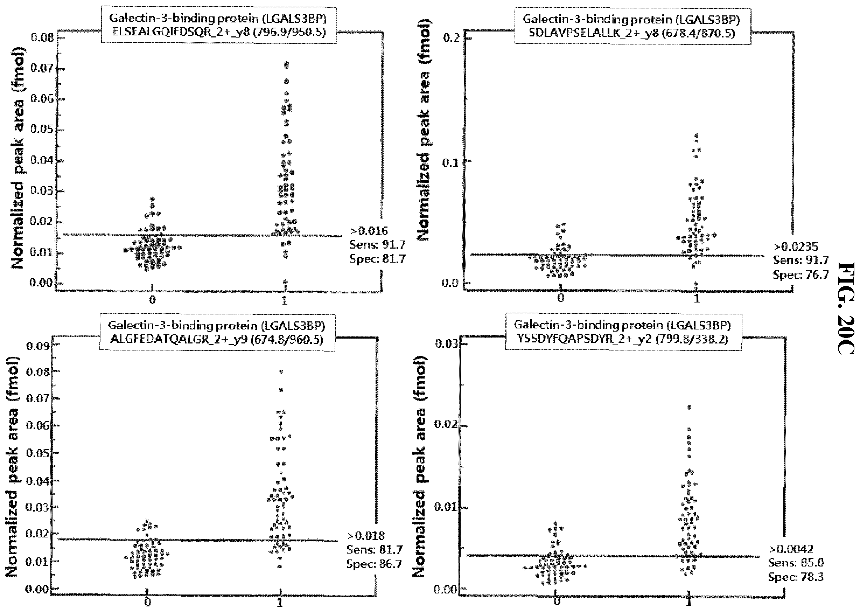

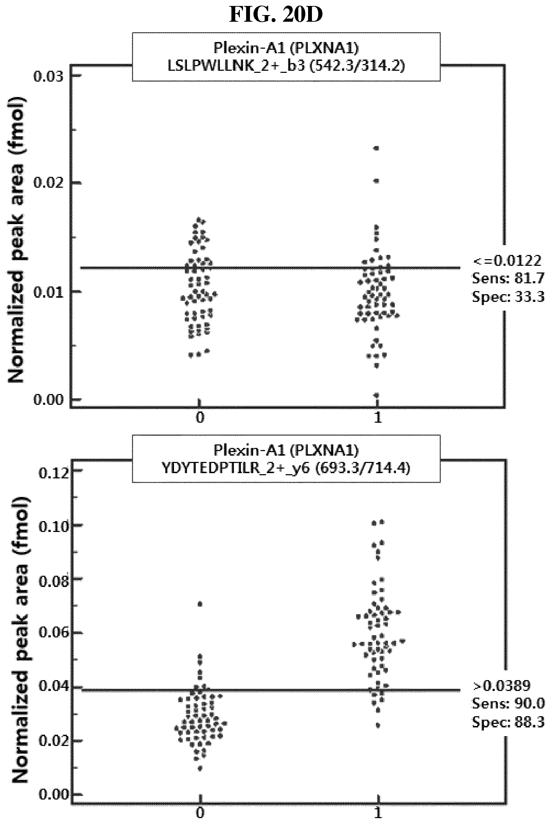

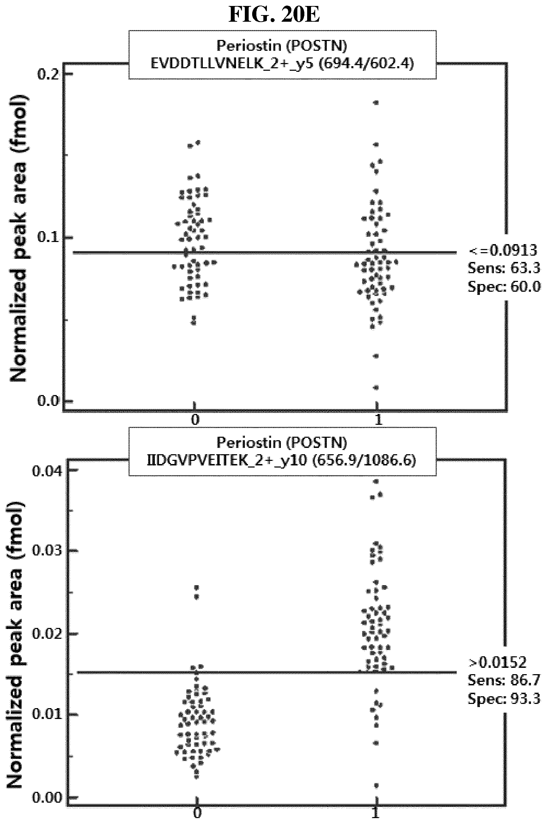

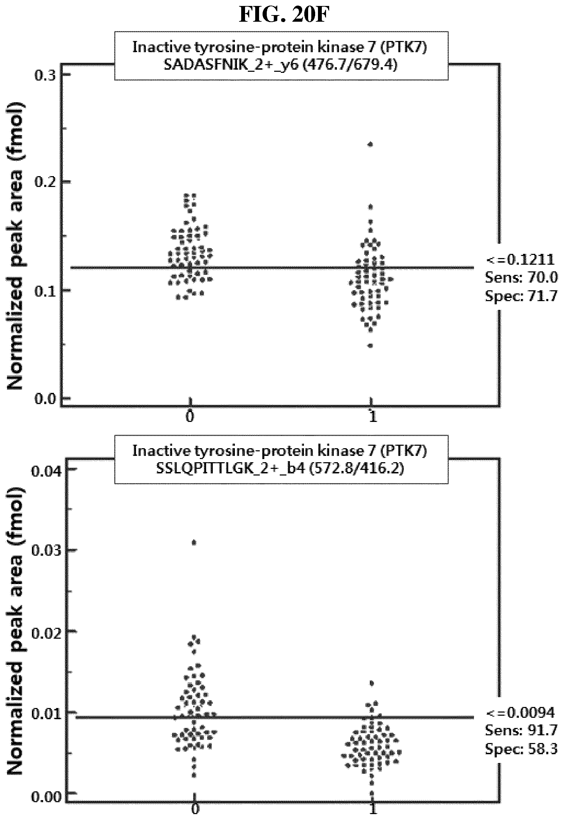

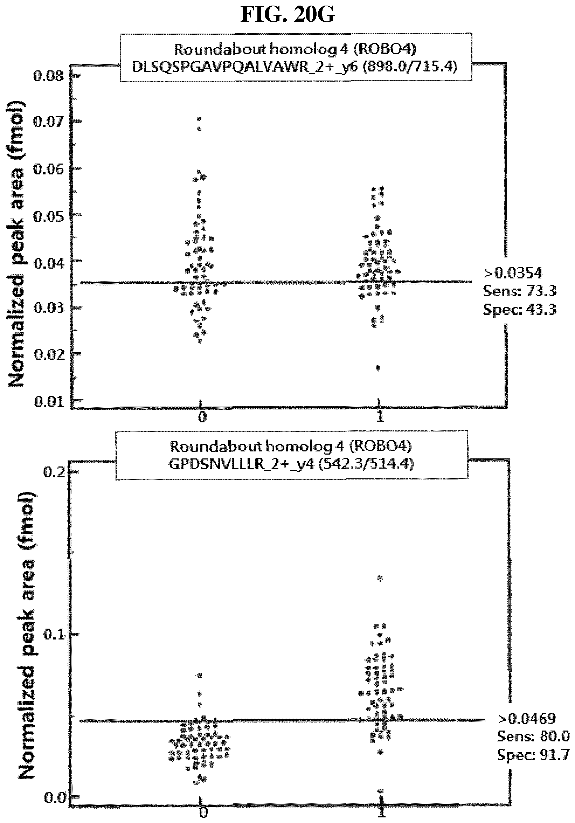

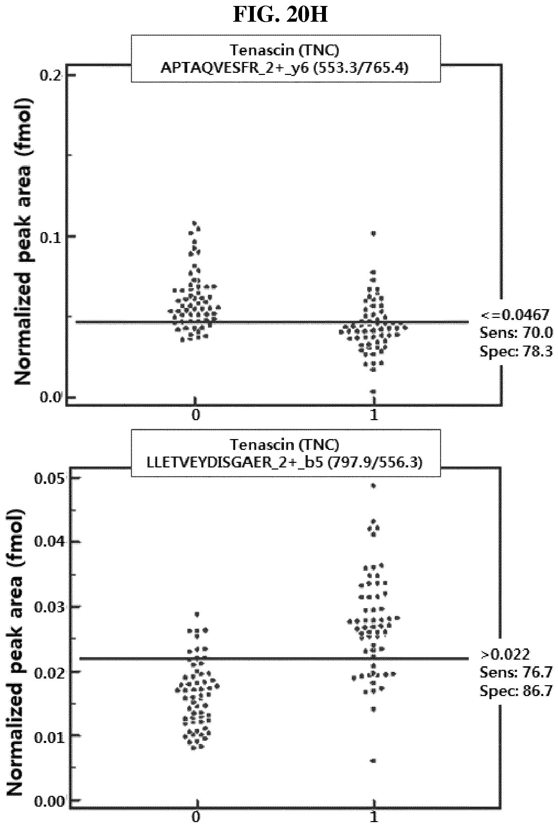

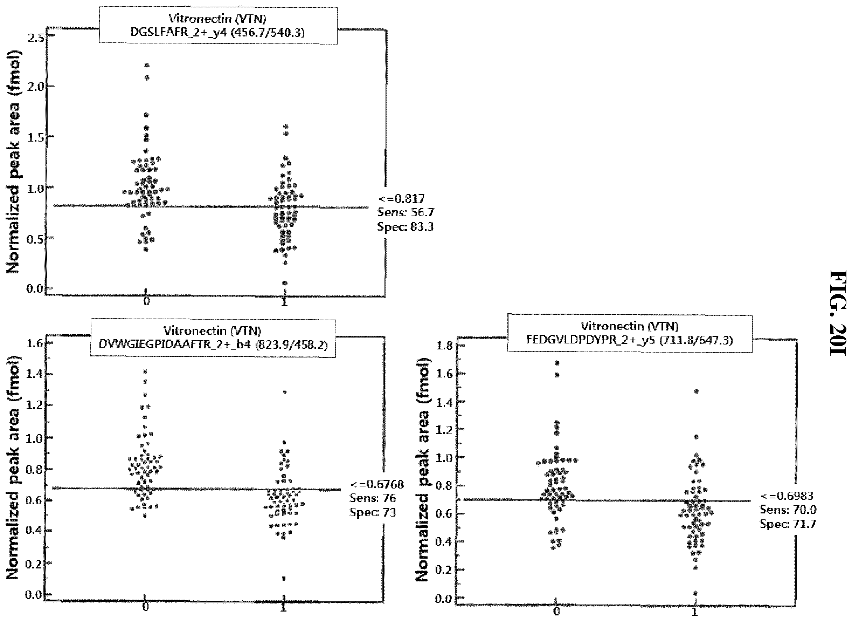

Results are shown in FIGs. 19a to 19z and 20a to 20i. ROC curves and interactive plots were determined on the target peptides showing significant difference (*p*-value < 0.05) between normal sample and HCC group. As a result, 35 glycoproteins showing AUC value of at least 0.7 were found, in which the de-glycosylated peptides were found to be 36, and the non-glycosylated peptides were 56.

The changes in the expression level of the analyzed peptides in HCC sample are disclosed in Tables 17-1 to 17-3.

In the tables below, the peptides with amino acid residue indicated in red are de-glycosylated peptides and others indicate non-glycosylated peptides.

**[Table 17-1]**

| **N.** | **Protein name** | **Peptide Sequence** | **Expressional Changes in HCC** |
|---|---|---|---|
| **1** | **Alpha-2-antiplasmin (SERPINF2)** | **LGNQEPGGQTALK** | **Decreased** |
| | | **NPDPSAPR** | **Decreased** |
| **2** | **Alpha-2-macroglobulin (A2M)** | **AIGYLNTGYQR** | **Increased** |
| | | **FEVQVTVPK** | **Increased** |
| | | **IAQWQSFQLEGGLK** | **Increased** |
| | | **NEDSLVFVQTDK** | **Increased** |
| | | **VSDQTLSLFFTVLQDVPVR** | **Increased** |
| | | **VSVQLEASPAFLAVPVEK** | **Increased** |
| **3** | **Apolipoprotein B-100 (APOB)** | **FEVDSPVYDATWSASLK** | **No change** |
| | | **LSLESLTSYFSIESSTK** | **Increased** |
| **4** | **Beta-galactosidase (GLB1)** | **NNVITLDITGK** | **Increased** |
| | | **VNYGAYINDFK** | **Increased** |
| **5** | **Bone morphogenetic protein 1 (BMP1)** | **GIFLDTIVPK** | **Increased** |
| | | **IILDFTSLDLYR** | **No change** |
| **6** | **Corticosteroid-binding globulin (SERPINA6)** | **AQLLQGLGFDLTER** | **Decreased** |
| | | **ITQDAQLK** | **No change** |
| | | **WSAGLTSSQVDLYIPK** | **No change** |
| **7** | **Complement factor H (CFH)** | **SPDVIDGSPISQK** | **No change** |
| | | **SSIDIENGFISESQYTYALK** | **Decreased** |
| **8** | **Cholinesterase (BCHE)** | **AILQSGSFNAPWAVTSLYEAR** | **Decreased** |
| | | **IFFPGVSEFGK** | **Decreased** |
| | | **WSDIWDATK** | **Decreased** |
| | | **YLTLNTESTR** | **Decreased** |
| **9** | **Clusterin (CLU)** | **ASSIIDELFQDR** | **Decreased** |
| | | **EIQNAVNGVK** | **Decreased** |
| | | **LADLTQGEDQYYLR** | **Decreased** |
| **10** | **Collagen alpha-1(XII) chain (COL12A1)** | **ITEVTSEGFR** | **No change** |
| | | **NVQVYDPTPNSLDVR** | **Decreased** |
| | | **VQISLVQYSR** | **No change** |
| | | **VYDPSTSTLNVR** | **No change** |
| **11** | **Carboxypeptidase N subunit 2 (CPN2)** | **AFGSNPDLTK** | **No change** |
| | | **LELLSLSK** | **Increased** |
| **12** | **Versican core protein (VCAN)** | **LLASDAGLYR** | **Increased** |
| | | **TDGQVSGEAIK** | **Increased** |
| | | **VVAEDITQTSR** | **No change** |
| **13** | **Receptor tyrosine-protein kinase erbB-3 (ERBB3)** | **LAEVPDLLEK** | **No change** |
| | | **NLDVTSLGFR** | **Decreased** |
| **14** | **Coagulation factor V (F5)** | **ASEFLGYWEPR** | **Decreased** |
| | | **TWDQSIALR** | **No change** |
| **15** | **Coagulation factor XI (F11)** | **LSSDGSPTK** | **Increased** |
| | | **WSGFSLK** | **Increased** |

**[Table 17-2]**

| | | | |
|---|---|---|---|
| | | **LSFQEFLK** | **No change** |
| **17** | **N-acetylglucosamine-6-sulfatase (GNS)** | **AFQNVFAPR** | **Increased** |
| | | **YYDYTLSINGK** | **Increased** |
| **18** | **G-protein coupled receptor 126 (GPR126)** | **ISVVIQNILR** | **Decreased** |
| | | **SLSSSSIGSDSTYLTSK** | **Decreased** |
| | | **VILPQTSDAYQVSVAK** | **Decreased** |
| **19** | **Heparin cofactor 2 (SERPIND1)** | **DFVDASSK** | **No change** |
| | | **EYYFAEAQIADFSDPAFISK** | **Decreased** |
| | | **NYNLVESLK** | **Decreased** |
| | | **SVNDLYIQK** | **Decreased** |
| | | **TLEAQLTPR** | **Decreased** |
| **20** | **Hypoxia up-regulated protein 1 (HYOU1)** | **DEPGEQVELK** | **Decreased** |
| | | **VFGSQDLTTVK** | **Increased** |
| | | **VIDETWAWK** | **In creased** |
| **21** | **Integrin alpha-2 (ITGA2)** | **FGIAVLGYLNR** | **Decreased** |
| | | **YFFDVSDEAALLEK** | **Decreased** |
| **22** | **Integrin alpha-3 (ITGA3)** | **DITIVTGAPR** | **Increased** |
| | | **TVEDVGSPLK** | **No change** |
| **23** | **Integrin alpha-6 (ITGA6)** | **LPNAGTQVR** | **Decreased** |
| | | **LWDSTFLEEYSK** | **No change** |
| **24** | **Integrin alpha-M (ITGAM)** | **EFDVTVTVR** | **No change** |
| | | **ILVVITDGEK** | **Increased** |
| **25** | **Integrin beta-2 (ITGB2)** | **ALNEITESGR** | **Decreased** |
| | | **LTDNSNQFQTEVGK** | **No change** |
| | | **YLIYVDESR** | **Decreased** |

**[Table 17-3]**

| | | | |
|---|---|---|---|
| **26** | **Plasma kallikrein (KLKB1)** | **DSVTGTLPK** | **Decreased** |
| | | **GVNFDVSK** | **Decreased** |
| | | **IAYGTQGSSGYSLR** | **Decreased** |
| | | **YSPGGTPTAIK** | **Decreased** |
| **27** | **Kinectin (KTN1)** | **LQTLVSEQPNK** | **Increased** |
| | | **TEDSSLTK** | **No change** |
| **28** | **ysosome-associated membrane glycoprotein 2 (LAMP** | **GILTVDELLAIR** | **No change** |
| | | **VQPFDVTQGK** | **Increased** |
| **29** | **Galectin-3-binding protein (LGALS3BP)** | **ALGFEDATQALGR** | **Increased** |
| | | **ELSEALGQIFDSQR** | **Increased** |
| | | **SDLAVPSELALLK** | **Increased** |
| | | **YSSDYFQAPSDYR** | **Increased** |
| **30** | **Plexin-Al (PLXNA1)** | **LSLPWLLNK** | **No change** |
| | | **YDYTEDPTILR** | **Increased** |
| **31** | **Periostin (POSTN)** | **EVDDTLLVNELK** | **No change** |
| | | **IIDGVPVEITEK** | **Increased** |
| **32** | **Inactive tyrosine-protein kinase 7 (PTK7)** | **SADASFNIK** | **Decreased** |
| | | **SSLQPITTLGK** | **Decreased** |
| **33** | **Roundabout homolog 4 (ROBO4)** | **DLSQSPGAVPQALVAWR** | **No change** |
| | | **GPDSNVLLLR** | **Increased** |
| **34** | **Tenascin (TNC)** | **APTAQVESFR** | **Decreased** |
| | | **LLETVEYDISGAER** | **Increased** |
| **35** | **Vitronectin (VTN)** | **DGSLFAFR** | **Decreased** |
| | | **DVWGIEGPIDAAFTR** | **Decreased** |
| | | **FEDGVLDPDYPR** | **Decreased** |

Unless defined or interpreted otherwise, all technical and scientific terms and any acronyms used herein have the same meanings as commonly understood by one of ordinary skill in the art in the field of the invention.

## Claims

1. A method of screening a biomarker for diagnosing or detecting cancer comprising steps of:
providing a biological sample comprising glycosylated proteins having a N-linked glycosylation motif, which is an asparagine-linked glycosylation motif;
de-glycosylating the proteins at the asparagine-linked glycosylation motif;
fragmenting the de-glycosylated proteins to obtain de-glycosylated peptides comprising the asparagine-linked motif and non-glycosylated peptides comprising a non-glycosylated motif and which do not comprise the asparagine-linked motif;
determining in the fragmented proteins an amount of the de-glycosylated peptide, an amount of the non-glycosylated peptide, and a ratio of the amount of the de-glycosylated peptide to the non-glycosylated peptide; and
selecting the proteins as a biomarker if the ratio is changed compared to that of a control, wherein the biological sample is from a patient and the control sample is from a healthy subject,
wherein the asparagine-linked glycosylation motif and the non-glycosylated peptide sequence that does not contain the asparagine-linked motif are specific for a given glycosylated protein,
wherein the non-glycosylated motif for a given protein is an amino acid sequence, which is not glycosylated and is from the same protein as the protein comprising the asparagine-linked glycosylation motif and does not contain a peptide motif Asp-Xxx-Ser/Thr, cysteine and methionine residues,
wherein the asparagine-linked glycosylation motif is represented by an amino acid sequence of AsnXxxSer (SEQ ID NO:3), AsnXxxThr (SEQ ID NO:4) or AsnXxxCys (SEQ ID NO:5),
wherein a residue of the de-glycosylation at the motif is Asn and a peptide fragment comprising AspXxxSer (SEQ ID NO:6), AspXxxThr (SEQ ID NO:7) or AspXxxCys (SEQ ID NO:8) is generated by the de-glycosylation,
wherein Xxx refers to any amino acid except proline,
wherein the de-glycosylation is performed by using a PNGase-F, and
wherein the amount is determined using a Mass spectrometry.

2. The method of any one of the previous claims, wherein the fragmentation is performed by treating the protein with at least one of a trypsin, a lysine-C or an arginine-C.

3. The method of any one of the previous claims, wherein the protein having an asparagine-linked glycosylation motif is provided as a biological sample comprising the same which is selected from the group consisting of a cell, a whole blood, a serum, a plasma, a saliva, a urine, a follicular fluid, a breast milk and a pancreatin.

## Patentansprüche

1. Verfahren zum Screenen eines Biomarkers zum Diagnostizieren oder Feststellen von Krebs, umfassend die Schritte des:
Bereitstellens einer biologischen Probe umfassend glycosylierte Proteine, die ein N-verknüpftes Glycosylierungsmuster aufweisen, das ein asparaginverknüpftes Glycosylierungsmuster ist;
Deglycosylierens der Proteine an dem asparaginverknüpften Glycosylierungsmuster;
Fragmentierens der deglycosylierten Proteine, um deglycosylierte Peptide zu erhalten, die das asparaginverknüpfte Muster und nichtglycosylierte Peptide umfassen, die ein nichtglycosyliertes Muster umfassen und die das asparaginverknüpfte Muster nicht umfassen;
Bestimmens, in den fragmentierten Proteinen, einer Menge des deglycosylierten Peptids, einer Menge des nichtglycosylierten Peptids und eines Verhältnisses der Menge des deglycosylierten Peptids zu dem nichtglycosylierten Peptid; und
Auswählens des Proteins als Biomarker, wenn das Verhältnis im Vergleich mit demjenigen einer Kontrolle geändert wird, wobei die biologische Probe von einem Patienten stammt und die Kontrollprobe von einem gesunden Subjekt stammt,
wobei das asparaginverknüpfte Glycosylierungsmuster und die Sequenz des nichtglycosylierten Peptids, die kein asparaginverknüpftes Glycosylierungsmuster enthält, für ein vorgegebenes glycosyliertes Protein spezifisch sind,
wobei das nichtglycosylierte Muster für ein vorgegebenes Protein eine Aminosäuresequenz ist, die nicht glycosyliert ist und von demselben Protein wie das Protein stammt, das das asparaginverknüpfte Glycosylierungsmuster umfasst und kein Peptidmuster Asp-Xxx-Ser/Thr, keine Cystein- und Methioninreste enthält,
wobei das asparaginverknüpfte Glycosylierungsmuster durch eine Aminosäuresequenz von AsnXxxSer (SEQ ID NO:3), AsnXxxThr (SEQ ID NO:4) oder AsnXxxCys (SEQ ID NO:5) dargestellt ist,
wobei ein Rest der Deglycosylierung in dem Muster Asn ist und ein Peptidfragment, das AsXxxSer (SEQ ID NO:6), AspXxxThr (SEQ ID NO:7) oder AspXxxCys (SEQ ID NO:8) umfasst, durch die Deglycosylierung erzeugt wird,
wobei Xxx sich auf irgendeine Aminosäure mit Ausnahme von Prolin bezieht,
wobei die Deglycosylierung durch Verwenden einer PNGase-F ausgeführt wird und
wobei die Menge unter Anwendung einer Massenspektrometrie bestimmt wird.

2. Verfahren nach irgendeinem der vorhergehenden Ansprüche, wobei die Fragmentierung durch Behandeln des Proteins mit mindestens einem von einem Trypsin, einem Lysin-C oder einem Arginin-C ausgeführt wird.

3. Verfahren nach irgendeinem der vorhergehenden Ansprüche, wobei das Protein, das ein asparaginverknüpftes Glycosylierungsmuster aufweist, als biologische Probe bereitgestellt wird, die dasselbe umfasst, das aus der Gruppe ausgewählt ist bestehend aus einer Zelle, einem Ganzblut, einem Serum, einem Plasma, einem Speichel, einem Urin, einer follikulären Flüssigkeit, einer Brustmilch und einem Pankreatin.

## Revendications

1. Procédé de criblage d'un biomarqueur de diagnostic ou de détection de cancer comprenant les étapes suivantes :
fourniture d'un échantillon biologique comprenant des protéines glycosylées ayant un motif de glycosylation lié à N, qui est un motif de glycosylation lié à l'asparagine ;
déglycosylation des protéines au niveau du motif de glycosylation lié à l'asparagine ;
fragmentation des protéines déglycosylées pour obtenir des peptides déglycosylés comprenant le motif lié à l'asparagine et des peptides non glycosylés comprenant un motif non glycosylé et qui ne comprennent pas le motif lié à l'asparagine ;
détermination dans les protéines fragmentées d'une quantité du peptide déglycosylé, d'une quantité du peptide non glycosylé, et d'un rapport de la quantité du peptide déglycosylé sur le peptide non glycosylé ; et
sélection des protéines en tant que biomarqueur si le rapport est modifié comparé à celui d'un témoin, dans lequel l'échantillon biologique provient d'un patient et l'échantillon témoin provient d'un sujet sain,
dans lequel le motif de glycosylation lié à l'asparagine et la séquence peptidique non glycosylée qui ne contient pas le motif lié à l'asparagine sont spécifiques d'une protéine glycosylée donnée,
dans lequel le motif non glycosylé pour une protéine donnée est une séquence d'acides aminés, qui n'est pas glycosylée et provient de la même protéine que la protéine comprenant le motif de glycosylation lié à l'asparagine et ne contient pas de motif peptidique Asp-Xxx-Ser/Thr, de résidus cystéine et méthionine,
dans lequel le motif de glycosylation lié à l'asparagine est représenté par une séquence d'acides aminés d'AsnXxxSer (SEQ ID NO : 3), d'AsnXxxThr (SEQ ID NO : 4) ou d'AsnXxxCys (SEQ ID NO : 5),
dans lequel un résidu de dé-glycosylation au niveau du motif est Asn et un fragment peptidique comprenant AspXxxSer (SEQ ID NO : 6), AspXxxThr (SEQ ID NO : 7) ou AspXxxCys (SEQ ID NO : 8) est généré par déglycosylation,
dans lequel Xxx fait référence à tout acide aminé à l'exception de la proline,
dans lequel la déglycosylation est réalisée en utilisant une PNGase-F, et
dans lequel la quantité est déterminée par spectrométrie de masse.

2. Procédé selon l'une quelconque des revendications précédentes, dans lequel la fragmentation est réalisée par traitement de la protéine avec au moins l'une d'une trypsine, d'une lysine-C ou d'une arginine-C.

3. Procédé selon l'une quelconque des revendications précédentes, dans lequel la protéine ayant le motif de glycosylation lié à l'asparagine est fournie sous la forme d'un échantillon biologique la comprenant qui est sélectionné dans le groupe constitué par une cellule, un sang total, un sérum, un plasma, une salive, une urine, un fluide folliculaire, un lait maternel et une pancréatine.
